# FASCICULE DE BREVET EUROPEEN

(11) **EP 2 010 494 B1**
(45) Date de publication et mention de la délivrance du brevet: **01.02.2012**
(21) Numéro de dépôt: 07788947.5
(22) Date de dépôt: 13.04.2007
(51) Int. Cl.: C07D 213/53, C07D 213/55, A61Q 5/06, C09B 67/00

(54) **COMPOSES MONOCHROMOPHORIQUES MONOCATIONIQUES PARTICULIERS DE TYPE HYDRAZONE COMPRENANT UN MOTIF 2-, 4-PYRIDINIUM OU 2-, 4-QUINOLINIUM, LEUR SYNTHESE, COMPOSITIONS TINCTORIALES LES COMPRENANT, PROCEDE DE COLORATION DE FIBRES KERATINIQUES**
SPEZIFISCHE MONOKATIONISCHE UND MONOCHROMOPHORE HYDRAZON-VERBINDUNGEN MIT EINER 2,4-PYRIDIN- ODER 2,4-CHINOLIN-EINHEIT, DEREN SYNTHESE, FÄRBEMITTELZUSAMMENSETZUNGEN DAMIT UND VERFAHREN ZUR FÄRBUNG VON KERATINFASERN
SPECIFIC MONOCATIONIC MONOCHROMOPHORIC COMPOUNDS OF HYDRAZONE TYPE COMPRISING A 2-, 4-PYRIDINIUM OR 2-, 4-QUINOLINIUM UNIT, SYNTHESIS THEREOF, DYE COMPOSITIONS CONTAINING THEM, AND METHOD FOR DYEING KERATIN FIBRES

(30) Priorité: 13.04.2006 FR 0603322; 02.05.2006 US 796516 P
(43) Date de publication de la demande: 07.01.2009
(73) Titulaire: L'Oréal, 75008 Paris (FR)
(72) Inventeur: DAVID, Hervé, F-94210 La Varenne Saint Hilaire (FR); MURGUET, Nadège, F-91120 Palaiseau (FR); GREAVES, Andrew, F-77144 Montevrain (FR)
(74) Mandataire: Wattremez, Catherine
(86) Numéro de dépôt international: PCT/FR2007/051111
(87) Numéro de publication internationale: WO 2007/125238

(56) Documents cités:
- EP-A- 1 437 123
- EP-A1- 0 850 638
- EP-A2- 1 172 082
- WO-A-95/01772
- WO-A-03/060015
- WO-A1-2004/072183
- GB-A- 924 601

## Description

La présente invention a pour objet des colorants monochromophoriques monocationiques particuliers de type hydrazone de formule particulière comprenant un motif 2- ou 4-pyridinium ou 2- ou 4-quinolinium, des compositions tinctoriales comprenant dans un milieu approprié pour la teinture des fibres kératiniques, de tels composés à titre de colorant direct, un procédé de coloration de fibres kératiniques mettant en oeuvre cette composition, ainsi qu'un dispositif à plusieurs compartiments. Elle concerne de même un procédé de synthèse de tels composés.

Il est connu de teindre les fibres kératiniques et en particulier les fibres kératiniques humaines, telles que les cheveux, avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique azinique, triarylméthane ou encore des colorants directs aromatiques comprenant une fonction hydrazone.

Les colorants directs à fonction hydrazone sont des composés intéressants mais ils présentent l'inconvénient de ne permettre d'accéder dans la majorité des cas, qu'à des nuances allant du jaune à l'orange. Dans de très rares cas, les colorants à motif hydrazone fournissent d'autres nuances. Par exemple, les colorants de type hydrazone à motif acridinium du brevet GB924601 fournissent des nuances violettes, les colorants de type hydrazone à motif 2-oxopyrimidinium du brevet FR1532806 fournissent des nuances allant du jaune au violet.

Cependant, ces colorants sont instables en conditions alcalines et éclaircissantes. Ils présentent également une mauvaise tenue aux shampooings répétés.

Il a été découvert de manière surprenante que l'on pouvait étendre de manière substantielle la gamme de couleurs susceptible d'être obtenue à partir de cette famille de colorants directs, ces colorants étant stables en conditions alcalines éclaircissantes. Ainsi, les composés selon l'invention permettent d'obtenir des nuances allant jusqu'au violet. Obtenir une telle variation était a priori loin d'être évidente car les colorants de type hydrazone connus ont une couleur variant relativement peu même lorsque la nature du groupement porté par le noyau aromatique et se trouvant en para de la fonction hydrazone passe d'un atome d'hydrogène à un atome d'halogène, ou encore à un groupement alcoxy.

Ces buts et d'autres sont atteints par la présente invention qui a donc pour objet des composés monochromophoriques monocationiques comprenant une fonction hydrazone de formules (I) et (II) suivantes, leurs formes mésomères ainsi que leurs sels d'additions avec un acide et leurs solvates :

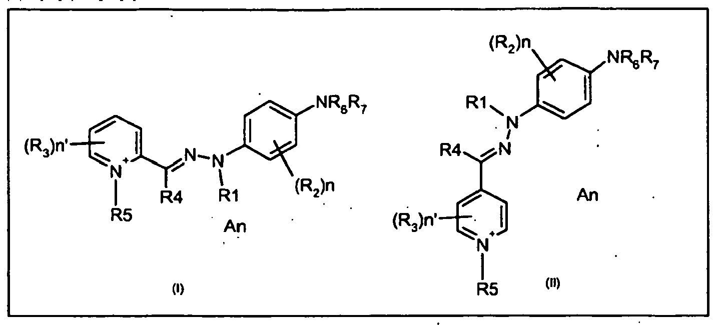

Formules (I) et/ou (II) dans lesquelles :
* Le radical R₁ représente :
   - un hydrogène
   - un radical alkyle en C₁-C₂₀, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, SO₂ ou leurs combinaisons ; le radical alkyle ne comportant pas de fonction nitro, nitroso, peroxo ou diazo
   - un radical phényle éventuellement substitué
   - un radical benzyle éventuellement substitué
   - un radical alkylcarbonyle (R-CO-) dans lequel R représente un radical alkyle en C₁-C₄.
   - un radical alkylsulfonyle (RSO₂-) dans lequel R représente un radical alkyle en C₁-C₄.
   - un radical arylsulfonyle (R'SO₂-) dans lequel R' représente un radical phényle ou benzyle éventuellement substitué.
   - un radical (di-)(alkyl)aminosulfonyle ((R)₂N-SO₂-) dans lequel les radicaux R indépendamment représentent un hydrogène, un radical alkyle en C₁-C₄.
   - un radical (di-)(alkyl) aminocarbonyle (R)₂N-CO-) dans lequel les radicaux R indépendamment représentent un hydrogène, un radical alkyle en C₁-C₄.
* Les radicaux R₅, identiques ou différents, représentent :
   - un radical alkyle en C₁-C₂₀, éventuellement substitué, éventuellement interrompu par un ou plusieurs hétéroatomes et/ou par un ou plusieurs groupements comprenant au moins un hétéroatome, de préférence choisis parmi l'oxygène, le soufre, CO, SO, SO₂ ou leurs combinaisons ; le radical alkyle ne comportant pas de fonction nitro, nitroso, peroxo ou diazo
   - un radical trimethylsilyl alkyle en C₁-C₄.
   - un radical phényle éventuellement substitué
   - un radical benzyle éventuellement substitué
* Les radicaux R₂ et R₃, indépendamment l'un de l'autre, identiques ou différents, représentent :
   - un atome d'halogène
   - un radical alkyle en C₁-C₁₆ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂ ou leurs combinaisons ; le radical alkyle ne comportant pas de fonction nitro, nitroso, peroxo ou diazo
   - un radical hydroxyle
   - un radical alcoxy en C₁-C₄ ; un groupement (poly)hydroxyalcoxy en C₂-C₄
   - un radical alcoxycarbonyle (RO-CO-) dans lequel R représente un radical alkyle en C₁-C₄.
   - un radical alkylcarbonyloxy (RCO-O-) dans lequel R représente un radical alkyle en C₁-C₄.
   - un radical aryloxy éventuellement substitué
   - un radical (di-)arylamino éventuellement substitué
   - un radical amino éventuellement substitué par un ou deux radicaux alkyle en C₁-C₄, identiques ou différents, éventuellement porteurs d'au moins un groupement hydroxyle ou alcoxy en C₁-C₂, lesdits radicaux alkyle pouvant éventuellement former avec l'atome d'azote auquel ils sont rattachés, un hétérocycle à 5 ou 7 chaînons, saturé ou insaturé, éventuellement substitué, éventuellement aromatique, comprenant éventuellement un autre hétéroatome identique ou différent de l'azote ;
   - un radical alkylcarbonylamino (RCO-NR'-) dans lequel le radical R représente un radical alkyle en C₁-C₄ et R' représente un hydrogène, un radical alkyle en C₁-C₄.
   - un groupement (di-)(alkyl)aminocarbonyle ((R)₂N-CO-) dans lequel les radicaux R, indépendamment l'un de l'autre représentent un hydrogène, un radical alkyle en C₁-C₄.
   - un radical uréido (N(R)₂-CO-NR'-) dans lequel les radicaux R, indépendamment les uns des autres, représentent un radical alkyle en C₁-C₄ et R' représente un hydrogène, un radical alkyle en C₁-C₄.
   - un radical (di-)(alkyl) aminosulfonyle ((R)₂N-SO₂-) dans lequel le radical R représente un atome d'hydrogène, un radical alkyle en C₁-C₄.
   - un radical alkylthio (R-S-) dans lequel le radical R représente un radical alkyle en C₁-C₄.
   - un radical alkylsulfonylamino (RSO₂-NR'-) dans lequel dans lequel le radical R représente un radical alkyle en C₁-C₄, et le radical R' représente un atome d'hydrogène, un radical alkyle en C₁-C₄.
   - un radical cyano (-C≡N)
   - un radical phényle
   - un radical trifluorométhyle (-CF₃)
   - un radical thio (-SH)
   - un radical alkylsulfinyle (RSO-) dans lequel le radical R représente un radical alkyle en C₁-C₄.
   - un radical alkylsulfonyle (RSO₂-) dans lequel le radical R représente un radical alkyle en C₁-C₄.
*R₁ peut former avec un radical R₂ en ortho du groupement NR₁ et avec l'atome d'azote substitué par R₁, un hétérocycle saturé ou insaturé à 5 ou 6 chaînons, substitué ou non.
* Deux radicaux R₂ adjacents peuvent former entre eux et avec les atomes de carbone auxquels ils sont reliés, un radical (hétéro)cyclique aromatique ou non, à 5 ou 6 chaînons, substitué ou non ;
* Deux radicaux R₃ adjacents peuvent former entre eux et avec les atomes de carbone auxquels ils sont rattachés, un cycle aromatique à 5 ou 6 chaînons, substitué ou non ;
* Le radical **R₄**, représente :
   - un atome d'hydrogène
   - un radical alkyle en C₁-C₁₆, éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂, ou leurs combinaisons ; le radical alkyle ne comportant pas de fonction nitro, nitroso, peroxo ou diazo.
   - un radical amino éventuellement substitué par un ou deux radicaux alkyle en C₁-C₄, identiques ou différents, éventuellement porteurs d'au moins un groupement hydroxyle ou alcoxy en C₁-C₂, lesdits radicaux alkyle pouvant éventuellement former avec l'atome d'azote auquel ils sont rattachés, un hétérocycle à 5 ou 6 chaînons saturé ou insaturé ou aromatique éventuellement substitué, comprenant éventuellement au moins un autre hétéroatome identique ou différent de l'azote
   - un radical alkylcarbonylamino (RCO-NR'-) dans lequel le radical R représente un radical alkyle en C₁-C₄ et R' représente un hydrogène, un radical alkyle en C₁-C₄
   - un radical uréido (N(R)₂-CO-NR'-) dans lequel les radicaux R, indépendamment les uns des autres, représentent un radical alkyle en C₁-C₄ et R' représente un hydrogène, un radical alkyle en C₁-C₄.
   - un radical alkylsulfonylamino (RSO₂-NR'-) dans lequel le radical R représente un radical alkyle en C₁-C₄ et R' représente un hydrogène, un radical alkyle en C₁-C₄
   - un radical hydroxycarbonyle
   - un radical alcoxycarbonyle en C₁-C₄
   - un radical cyano
   - un radical phényle éventuellement substitué
   - un radical benzyle éventuellement substitué
* Les radicaux **R₆** et **R₇,** identiques ou différents, représentent :
   - un atome d'hydrogène
   - un radical alkyle en C₁-C₁₆ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂ ou leurs combinaisons ; le radical alkyle ne comportant pas de fonction nitro, nitroso, peroxo ou diazo.
   - un radical (alcoxy)aryle (-Ph-OR) dans lequel le radical R représente un hydrogène, un radical alkyle en C₁-C₄
   - un radical (di-)(alkyl) aminoaryle (-Ph-N(R)₂) dans lequel les radicaux R indépendamment l'un de l'autre représentent un hydrogène, un radical alkyle en C₁-C₄ éventuellement substitué par un hydroxyle
   - un radical alkylsulfonyle (RSO₂-) dans lequel le radical R représente un radical alkyle en C₁-C₄
   - un radical aminocarbonyle, un radical (di)alkyl(C₁-C₄)aminocarbonle
   - éventuellement un radical CH₃CO-
   - un phényle
   - un benzyle éventuellement substitué par un ou plusieurs hydroxy et/ou amino.
* les radicaux R₆ et R₇ peuvent éventuellement former avec l'atome d'azote auquel ils sont rattachés, un hétérocycle comprenant de 5 à 7 chaînons, saturé ou insaturé, éventuellement substitué, comprenant éventuellement un autre hétéroatome identique ou différent de l'azote
* l'un des radicaux R₆ ou R₇ peut également former avec l'atome d'azote auquel il est rattaché et avec un radical R₂ situé en ortho du groupement NR₆R₇, un hétérocycle saturé ou insaturé à 5 ou 6 chaînons substitué ou non
* les radicaux R₆ et R₇ peuvent former avec l'atome d'azote auquel ils sont rattachés et chacun avec un radical R₂ situé en ortho du groupement NR₆R₇, un hétérocycle saturé ou insaturé à 5 ou 6 chaînons substitué ou non
* n est un entier compris entre 0 et 4, lorsque n est inférieur à 4, le ou les atomes de carbone non substitués porte(nt) un atome d'hydrogène
* n' est un entier compris entre 0 et 4, lorsque n' est inférieur à 4, le ou les atomes de carbone non substitués porte(nt) un atome d'hydrogène
* l'électroneutralité des composés de formules (I) et/ou (II) étant assurée par un ou plusieurs anions An cosmétiquement acceptables, identiques ou non.

La présente invention a de même pour objet des compositions tinctoriales comprenant, dans un milieu approprié pour la teinture des fibres kératiniques, de tels composés, à titre de colorants directs.

Elle concerne de plus un procédé de coloration de fibres kératiniques consistant à mettre en contact une composition selon l'invention avec lesdites fibres, sèches ou humides, pendant une durée suffisante pour obtenir l'effet souhaité.

Elle a aussi pour objet un dispositif à plusieurs compartiments comprenant, dans un premier compartiment, la composition selon l'invention, et dans un second compartiment, une composition oxydante.

Un dernier objet de l'invention est enfin constitué par plusieurs procédés de préparation des composés selon l'invention.

On a constaté que les composés à fonction hydrazone tels que définis précédemment, permettaient d'élargir la palette de couleur et présentaient une bonne ténacité vis-à-vis d'agents extérieurs comme notamment les shampooings, et cela, même lorsque la fibre kératinique est sensibilisée. Ils sont également stables en conditions alcalines éclaircissantes.

Par ailleurs ces composés peuvent constituer des intermédiaires de synthèse pour de colorants comprenant plusieurs chromophores reliés entre eux par un ou plusieurs bras de liaison.

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine.

Au sens de la présente invention, et à moins qu'une indication différente ne soit donnée :
- Un radical alkyle est linéaire ou ramifié,
- Un radical alkyle ou la partie alkyle, d'un radical est dite 'substituée' lorsqu'elle comprend au moins un substituant choisi parmi les groupements :
   - hydroxyle,
   - alcoxy en C₁-C₄, (poly)hydroxyalcoxy en C₂-C₄,
   - amino éventuellement substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.

De préférence, les radicaux alkyle ne portent pas de groupement alkyle-SO₂-O-, aryle-SO₂-O- avec un groupement aryle substitué par un radical alkyle.
- Un radical aryle ou hétéroaryle ou la partie aryle ou hétéroaryle d'un radical est dite 'substituée' lorsqu'elle comprend au moins un substituant porté par un atome de carbone, choisi parmi
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyles, identiques ou différents, en C₁-C₄, éventuellement porteurs d'au moins un groupement hydroxyle ou, les deux radicaux pouvant former avec l'atome d'azote auquel ils sont rattachés, un hétérocycle à 5 ou 7 chaînons, de préférence de 5 ou 6 chaînons, comprenant éventuellement un autre hétéroatome identique ou différent de l'azote ;
   - un atome d'halogène tel que chlore, fluor ou brome ;
   - un groupement hydroxyle
   - un radical alcoxy en C₁-C₂ ; un radical (poly)-hydroxyalcoxy en C₂-C₄ ;
   - un radical amino éventuellement substitué par un ou deux radicaux alkyles, identiques ou différents, en C₁-C₄ éventuellement porteurs d'au moins un groupement hydroxyle ou amino par deux radicaux alkyles en C₁-C₂ éventuellement substitués ;
   - un radical alkylcarbonylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ;
   - un radical (di-)(alkyl-)aminocarbonyl ((R)₂N-CO-) dans lequel les radicaux R, identiques ou non, représentent un atome d'hydrogène, un radical alkyle en C₁-C₄ éventuellement porteur d'au moins un groupement hydroxyle ;
   - un radical alkylsulfonylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ;
   - un radical aminosulfonyle ((R)₂N-SO₂-) dans lequel les radicaux R, identiques ou non, représentent un atome d'hydrogène, un radical alkyle en C₁-C₄ éventuellement porteur d'au moins un groupement hydroxyle.
- La partie cyclique ou hétérocyclique d'un radical non aromatique ou un radical (hétéro-)cyclique est dit substitué lorsqu'il comprend au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.

Comme indiqué auparavant, un premier objet de l'invention consiste en des composés correspondant aux formules (I) et/ou (II) précitées.

Selon un mode de réalisation particulier de l'invention, le radical R₁ représente :
- un hydrogène
- un radical alkyle en C₁-C₆, éventuellement substitué par au moins un groupement hydroxy, par au moins un groupement alkoxy en C₁-C₂, par au moins un groupement un groupement hydroxycarbonyle, éventuellement par au moins un groupe amino substitué ou non par un ou deux radicaux alkyle identiques ou non en C₁-C₂
- un radical phényle éventuellement substitué par au moins un atome d'halogène tels que le chlore, le brome, l'iode, le fluor ; par au moins un groupement hydroxyle
- un radical benzyle éventuellement substitué par un ou plusieurs hydroxy et/ou amino
- un radical alkyl(C₁-C₄)carbonyle
- un radical alkyl(C₁-C₄)sulfonyle.

De préférence, le radical R₁ représente un atome d'hydrogène ; un radical méthyle, un radical 2-hydroxyéthyle, un radical 3-methoxypropyle, un radical (di-) (methyl)aminoéthyle, un radical CH₃CO-, un radical CH₃SO₂-, un radical phényle, un radical 4-chlorophényl, un radical benzyle éventuellement substitué par un ou deux groupement(s) hydroxy, amino ou leur combinaison.

Conformément à une variante encore plus particulière de l'invention, R₁ représente un atome d'hydrogène ; un radical méthyle ; un radical 2-hydroxyéthyle ; un radical CH₃CO-, un radical CH₃SO₂- ; un radical benzyle non substitué.

Conformément à une variante plus précise de l'invention, les radicaux R₅, identiques ou non, représentent
- un radical alkyle en C₁-C₁₆, de préférence en C₁-C₈, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, SO₂ ou leurs combinaisons ; le radical alkyle ne comportant pas de fonction nitro, nitroso, peroxo ou diazo.
- un radical triméthylsilylalkyle en C₁-C₄
- un radical phényle éventuellement substitué.
- un radical benzyle éventuellement substitué.

De préférence, les radicaux R₅, identiques ou différents, représentent les radicaux méthyle, éthyle, propyle, butyle, pentyle, hexyle, heptyle, octyle, 2-hydroxyéthyle, 3-hydroxypropyle, 6-hydroxyhexyle, 2-methoxyéthyle, 3-methoxypropyle, 3-trimethylsilylpropyle, 2-((di-)methyl)aminopropyle, 3-((di-)methyl)aminopropyle, phényle; 4-chlorophényle, benzyle, 3,4-dihydroxybenzyle.

Selon une variante encore plus particulière de l'invention, les radicaux R₅, identiques ou différents, représentent les radicaux méthyle, éthyle, propyle, butyle, 3-trimethylsilylpropyle, 4-chlorophényle, benzyle.

Plus particulièrement, les radicaux R₂, R₃, identiques ou différents, représentent :
- un atome d'halogène choisi parmi le brome, le chlore, le fluor, l'iode
- un radical alkyle en C₁-C₁₆ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂, ou leurs combinaisons ; le radical alkyle ne comportant pas de fonction nitro, nitroso, peroxo ou diazo
- un radical hydroxyle
- un radical alcoxy en C₁-C₄ ; un groupement (poly)hydroxyalcoxy en C₂-C₄
- un radical alcoxy(C₁-C₄)carbonyle
- un radical alkyl(C₁-C₄)carbonyloxy
- un radical aryloxy éventuellement substitué
- un radical amino éventuellement substitué par un ou deux radicaux alkyle en C₁-C₄, identiques ou différents, éventuellement porteurs d'au moins un groupement hydroxyle ou alcoxy en C₁-C₂, lesdits radicaux alkyle pouvant éventuellement former avec l'atome d'azoté auquel ils sont rattachés, un hétérocycle à 5 ou 7 chaînons, saturé ou insaturé, éventuellement substitué, éventuellement aromatique, comprenant éventuellement un autre hétéroatome identique ou différent de l'azote ; par un ou deux radicaux phényle, aminophényle, N,N-diethylaminophényl ou méthoxyphényle
- un radical alkyl(C₁-C₄)carbonylamino dans lequel la fonction amino est substituée ou non par un radical alkyle en C₁-C₄
- un radical aminocarbonyle, un groupement (di)alkyl(C₁-C₄)aminocarbonyle
- un radical uréido substitué ou non par un ou plusieurs radicaux alkyle en C₁-C₄
- un radical aminosulfonyle, (di)alkyl(C₁-C₄)aminosulfonyle
- un radical alkyl(C₁-C₄)thio
- un radical alkyl(C₁-C₄)sulfonylamino dans lequel la fonction amino est substituée ou non par un radical alkyle en C₁-C₄
- un radical cyano
- un radical phényl
- un radical trifluorométhyle
- un radical thio
- un radical alkyl(C₁-C₄)sulfinyle
- un radical alkyl(C₁-C₄)sulfonyle.

De façon préférée, les radicaux R₂, R₃, identiques ou différents, représentent :
- un atome d'halogène choisi parmi le chlore, le fluor
- un radical alkyle en C₁-C₈ éventuellement substitué par un ou plusieurs hydroxy ou ((di-)alkyl)amino ou sulfonylamino, éventuellement interrompu par un ou plusieurs hétéroatomes et/ou par un ou plusieurs groupements comprenant au moins un hétéroatome de préférence choisi parmi l'oxygène, le soufre, SO, SO₂, ou leurs combinaisons
- un radical hydroxyle
- un radical alcoxy en C₁-C₄
- un radical alcoxy(C₁-C₄)carbonyle
- un radical aryloxy éventuellement substitué
- un radical amino éventuellement substitué :
   - par un ou deux radicaux alkyle en C₁-C₄, identiques ou différents, éventuellement porteurs d'au moins un groupement hydroxyle ou alcoxy en C₁-C₂,
   - par un ou deux radicaux phényle, aminophényle ou méthoxyphényle
- un radical alkyl(C₁-C₄)carbonylamino dans lequel la fonction amino est substituée ou non par un radical alkyle en C₁-C₄
- un radical aminocarbonyle
- un radical aminosulfonyle, (di)alkyl(C₁-C₄)aminosulfonyle
- un radical alkyl(C₁-C₄)thio
- un radical phényle
- un radical trifluorométhyle
- un radical thio.

Plus particulièrement, les radicaux R₂, R₃, identiques ou différents, représentent :
- un atome de chlore
- un radical alkyle en C₁-C₃, éventuellement substitué par un groupement hydroxy
- un radical hydroxyle
- un radical alcoxy en C₁-C₄
- un radical alcoxy(C₂-C₃)carbonyle
- un radical phényloxy
- un radical amino éventuellement substitué :
   - par un ou deux radicaux alkyle en C₁-C₄, de préférence en C₁-C₂, identiques ou différents, éventuellement porteurs d'au moins un groupement hydroxyle ou alcoxy en C₁-C₂,
   - par un ou deux radicaux phényle, aminophényle ou méthoxyphényle
- un radical alkyl(C₁-C₂)carbonylamino dans lequel la fonction amino est substituée ou non par un radical alkyle en C₁-C₄.

De préférence, les radicaux R₂, R₃, identiques ou différents, représentent :
- un atome de chlore ou de fluor
- un méthyle, un éthyle, un propyle, un butyle, un pentyle, un hexyle
- un 2-hydroxyéthyle, 3-hydroxypropyle, 6-hydroxyhexyle
- un hydroxyle
- un methoxy, un ethoxy, un propoxy, un butoxy
- un hydroxycarbonyle
- un methoxycarbonyle, un ethoxycarbonyle
- un phenyloxy
- un phenylamino
- un amino
- un (di-)methylamino, un (di-)ethylamino
- un aminophényleamino ou méthoxyphényleamino
- un radical 4-N,N-diéthylaminophénylamino
- un methylcarbonylamino
- un aminocarbonyle
- un aminosulfonyle, dimethylaminosulfonyle
- un radical methylthio
- un radical phényle
- un radical trifluorométhyle
- un radical thio.

Selon un autre mode de réalisation particulier de l'invention, les composés de formule (I) et/ou (II) sont tels que deux radicaux R₂ adjacents forment entre eux et avec les atomes de carbone auxquels ils sont reliés, un radical cyclique, éventuellement aromatique comprenant 5 ou 6 chaînons, substitué ou non.

Selon ce mode de réalisation particulier, deux radicaux R₂ adjacents forment entre eux et avec les atomes de carbone auxquels ils sont reliés, un radical cyclique aromatique comprenant 6 chaînons, non substitué.

Selon un autre mode de réalisation, deux radicaux R₃ adjacents peuvent former entre eux et avec les atomes de carbone auxquels ils sont rattachés, un cycle aromatique à 6 chaînons, substitué ou non. De préférence, si ledit cycle aromatique est substitué, il l'est par un ou plusieurs radicaux choisis parmi l'hydroxyle, l'amino, le diéthylamino, l'hydroxycarbonyle, le methoxycarbonyle, le chlore, le methyle, l'éthyle, le methoxy.

Selon ce mode de réalisation particulier, deux radicaux R₃ adjacents forment entre eux et avec les atomes de carbone auxquels ils sont reliés, un radical cyclique aromatique comprenant 6 chaînons, non substitué.

Avantageusement, le radical R₄ représente plus particulièrement :
- un atome d'hydrogène
- un radical alkyle en C₁-C₈, éventuellement substitué, de préférence en C₁-C₄ non substitué
- un radical hydroxycarbonyle
- un radical alcoxycarbonyle en C₁-C₂
- un radical phényle
- un radical benzyle éventuellement substitué par un ou deux groupement(s) hydroxy, amino ou leur combinaison.

De préférence, le radical R₄ représente un atome d'hydrogène, un radical méthyle, un radical méthoxycarbonyle, éventuellement un radical hydroxycarbonyle, un radical phényle, un radical benzyle éventuellement substitué par un ou deux hydroxy ou amino ou leur combinaison, et encore plus particulièrement un atome d'hydrogène, un radical méthyle, un radical méthoxycarbonyle, un radical hydroxycarbonyle, un radical benzyle non substitué.

En ce qui concerne les radicaux R₆ et R₇, ces derniers, identiques ou non, représentent plus particulièrement :
- un atome d'hydrogène
- un radical alkyle en C₁-C₈ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂ ou leurs combinaisons ; le radical alkyle ne comportant pas de fonction nitro, nitroso, peroxo ou diazo.
- un phényle éventuellement substitué par un ou plusieurs radicaux alcoxy en C₁-C₄ et/ou par un ou plusieurs radicaux (di-)(alkyl (C1-C4)-)amino.
- un benzyle éventuellement substitué par un ou plusieurs hydroxyle et/ou amino
- un radical aminocarbonyle, un radical (di)alkyl(C₁-C₄)aminocarbonyle
- éventuellement un radical CH₃CO-.

De préférence, les radicaux R₆ et R₇, identiques ou non, représentent :
- Un atome d'hydrogène
- Un méthyle, un éthyle, un 2-hydroxyéthyle, 3-hydroxypropyle, un 3-methoxypropyle
- Un phényle
- Un benzyle éventuellement substitué par un ou deux hydroxyles et/ou amino
- Un methoxyphényle
- Un aminophényle
- Un (4-N,N-)diéthylaminophényle
- éventuellement un radical CH₃CO-.

En ce qui concerne n, il s'agit d'un nombre entier compris entre 0 et 4, lorsque n est inférieur à 4, le ou les atomes de carbone non substitués porte(nt) un atome d'hydrogène. De préférence, n est compris entre 0 et 2.

Pour ce qui a trait à n', ce coefficient est un nombre entier compris entre 0 et 4, lorsque n' est inférieur à 4, le ou les atomes de carbone non substitués porte(nt) un atome d'hydrogène. De préférence n' est compris entre 0 et 2.

Conformément à une variante intéressante de l'invention, les composés de formule (I) et/ou (II) sont tels que les radicaux R₆ et R₇, notamment dans le cas de radicaux alkyle tels que définis auparavant, forment avec l'atome d'azote auquel ils sont rattachés, un hétérocycle à 5, 6 ou 7 chaînons, de préférence saturé, comprenant éventuellement un autre hétéroatome identique ou différent de l'azote, tel que de préférence l'oxygène.

A titre d'exemple de tel groupement, on peut citer les cycles pyrrolidine, pipéridine, pipérazine, morpholine, homopipérazine, homopipéridine.

Selon une seconde variante avantageuse de l'invention, les composés de formule (I) et/ou (II) sont tels que l'un des radicaux R₆ ou R₇ peut également former avec l'atome d'azote auquel il est rattaché et avec un radical R₂ situé en ortho du groupement NR₆R₇, un hétérocycle saturé ou insaturé à 5 ou 6 chaînons substitué, ou non, plus particulièrement par un groupement hydroxyle ou méthoxy ;

Par exemple, le groupement -NR₆R₇, avec le noyau aromatique éventuellement substitué par un hydroxyle peut correspondre aux composés suivants :

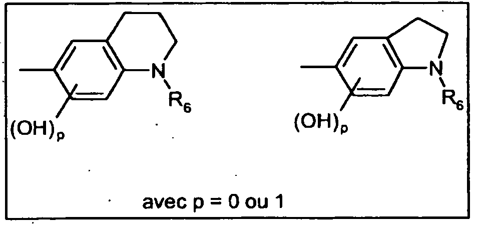

Selon une troisième variante avantageuse de l'invention, les composés de formule **(I)** et/ou (II) sont tels que les deux radicaux R₆ et R₇ peuvent former avec l'atome d'azote auquel ils sont rattachés et chacun avec un radical R₂ situé en ortho du groupement NR₆R₇. un hétérocycle saturé ou insaturé à 5 ou 6 chaînons substitué ou non, plus particulièrement par un groupement hydroxyle ou méthoxy.

Par exemple, le groupement -NR₆R₇, avec le noyau aromatique éventuellement substitué par un hydroxyle peut correspondre aux composés suivants :

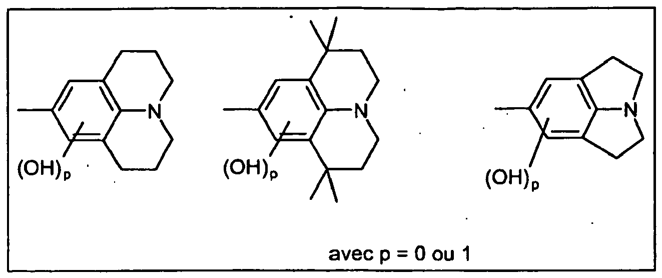

Dans les formules des composés de formules (I) et/ou (II), An représente un anion ou un mélange d'anions, organiques ou minéraux permettant d'équilibrer la ou les charges des composés de formule (I) et/ou (II), par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en C₁-C₆, comme l'ion méthylsulfate ou éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyles tel que le mésylate.

Les sels d'addition avec un acide des composés de formules (1) et/ou (II), peuvent être à titre d'exemple les sels d'addition avec un acide organique ou minéral tel que l'acide chlorhydrique, l'acide bromhydrique, l'acide sulfurique ou les acides (alkyl- ou phényl-) sulfoniques tels que l'acide p-toluènesulfonique ou l'acide méthylsulfonique.

Les solvates des composés de formules (I) et/ou (II), représentent les hydrates de tels composés et/ou l'association d'un composé de formule (I) et/ou (II), avec un alcool linéaire ou ramifié en C₁-C₄ tels que le méthanol, l'éthanol, l'isopropanol, le n-propanol.

Selon un mode de réalisation particulier, les composés de formule (I) et/ou (II) sont représentés par les formules suivantes, ainsi que leurs formes résonnantes et/ou leurs sels d'addition avec un acide et/ou leurs solvates : 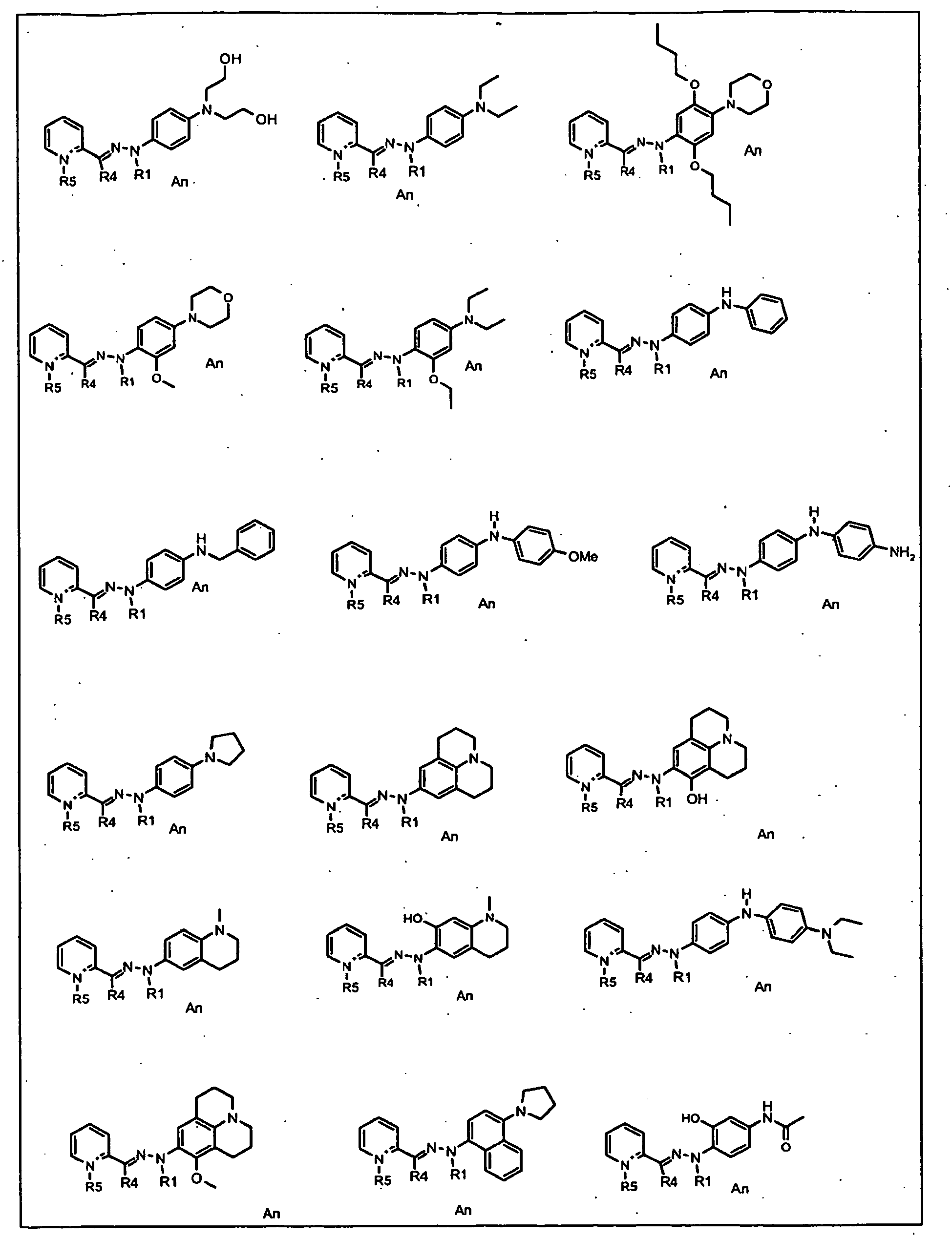 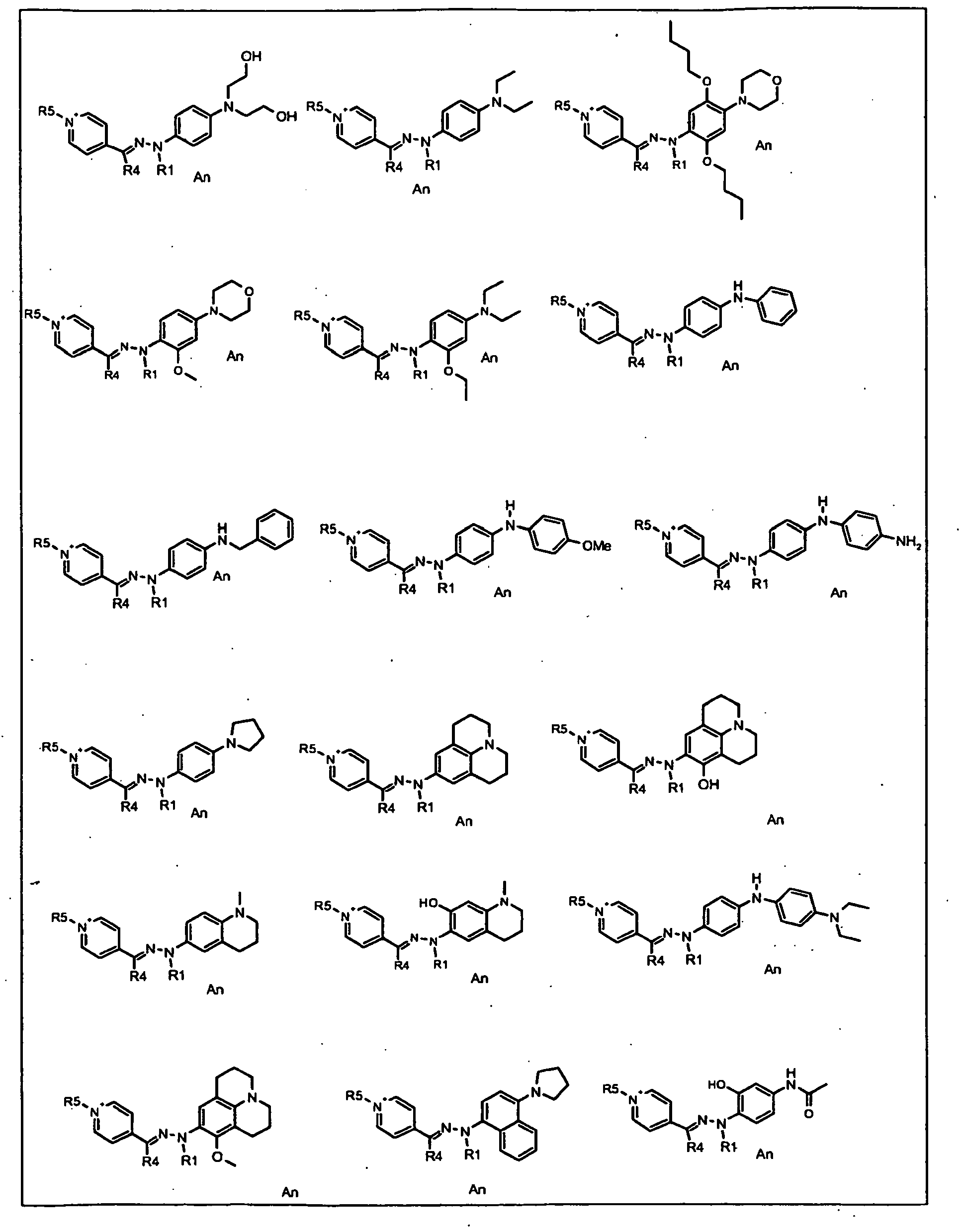 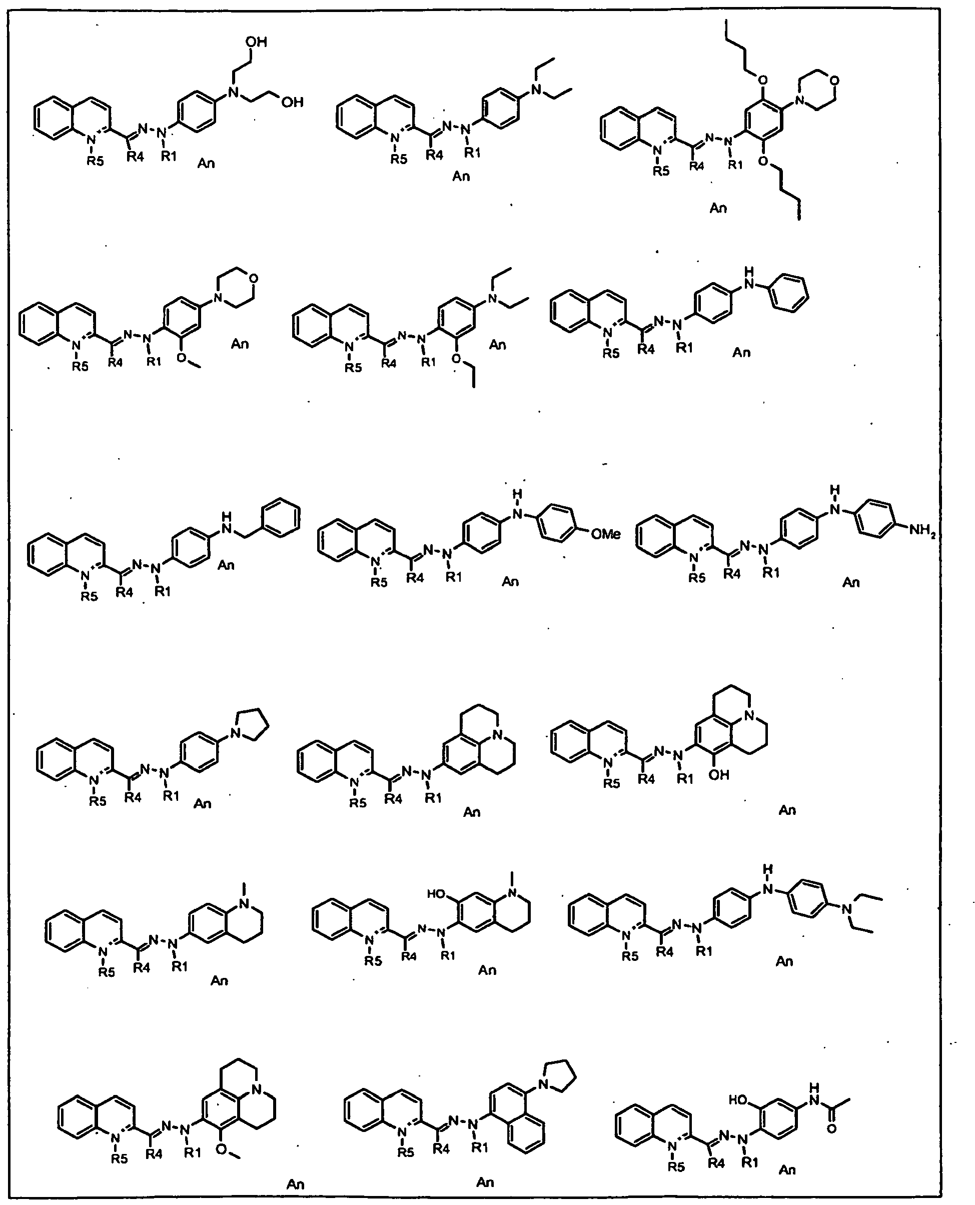 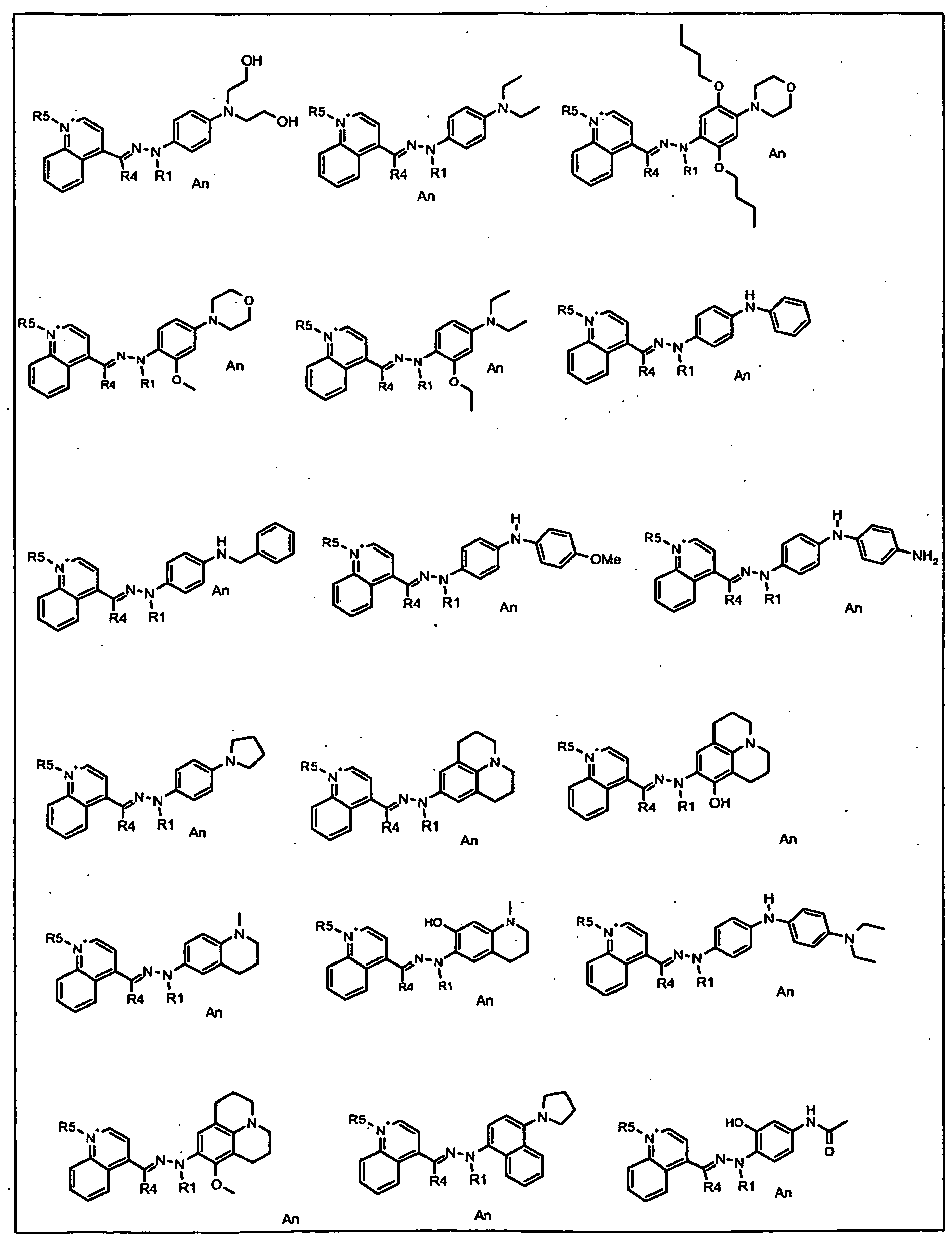 dans lesquelles les radicaux R₁, R₄, R₅, An sont tels que définis précédemment.

De manière particulière, les colorants monochromophoriques monocationiques particuliers de type hydrazone de formule particulière comprenant un motif 2- ou 4-pyridinium ou 2- ou 4-quinolinium de formules (I) et/ou (II) selon la présente invention, sont représentés par les composés suivants : 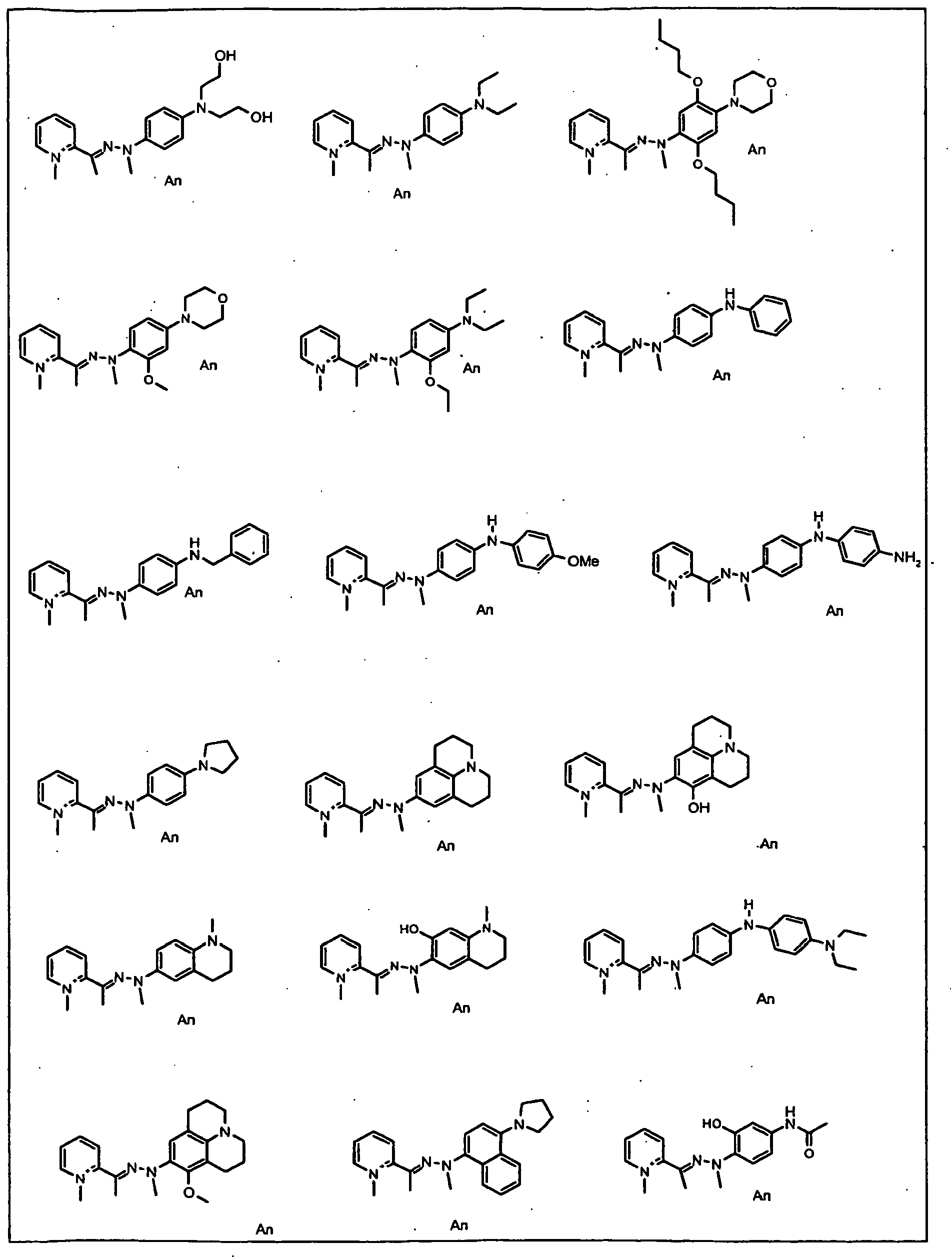 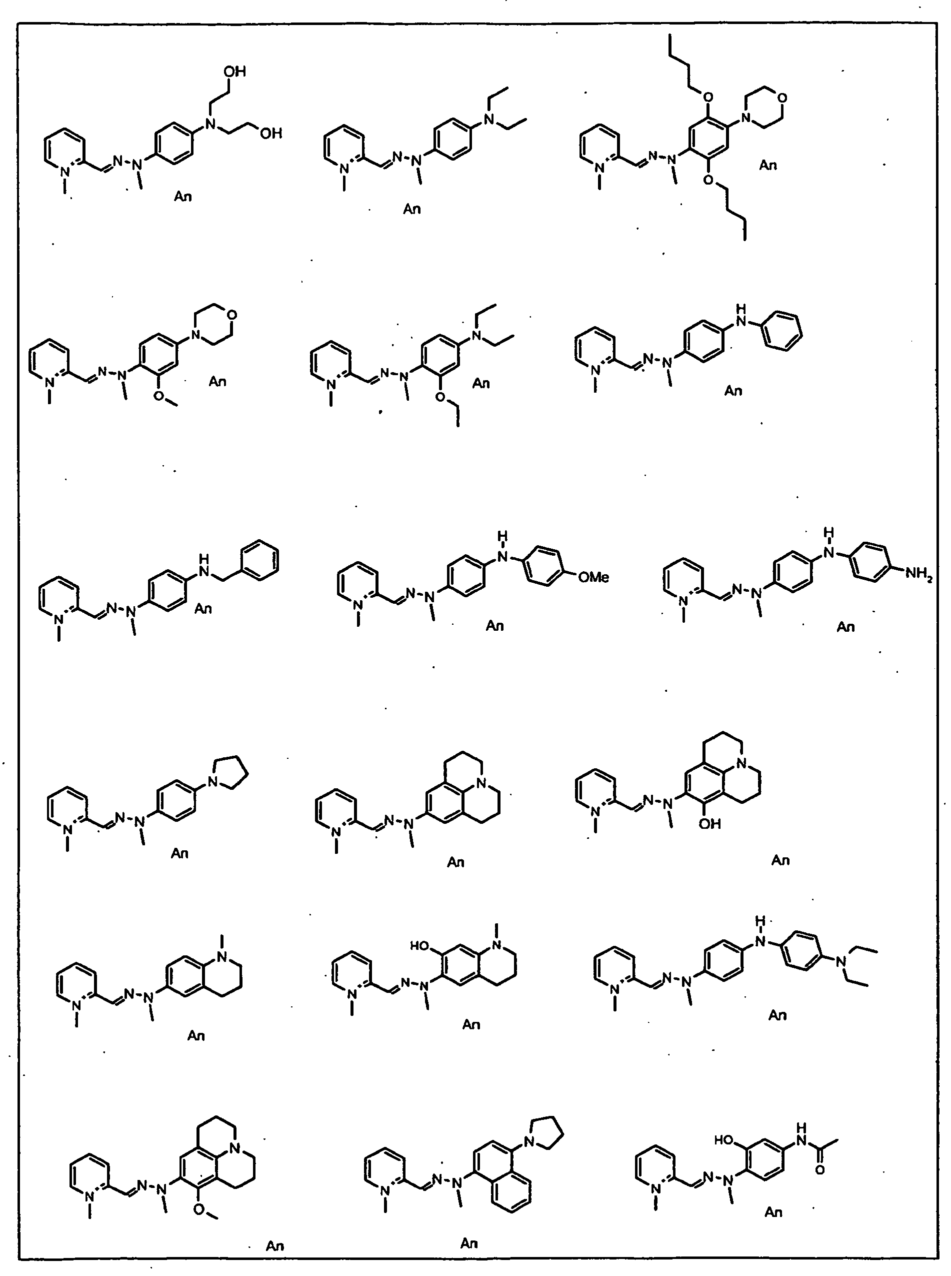 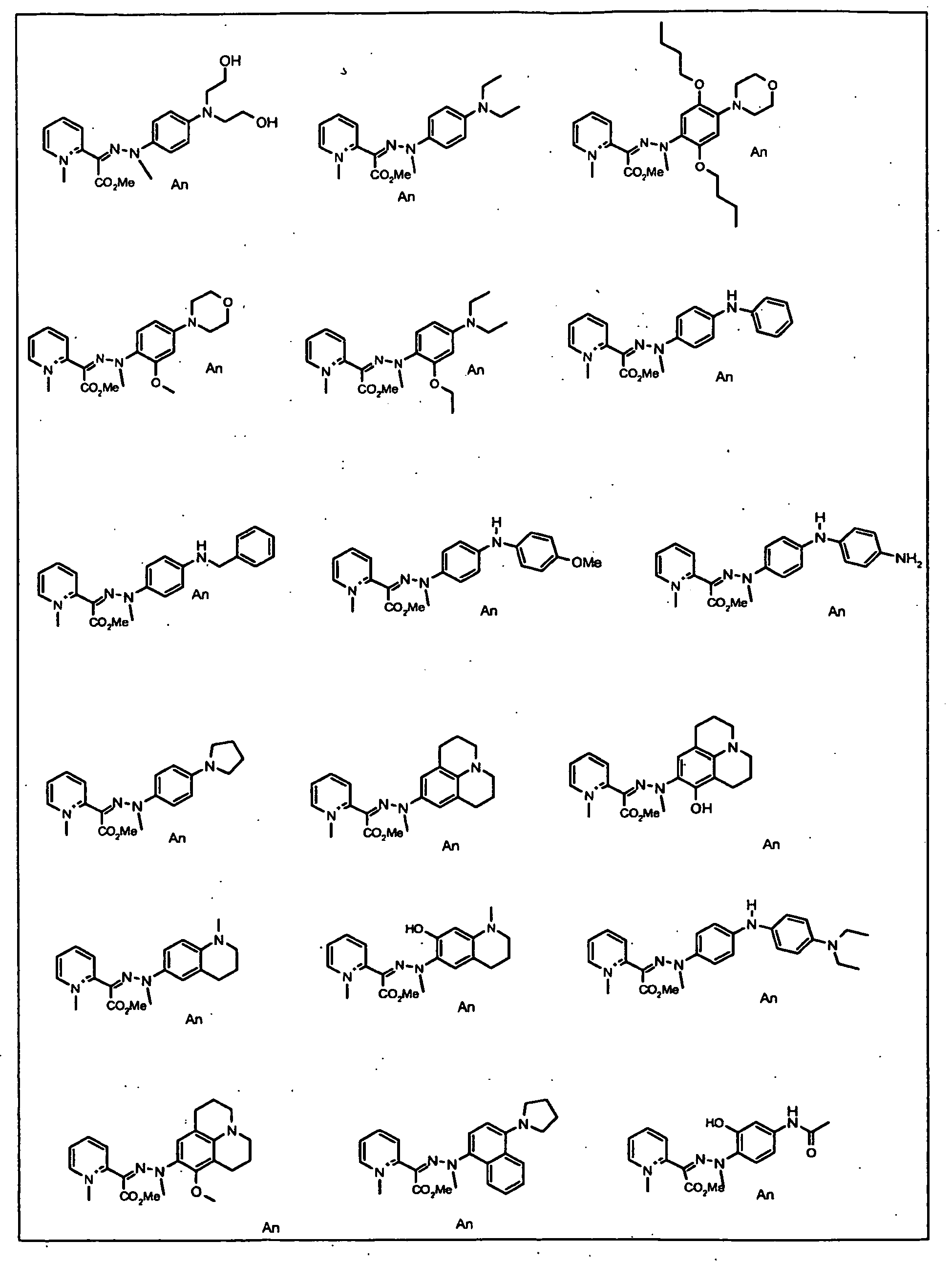 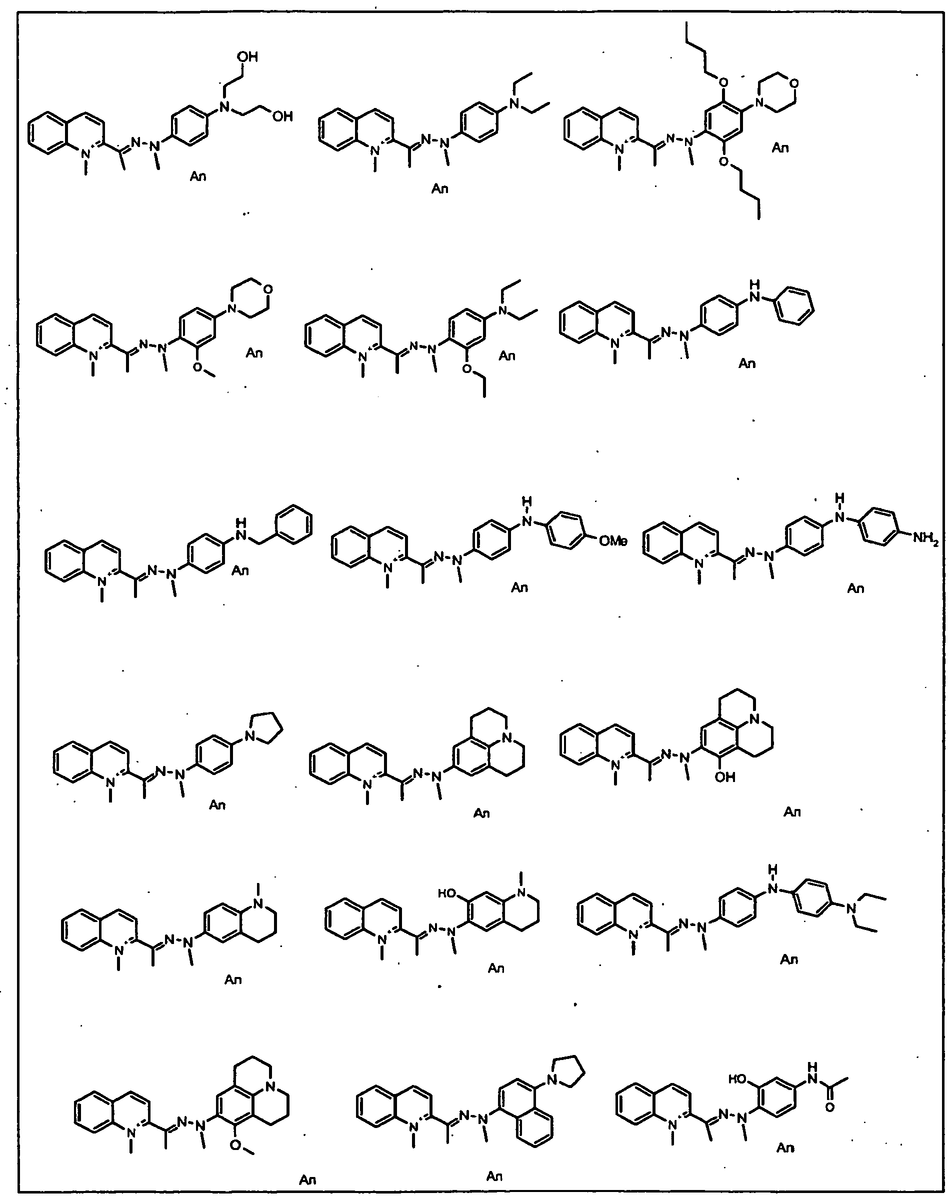 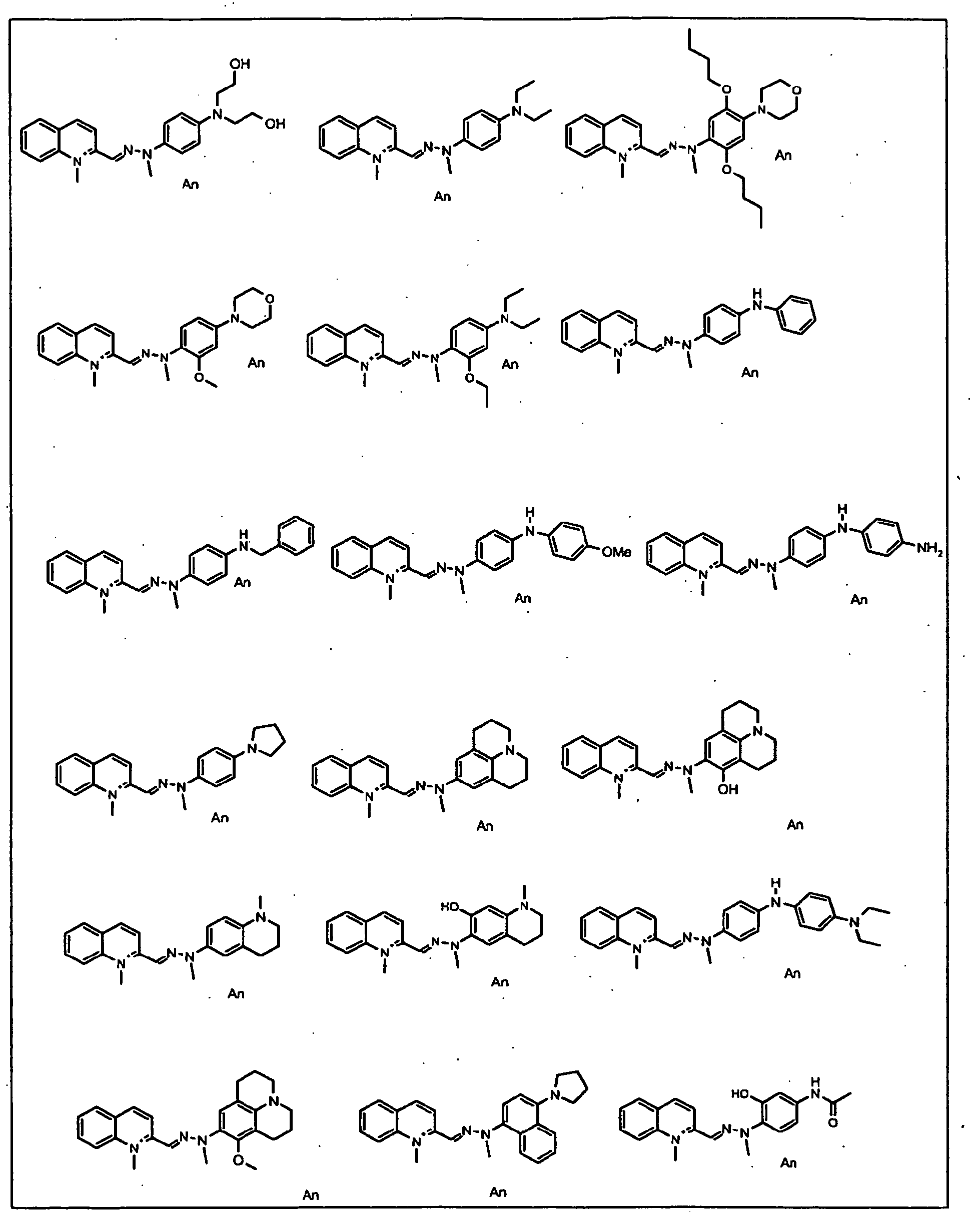 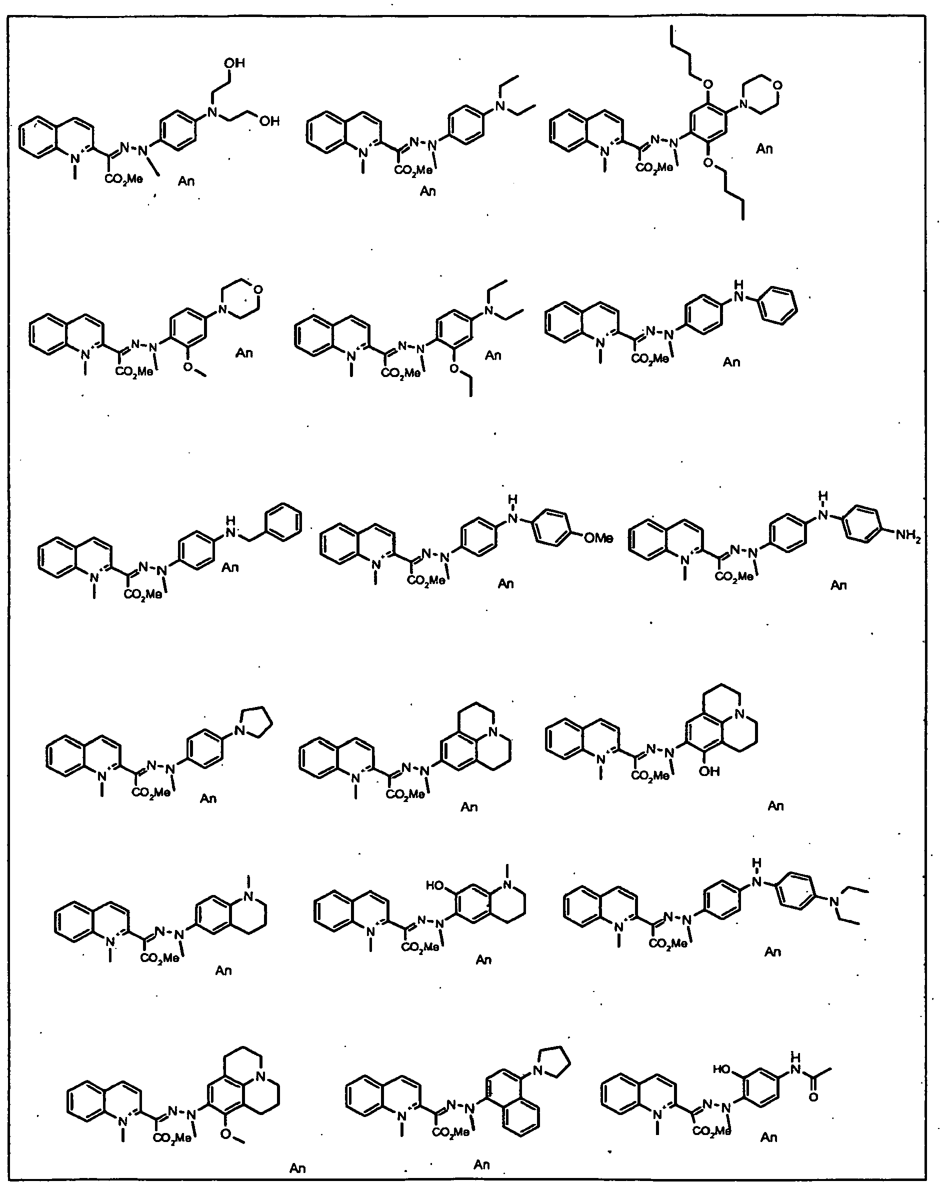 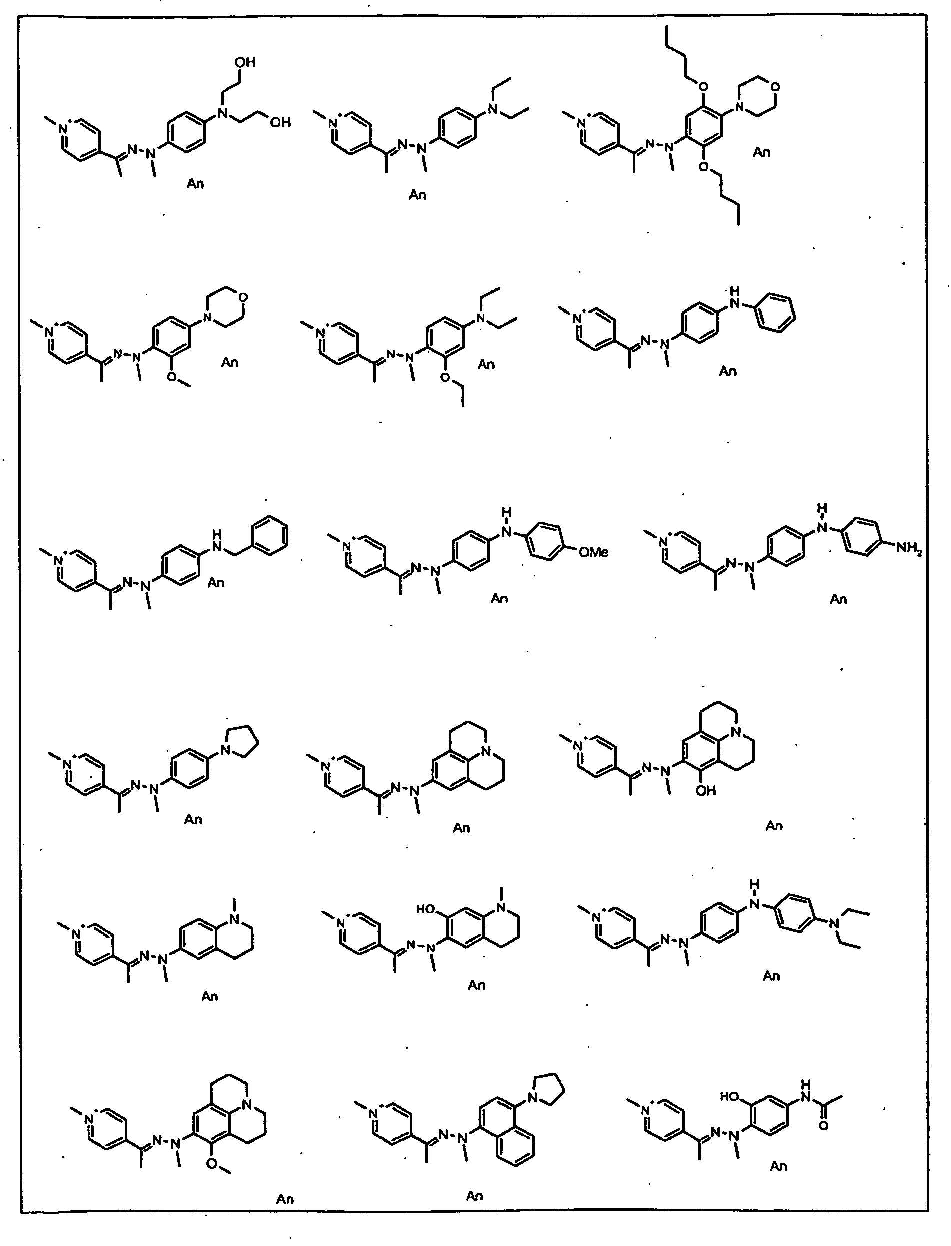 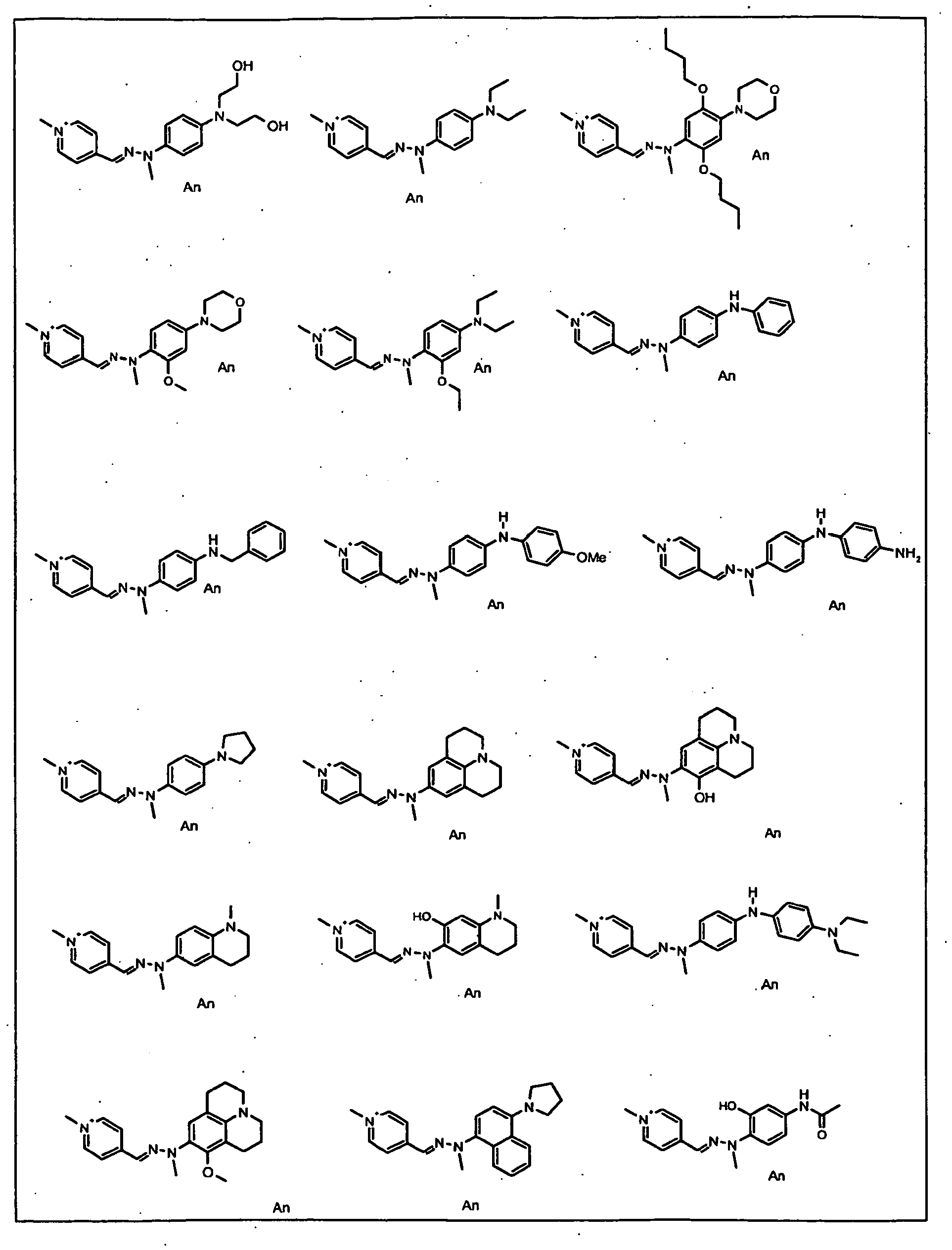 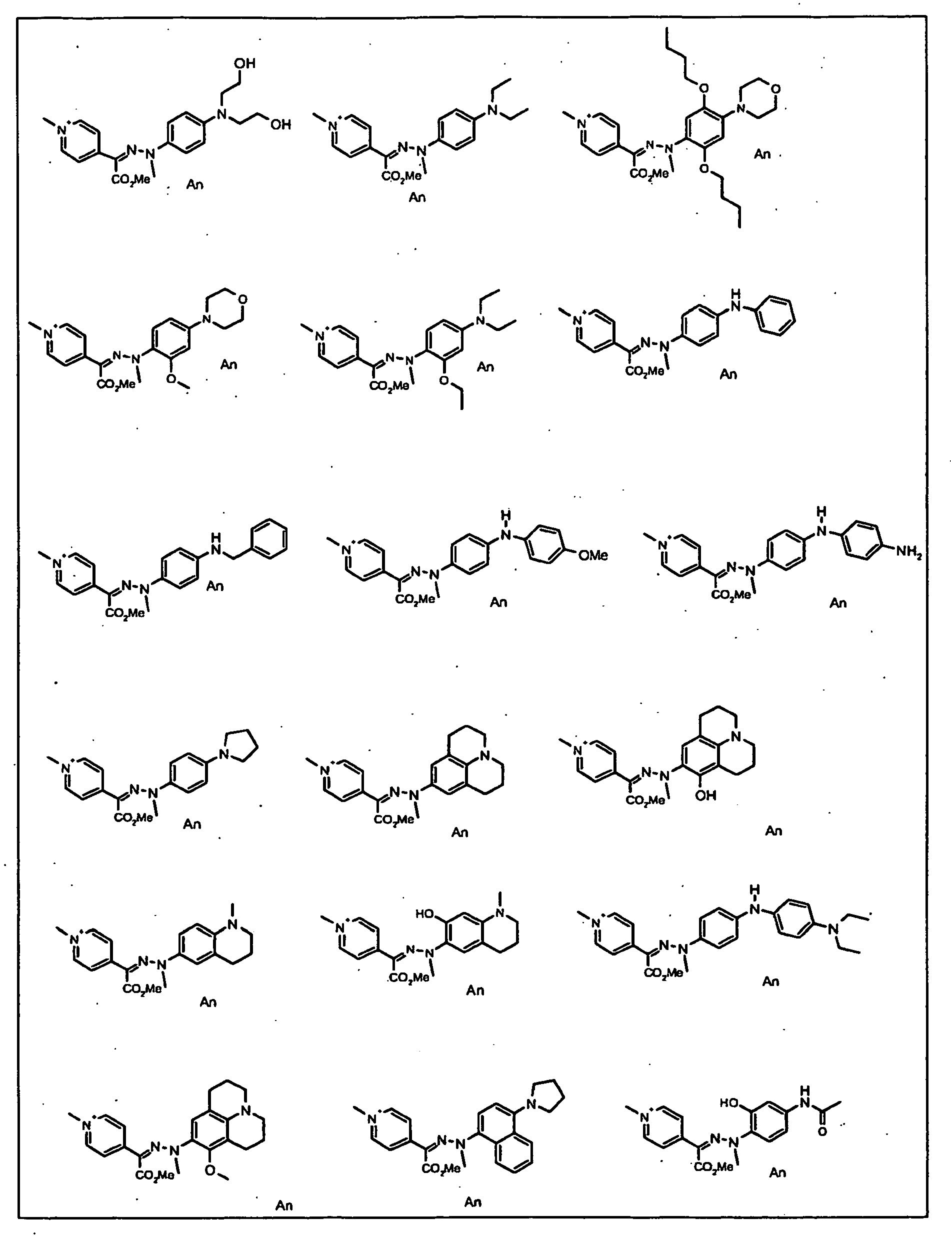 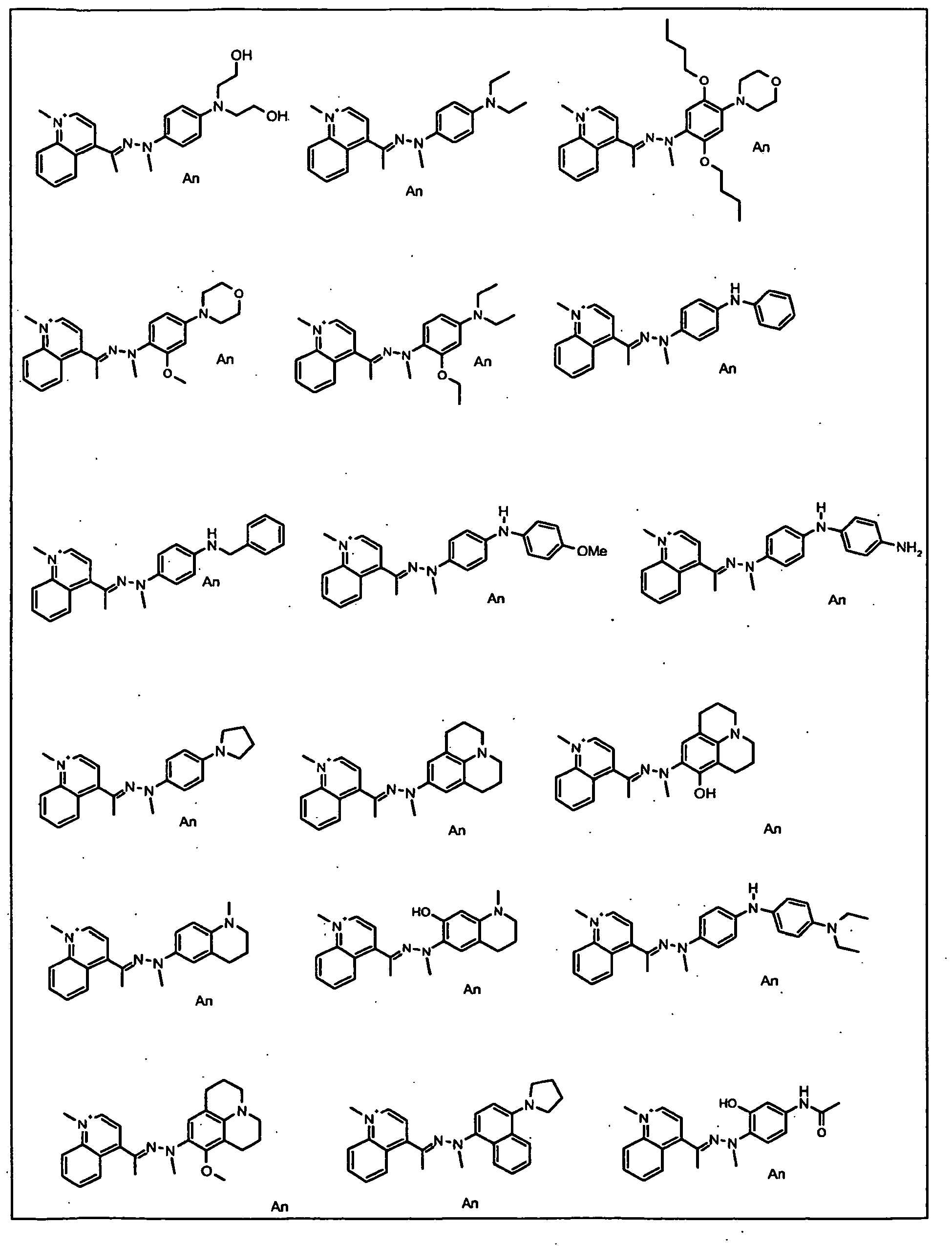 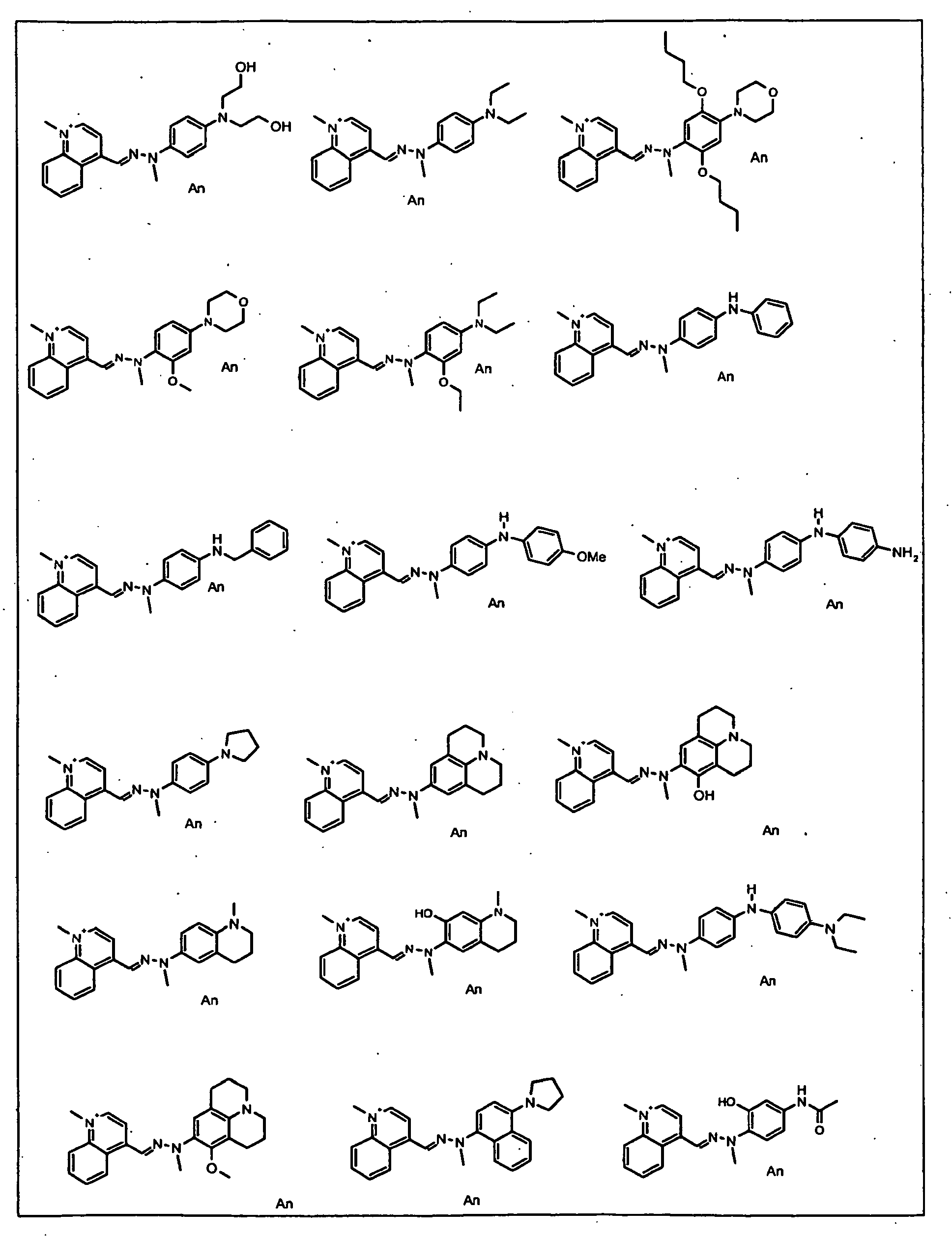 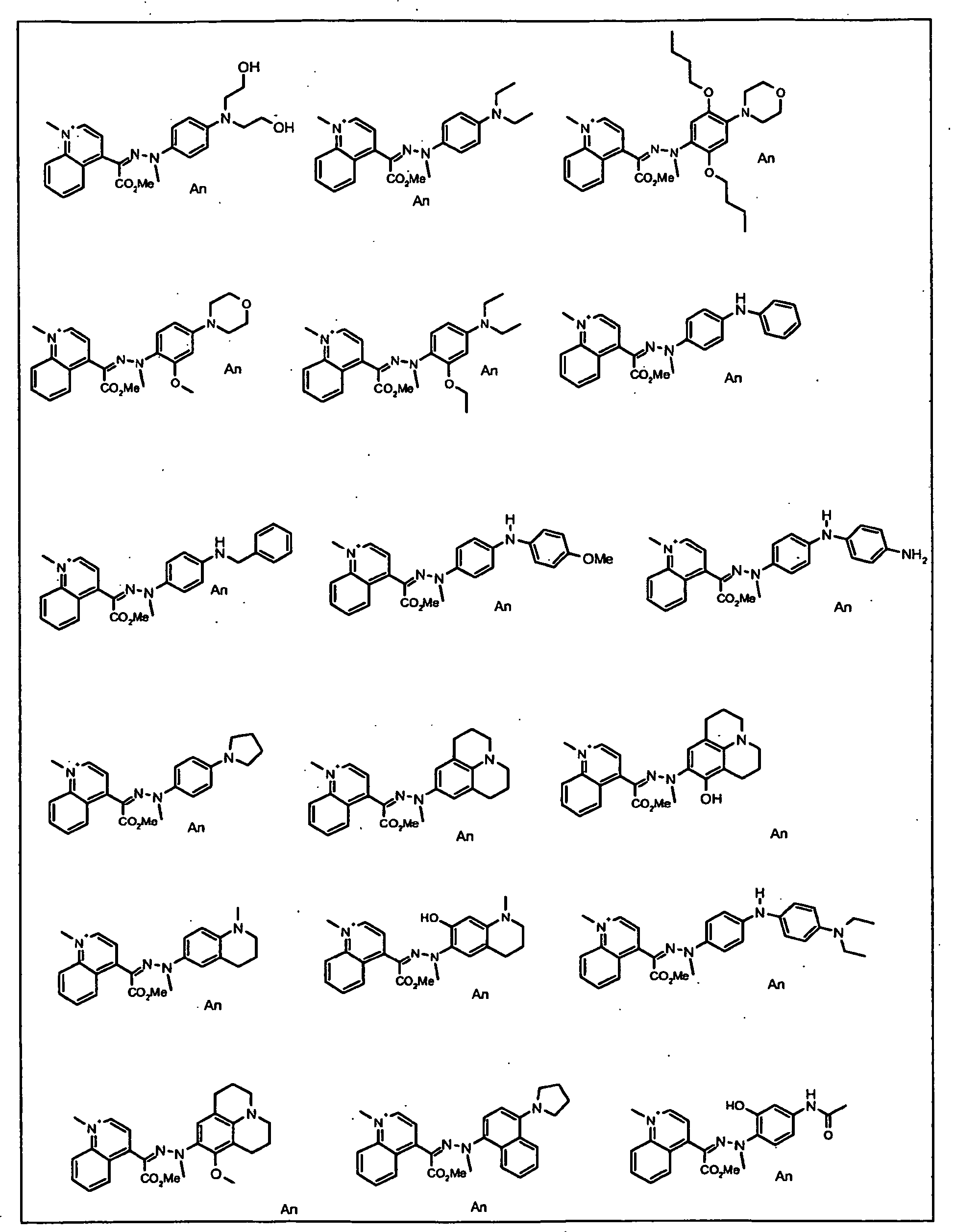 dans lesquelles An est tel que défini précédemment.

Un autre objet de l'invention concerne des procédés de préparation des composés qui viennent d'être décrits.

Selon un premier mode de réalisation, les composés de formules (I) et/ou (II) peuvent être obtenus en mettent en oeuvre les étapes décrites ci-dessous :
(a) Condensation d'un aldéhyde ou d'une cétone hétéroaromatique avec un dérivé d'hydrazine : 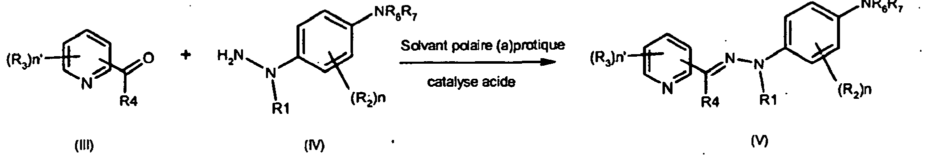
(b) Quaternisation du noyau hétérocyclique :

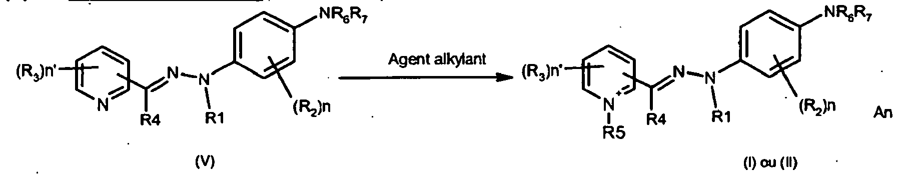

Selon ce procédé, on effectue une première étape de condensation d'un dérivé d'hydrazine en présence d'un aldéhyde ou d'une cétone hétéroaromatique de manière connue de l'homme du métier (voir à titre d'exemple le brevet GB924601).

Habituellement cette réaction a lieu à une température comprise entre -20°C et 120°C de préférence entre 0°C et 70°C.

De manière classique, la réaction a lieu dans un solvant approprié comme par exemple l'eau, les alcools tels que le méthanol, l'éthanol, le propanol ; la diméthylformamide, le tétrahydrofuranne, la N-méthylpyrrolidone, le 1,3-diméthyl-2-oxohexahydropyrimidine ou leur mélange:

La réaction a habituellement lieu en présence d'une quantité catalytique d'acide choisi parmi les acides organiques tels que l'acide acétique, l'acide propionique, l'acide paratoluènesulfonique ou les acides inorganiques tels que l'acide sulfurique, l'acide chlorhydrique.

Une fois la réaction réalisée, on effectue dans une seconde étape une réaction de quaternisation.

Habituellement cette réaction est mise en oeuvre dans un solvant qui peut être celui de l'étape précédente ou dans un autre solvant organique approprié tel que l'acétate d'éthyle, le dichlorométhane, le 1,2-dichloroéthane, le toluène,....

L'agent alkylant R₅X peut être un halogénure d'alkyle comme par exemple l'iodure de méthyle, le 1-bromopropyle, le 3-chlorohexane ou bien un alkyl- ou arylsulfonate comme par exemple le méthyl 4-toluènesulfonate ou le benzyl 4-toluènesulfonate, ou bien un dialkylsulfate comme par exemple, le diméthylsulfate, le diéthylsulfate ou le dipropylsulfate.

La température est classiquement comprise entre 0 et 100°C, de préférence entre 20°C et 70°C.

A titre d'exemple, on peut mettre en oeuvre ce premier procédé pour la préparation du composé suivant :

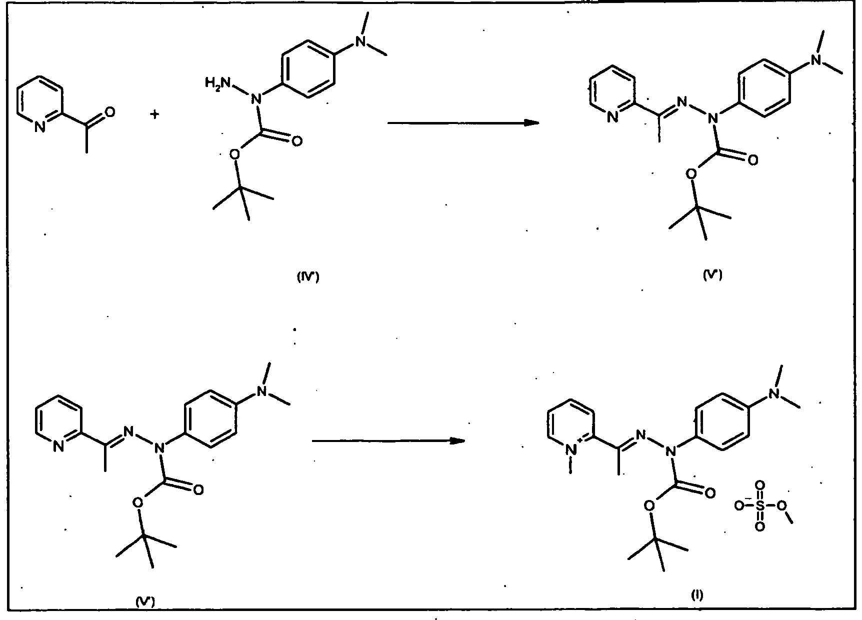

Il est à noter que le composé (IV') représenté ci-dessus peut être obtenu en utilisant le protocole opératoire décrit dans Org. Lett., 3, 23, 2001, 3803-3806 ou WO99/43643.

Selon un deuxième mode de réalisation, les composés de formules (I) et/ou (II) peuvent être obtenus en mettent en oeuvre les étapes décrites ci-dessous :
(a) Condensation d'un aldéhyde ou d'une cétone hétéroaromatique avec un dérivé d'hydrazine 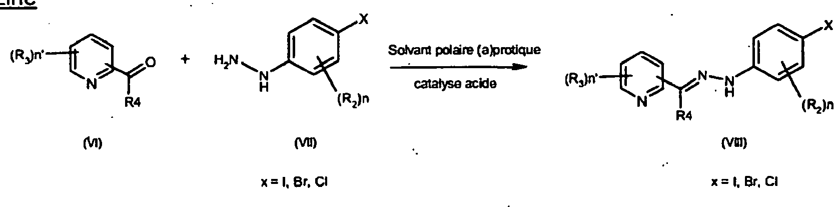
(b) Réaction de quaternisation du noyau hétérocyclique 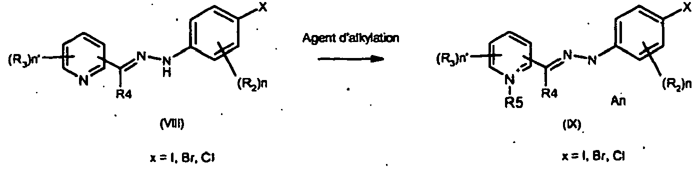
(c) Réaction d'amination 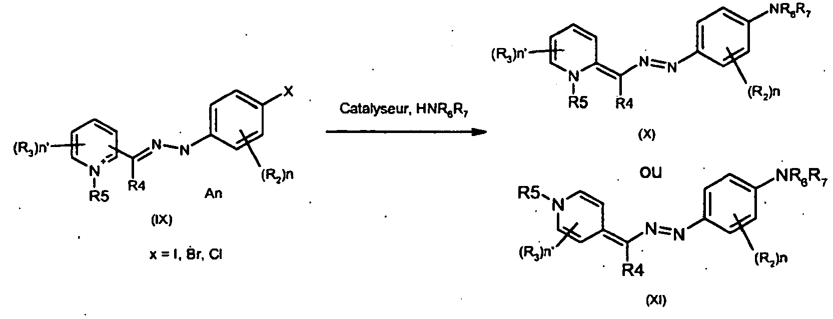
(d) Réaction d'alkylation

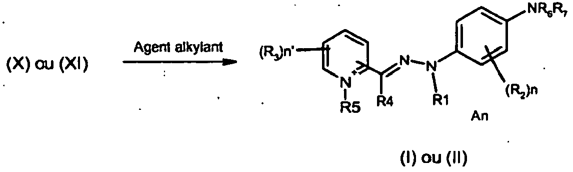

Les conditions de mises en oeuvre des étapes (a) et (b) sont les mêmes que celles décrites dans la variante de synthèse précédente et l'on pourra s'y reporter.

Dans une troisième étape, on réalise une réaction d'amination entre l'halogénure. arylique précédemment obtenu et une amine aliphatique ou arylique HNR₆R₇ de manière connue de l'homme du métier.

Lorsque HN₆R₇ est une amine aliphatique, la réaction d'amination peut se faire via une catalyse au cuivre (Cul, Cu₂O,...). A titre d'exemple, cette étape pourra être réalisée en s'inspirant des protocoles opératoires décrits dans les publications suivantes :
- Org. Lett. 4, 4, 2002, 581-584
- Org. Lett. 5, 6, 2003, 793-796
- Tetrahedron 61, 27, 2005, 6553-6560

Lorsque HNR₆R₇ est une amine aliphatique ou aromatique, la réaction d'amination peut se faire via une catalyse au palladium (Pd₂(dba)₃, Pd (OAc)₂,...). A titre d'exemple, cette étape pourra être réalisée en s'inspirant des protocoles opératoires décrits dans les publications suivantes :
- J. Org. Chem. 69, 2004, 9135-9142
- Ang. Chem. Int. Ed. Engl; 34, 12, 1995, 1348-1350

Une fois la réaction réalisée, on effectue dans une quatrième étape une réaction d'alkylation en présence d'une base inorganique (MgO, K₂CO₃, Na₂CO₃,...).

L'agent alkylant R₁X peut être un halogénure d'alkyle comme par exemple l'iodure de méthyle, le 1-bromopropyle, le 3-chlorohexane ou bien un alkyl ou arylsulfonate comme par exemple le méthyl 4-toluènesulfonate ou le benzyl 4-toluènesulfonate, ou bien un dialkylsulfate comme par exemple, le diméthylsulfate, le diéthylsulfate ou le dipropylsulfate.

Habituellement cette réaction est mise en oeuvre dans un autre solvant organique approprié tel que le dichlorométhane, l'isopropanol, l'acétate d'éthyle, le 1,2-dichloroéthane, le toluène,....

La température est classiquement comprise entre 0 et 100°C de préférence entre 20°C et 70°C.

A titre d'exemple, on peut mettre en oeuvre ce second procédé pour la préparation du composé suivant :

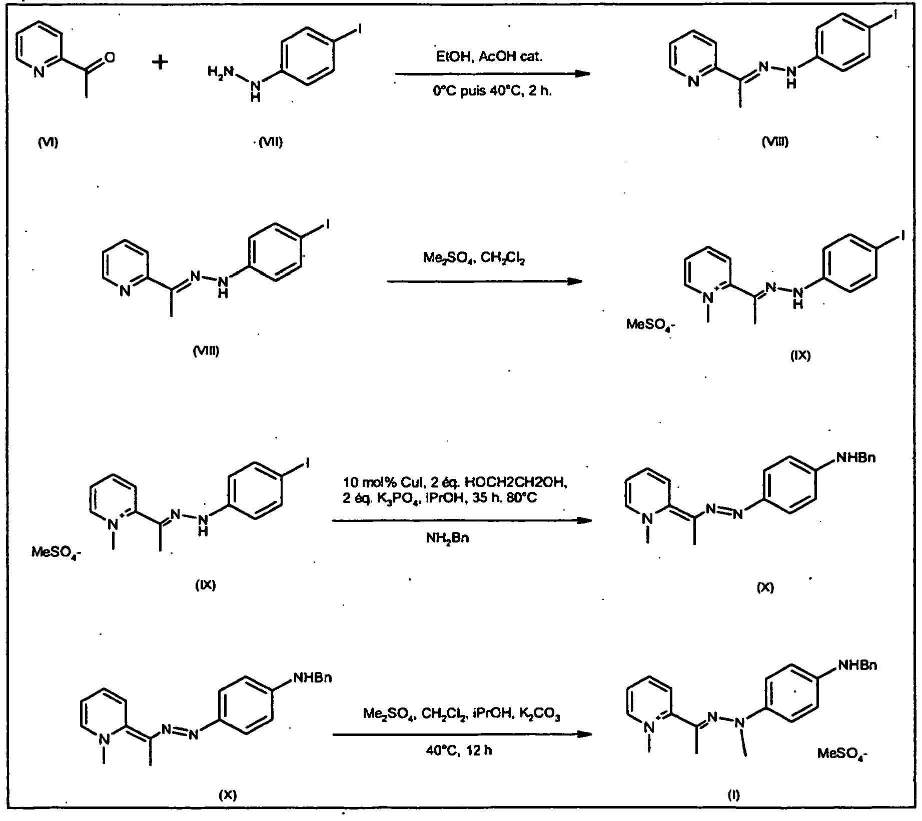

Selon un troisième mode de réalisation, les composés de formules (I) et/ou (II) peuvent être obtenus en mettent en oeuvre les étapes décrites ci-dessous :
(a) Réaction de quaternisation 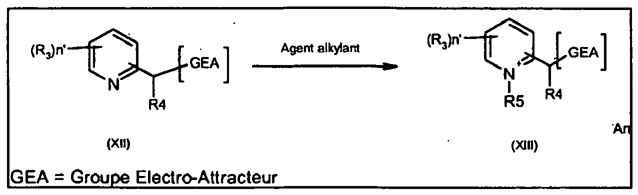
(b) Réaction de copulation d'un sel de diazonium sur un système activé 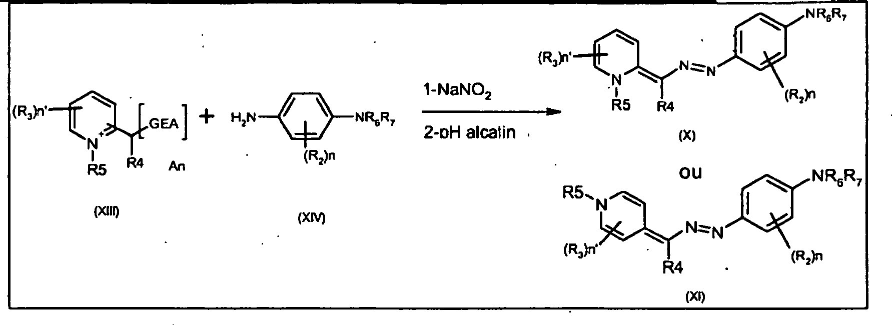
(c) Réaction d'alkylation

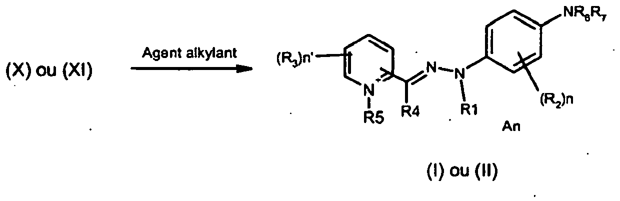

Selon ce procédé, on effectue une première étape de quaternisation du composé (XII). Habituellement cette réaction est mise en oeuvre dans un solvant approprié parmi lesquels on peut citer les alcools tels que le méthanol, l'éthanol, le propanol, l'isopropanol ; la dimethylformamide, le tetrahydrofuranne, la N-methylpyrrolidone, le 1,3-dimethyl-2-oxohexahydropyrimidine, l'acétate d'éthyle, le 1,2-dichloroéthane, le toluène,....

L'agent alkylant R₅X peut être un halogénure d'alkyle comme par exemple l'iodure de méthyle, le 1-bromopropyle, le 3-chlorohexane ou bien un alkyl- ou arylsulfonate comme par exemple le méthyl 4-toluènesulfonate ou le benzyl 4-toluènesulfonate, ou bien dialkylsulfate comme par exemple, le diméthylsulfate, le diéthylsulfate ou le dipropylsulfate.

La température est classiquement comprise entre 0 et 100°C de préférence entre 20°C et 70°C.

Les composés (XII) sont commerciaux ou peuvent être obtenus par exemple à partir des protocoles opératoires décrits dans :
- Chem. Pharm. Bull 30(5) 1680-1691, 1982
- Tet. Lett. 27(49) 6005-8, 1986

Dans une seconde étape, on réalise une réaction de copulation d'un sel de diazonium obtenu à partir d'une amine aromatique (XIV) sur le composé (XIII) précédemment obtenu de manière connue de l'homme du métier (voir à titre d'exemple le brevet FR2123267).

Habituellement cette réaction est mise en oeuvre dans un solvant approprié comme par exemple l'eau, les alcools tels que le méthanol, l'éthanol, le propanol, l'isopropanol.

La température est classiquement comprise entre -20 et 40°C de préférence entre -5°C et 20°C.

Le pH de la réaction est compris entre 1 et 13 de préférence entre 3 et 12. Le pH peut être contrôlé à l'aide d'acides organiques ou inorganiques tels que l'acide acétique, l'acide propionique, l'acide chlorhydrique, l'acide bromhydrique, l'acide iodhydrique, l'acide sulfurique ou à l'aide de bases inorganiques tels que les carbonates de potassium, de sodium, de césium, les hydroxydes de sodium, de potassium, de lithium...

Dans cette seconde étape, il est aussi possible d'utiliser des sels de diazonium disponibles dans le commerce tels que :

| Structures de sels de diazonium commerciaux | | |
|---|---|---|
| 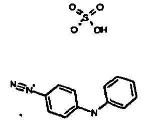 | 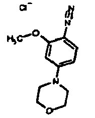 | 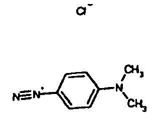 |
| 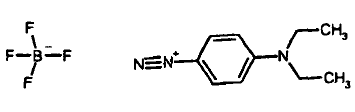 | 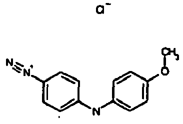 | 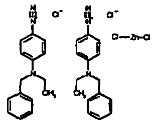 |
| 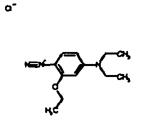 | 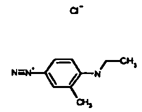 | 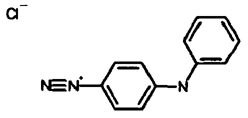 |
| 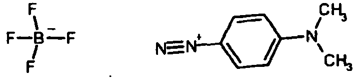 | 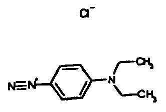 | 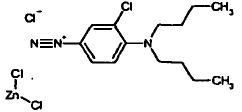 |
| 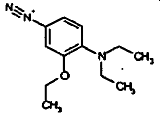 | 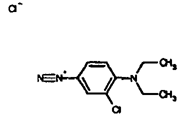 | 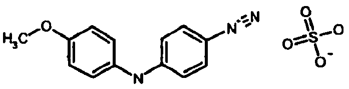 |
| 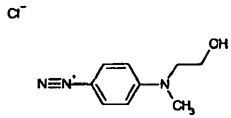 | 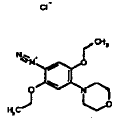 | 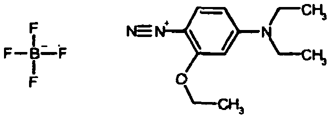 |
| 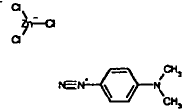 | 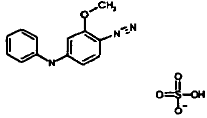 | 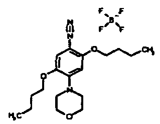 |
| 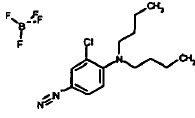 | 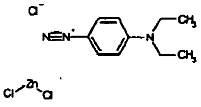 | 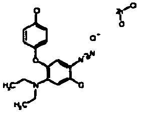 |
| 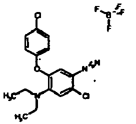 | 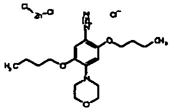 | 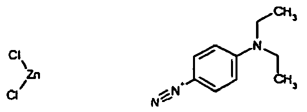 |
| 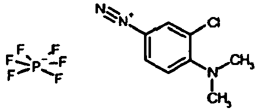 | 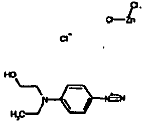 | 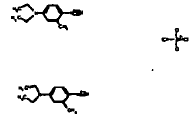 |
| 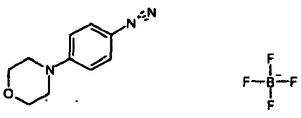 | 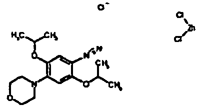 | |

On pourra de même envisager de les réaliser soi-même à partir des amines aromatiques (XIV) correspondantes (voir à titre d'exemple le brevet FR2123267) ;

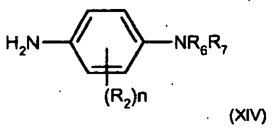

Les amines aromatiques (XIV) sont elles-mêmes commerciales ou peuvent être synthétisées en s'inspirant par exemple des brevets FR2806299, FR2807650, DE3016216, DE3433594, EP911317.

Une fois deuxième étape réalisée, on effectue, dans une troisième étape, une réaction d'alkylation en présence d'une base inorganique (MgO, K₂CO₃, Na₂CO₃, ... ).

L'agent alkylant R₁X et le solvant mise en oeuvre ont été décrits lors d'une variante de synthèse précédente et l'on pourra s'y référer.

Un autre objet de la présente invention est constitué par une composition comprenant, dans un milieu approprié pour la teinture des fibres kératiniques, au moins un composé de formules (I) et/ou (II), ou ses sels d'addition avec un acide, à titre de colorant direct.

La concentration en composé de formules (I) et/ou (II) ou en chacun des composés de formules (I) et/ou (II) peut varier entre 0,001 et 20 % en poids par rapport au poids total de la composition tinctoriale, plus particulièrement entre 0,01 à 10 % en poids, et de préférence entre 0,05 et 5 % en poids.

La composition selon l'invention peut de plus comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis=(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la. 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazine pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir de plus un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamine benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène et leurs sels d'addition avec un acide.

Dans la composition de la présente invention, le ou les coupleurs sont en général présents en quantité comprise entre 0,001 et 10 % en poids du poids total de la composition tinctoriale et plus préférentiellement de 0,005 à 6 % en poids. La ou les bases d'oxydation sont présentes en quantité de préférence comprise entre 0,001 à 10 % en poids du poids total de la composition tinctoriale, et plus préférentiellement de 0,005 à 6 % en poids.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition selon l'invention peut éventuellement comprendre au moins un colorant direct additionnel différent des composés de formules (I) et/ou (II). Celui-ci peut être choisi parmi les espèces cationiques où non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-((3-hydroxyéthyl)amino benzène,
- la 2-nitro-4'-hydroxydiphénylamine,
- le 1-amino-2-nitro-4-hydroxy-5-méthylbenzène.

Le colorant direct additionnel peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoique,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple:
- le 1-(β-hydroxyéthyl)amino-4-N,N-bis-([3-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, Nβ-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 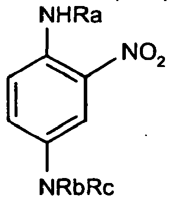 dans laquelle :
- Rb représente un radical alkyle en C₁-C₄, un radical β-hydroxyéthyle ou β-hydroxypropyte ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954:

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants; décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]amilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthylramino]-phényl-uréido-6-méthyl-1,4-benzoquinone imine.

Parmi les colorants de type tétraazapentaméthiniques utilisables selon l'invention, on peut citer les composés suivants: le chlorure de 2-((E)-{(E)-[(1,3-dimethyl-1,3-dihydro-2H-imidazol-2-ylidene)hydrazono]methyl}diazenyl)-1,3-dimethyl-1H-imidazol-3-ium, le chlorure de 2-{(EH(1Z)-N-(1,3-dimethyl-1,3-dihydro-2H-imidazol-2-ylidene)ethanehydrazonoyl] diazenyl}-1,3-dimethyl-1H-imidazol-3-ium, le chlorure de 4-methoxy-2-((E)-{(1E)-1-[(2E)-(4-methoxy-1-methylpyridin-2(1H)-ylidene)hydrazono]ethyl} diazenyl)-1-methylpyridinium, le chlorure de 1-methyl-2-((E)-{(1E)-1-[(2E)-(1-methylpyridin-2(1H)-ylidene)hydrazono]ethyl} diazenyl)pyridinium, le chlorure de 1-(2-hydroxyethyl)-2-[(E)-((1E)-1-{(2E)-[1-(2-hydroxyethyl)pyridin-2(1H)-ylidene]hydrazono) ethyl)diazenyl]pyridinium, le chlorure de 1-methyl-2-((E)-{(E)-[(2Z)-(1-methylpyridin-2(1H)-ylidene)hydrazono]methyl}diazenyl) pyridinium, l'acétate de 1-(2-hydroxyethyl)-2-[(E)-((E)-{(2E)-[1-(2-hydroxyethyt)pyridin-2(1H)-ylidene]hydrazono}methyl)diazenyl]pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorant(s) direct(s) additionnels dans la composition varie en général de 0,001 à 20% en poids par rapport au poids de la composition, et de préférence de 0,01 à 10% en poids par rapport au poids de la composition.

Le milieu approprié pour la teinture, appelé aussi support de teinture, est généralement constitué par de l'eau ou par un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition.

La composition tinctoriale conforme à l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 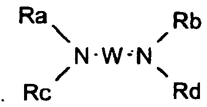 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, de pâtes ou sous toute autre forme appropriée pour réaliser une teinture des fibres kératiniques, et notamment des cheveux humains.

La composition selon l'invention peut en outre comprendre au moins un agent oxydant. On parle dans ce cas de composition prête à l'emploi.

Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

Selon cette variante, la composition de l'invention comprend au moins un agent oxydant afin d'obtenir notamment un éclaircissement des fibres. Les agents oxydants classiquement utilisés pour la coloration des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Selon un mode de réalisation particulier, la composition contient un agent oxydant de type peroxyde et/ou un agent oxydant de type persels, par exemple un mélange peroxyde d'hydrogène et persulfates, le peroxyde d'hydrogène seul ou le persulfate seul.

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

Le pH de la composition peut être réglé au moyen d'un agent alcalinisant ou acidifiant, notamment choisis parmi ceux mentionnés auparavant, dans le cadre de la description selon l'invention.

L'invention a aussi pour objet un procédé de coloration qui comprend l'application d'une composition tinctoriale selon l'invention sur les fibres kératiniques, sèches ou humides.

L'application sur les fibres de la composition tinctoriale comprenant le ou les composés de formule (I) et/ou (II) ou ses sels d'addition avec un acide, éventuellement au moins une base d'oxydation éventuellement associée à au moins un coupleur, éventuellement au moins un colorant direct additionnel, peut être mise en oeuvre en présence d'agent oxydant.

Cet agent oxydant peut être ajouté à la composition comprenant le ou les composés de formule (I) et/ou (II) ainsi que les éventuels base d'oxydation, coupleurs et/ou colorants directs additionnels, soit au moment de l'emploi, soit directement sur la fibre kératinique.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 4 et 12 environ, et encore plus préférentiellement entre 7 et 11,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, etc notamment des cheveux humains.

Selon un mode de réalisation particulier, la composition selon l'invention est exempte de base d'oxydation et de coupleur.

La composition appliquée peut éventuellement comprendre au moins un agent oxydant.

La composition est donc appliquée sur les fibres kératiniques, sèches ou humides, puis laissée pendant un temps dé pose suffisant pour obtenir la coloration recherchée.

Quelle que soit la variante retenue (avec ou sans agent oxydant) le temps de pose est en général compris entre quelques secondes et une heure, de préférence entre 3 et 30 minutes.

La température à laquelle la composition est laissée agir, est en général comprise entre 15 et 220°C, plus particulièrement entre 15 et 80°C, de préférence entre 15 et 40 °C.

A l'issue du temps de pose, la composition est éliminée par un rinçage à l'eau, suivi éventuellement d'un lavage avec un shampooing, puis éventuellement d'un séchage.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913.

Les exemples qui suivent survent à illustrer l'invention sans toutefois présenter un caractère limitatif.

### EXEMPLE

### Exemple 1-

### Synthèse du sel de méthosulfate de 2-{(E)-[{4-[(4-methoxylphenyl)amino]phenyl} (methyl)hydrazono]methyl}-1-methylpyridinium

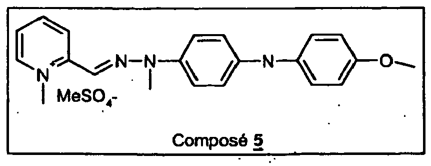

### Etape 1 :

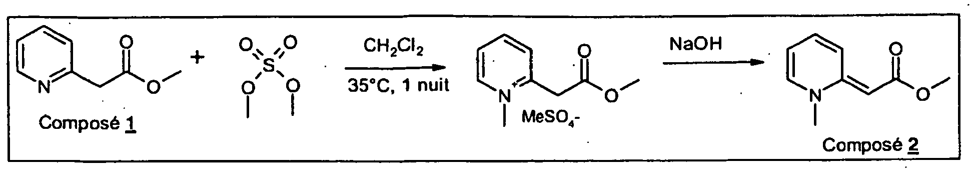

Le composé **1** est commercial et le composé **2** peut aussi être obtenu en s'inspirant du protocole opératoire décrit dans J.A.C.S., 126(48), 15777-15783, 2004.

Dans un ballon de 100mL surmonté d'un réfrigérant, on met sous agitation à température ambiante le composé **1** (36g ; 1éq) solubilisé dans 25mL de dichlorométhane. On ajoute par la suite le diméthylsulfate (36g ; 1.2éq). Le milieu réactionnel est mis à chauffer à 35°C pendant 1 nuit.

Après réaction, on élimine sous pression réduite le dichlorométhane puis on verse le milieu réactionnel sur un mélange composé d'eau, de glace et de soude (pH 11-12). Un produit jaune précipite. Celui-ci est filtré sur fritté, lavé plusieurs fois à l'eau puis séché sous vide. 32,6 g d'une poudre jaune correspondant au composé **2** sont obtenus.

Les spectres RMN et les spectres de Masse sont conformes au produit 2 attendu.

### Etape 2 :

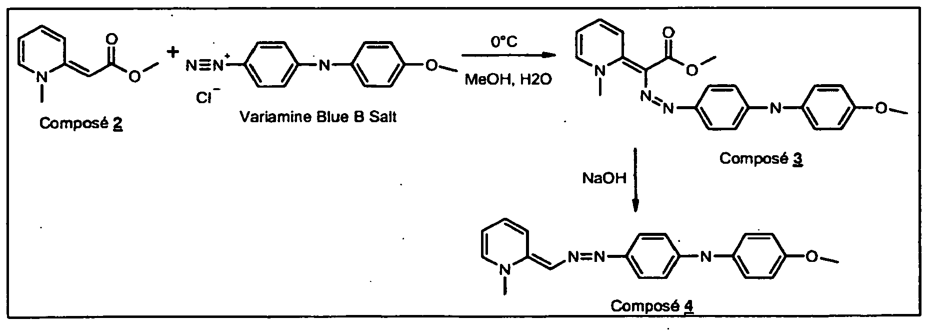

Dans un tricol muni d'un thermomètre et d'un pH-mètre, on introduit le composé **2** (4g) en solution dans un mélange eau 1 méthanol (50mL / 50mL). La solution est refroidie à 5°C par un bain d'eau glacée. La Variamine Blue (6.3g, produit commercial), solubilisée dans 50mL de méthanol est ajoutée au goutte à goutte à 5°C au milieu réactionnel précédent.

On laisse agiter 4h tout en laissant par la suite la température du milieu réactionnel revenir à température ambiante.

Après réaction, on verse le milieu réactionnel sur un mélange d'eau glacée et de soude (pH12-13) ; un précipité apparaît. Une analyse réalisée sur le précipité indique qu'il s'agit du composé **3**. Cette solution hétérogène basique est laissée sous agitation pendant 2 jours à température ambiante.

Un solide de couleur différente du composé **3** est obtenu. Celui-ci est filtré puis séché sous vide.

Le produit obtenu est purifié sur colonne de silice (éluant CH2CI2 / MeOH / AcOH 8/2/1).
417mg d'une poudre violet foncée correspondant au composé **4** sont obtenus.

Les spectres RMN et les spectres de Masse sont conformes au produit 4 attendu.

### Etape 3 :

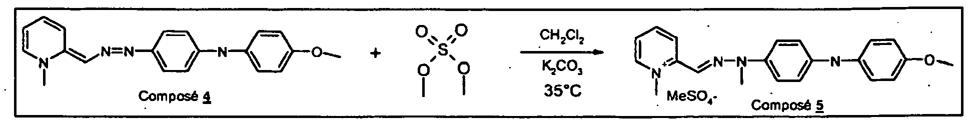

Dans un ballon surmonté d'un bulleur, on met sous agitation et à 35°C le composé **4** (200g) solubilisé dans 5mL de dichlorométhane et on additionne 3 pointes de spatule de K₂CO₃.

On introduit ensuite à 35°C le diméthylsulfate (0.082g ; 1.1éq) et on laisse agiter 1 nuit à 35°C.

Après réaction, un produit a précipité. On le filtre et celui-ci est réempaté au dichlorométhane.

On obtient le composé 5 sous la forme d'une poudre orange (500mg).

Les spectres RMN et les spectres de Masse sont conformes au produit 5 attendu.

### Exemple 2 -

### Synthèse du sel de méthosulfate de 2-((1Z)-2-methoxy-N-{4-[(4-methoxyphenyl) amino]phenyl}-N-methyl-2-oxoethanehydrazonoyl)-1-methylpyridinium (6)

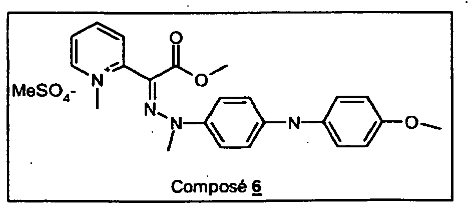

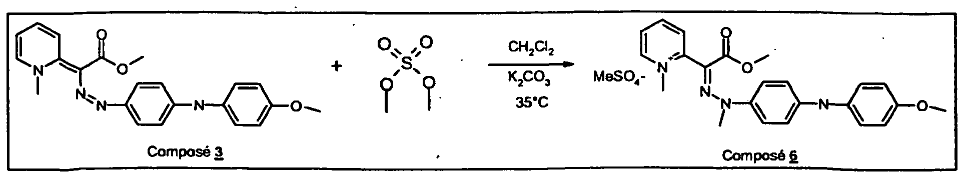

Dans un ballon de 10mL surmonté d'un bulleur, on met sous agitation et à 35°C le composé 3 précédemment obtenu (200mg ; 1éq) solubilisé dans 5mL de dichlorométhane. Deux pointes de spatule de K₂CO₃ sont ajoutées.

On introduit ensuite à 35°C le diméthylsulfate (0.082g ; 1.1éq) et on laisse agiter 1 nuit à 35°C. Après réaction, le produit est précipité avec 100mL d'éther diisopropylique.

On le filtre et on le reprend par 20mL de méthanol, puis on évapore à sec le solvant. 420 mg d'un solide jaune-orangé correspondant au composé 6 sont obtenus.

Les spectres RMN et les spectres de Masse sont conformes au produit 6 attendu.

### Exemple 3-

### Synthèse du sel de méthosulfate de 2-((E)-[(2,5-dibutoxy-4-morpholin-4-ylphenyl)(methyl) hydrazono]methyl}-1-methylpyridinium (9)

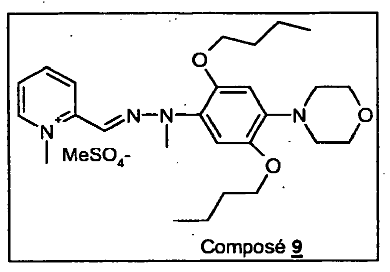

### Etape 1 :

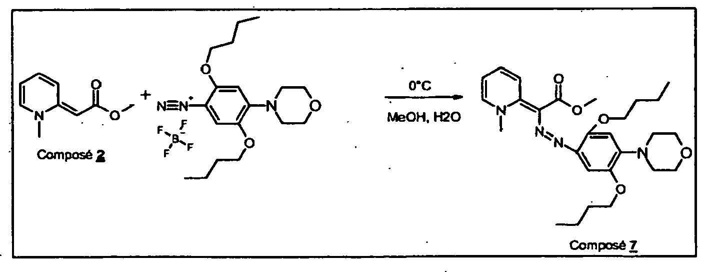

Dans un tricol surmonté d'un thermomètre et d'un pH-mètre, on introduit le composé **2** (0.8g) en solution dans un mélange eau / méthanol (10mL / 15mL). La solution est refroidie à 5°C par un bain d'eau glacée. La 2,5-dibutoxy-4-(4-morpholinyl)benzenediazonium tetrafluoroborate (2.04g, produit commercial), solubilisée dans 10mL de méthanol est ajoutée au goutte à goutte au milieu réactionnel précédent. On laisse agiter 3h tout en laissant la température du milieu réactionnel revenir à température ambiante.Après réaction, on verse le milieu réactionnel sur un mélange d'eau glacée et de soude (pH12-13).

On extrait ensuite au dichlorométhane la phase aqueuse obtenue. La phase organique est séchée sur Na2SO4 puis concentrée sous vide. Le produit obtenu est repris par de l'éther diisopropylique, filtré, puis séché sous vide. Une poudre violette foncée (1.73g) correspondant au composé **7** est obtenue.

Les spectres RMN et les spectres de Masse sont conformes au produit 7 attendu.

### Etape 2 :

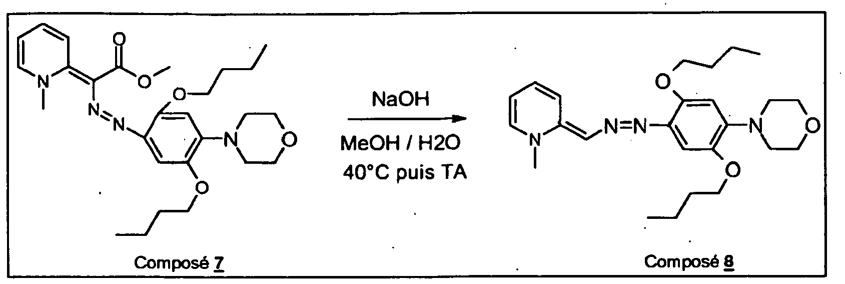

Dans un tricot muni d'un pH-mètre, on introduit le composé **7** (1,5g), en solution dans 25mL de méthanol et 5mL d'eau.

On ajoute au milieu réactionnel précédent une solution aqueuse concentrée d'hydroxyde de sodium pour atteindre un pH de 12-13.

On laisse agiter à 40°C pendant 3 heures puis à température ambiante pendant une nuit.

Après réaction, on verse le milieu réactionnel sur 200mL d'eau glacée ; une poudre foncée précipite. Celle-ci est filtrée sur fritté, puis séchée sous vide. 350mg dune poudre violette foncée correspondant au composé **8** sont obtenus.

Les spectres RMN et les spectres de Masse sont conformes au produit 8 attendu.

### Etape 3 :

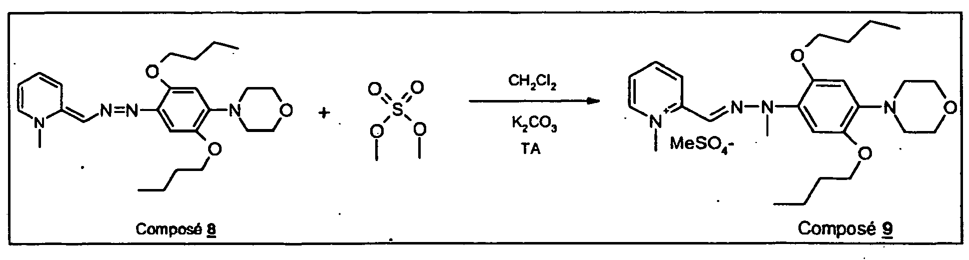

Dans un ballon surmonté d'un bulleur, on met sous agitation et à 35°C le composé **8** précédemment obtenu (306mg ) solubilisé dans 5mL de dichlorométhane. Trois pointes de spatule de K2CO3 sont ajoutés au milieu réactionnel précédent.

Le diméthylsulfate (0.1 g ; 1.1 éq) est ensuite introduit au milieu réactionnel et celui-ci est laissé sous agitation une nuit.

Après réaction, le produit est précipité par addition de 100mL d'acétate d'éthyle au milieu réactionnel, puis filtré, et enfin séché sous vide. On obtient 320 mg d'une poudre orange foncée correspondant au composé **9.**

Les spectres RMN et les spectres de Masse sont conformes au produit 9 attendu.

### Exemple 4-

### Synthèse du sel de méthosulfate de 2-{(E)-[[4-<diethylamino)phenyl]methyl) hydrazono]methyl}-1-methylpyridinium (12)

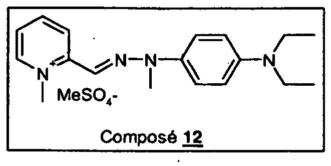

### Etape 1 :

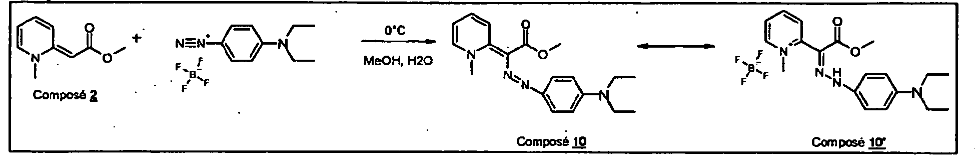

Dans un tricol équipé d'un thermomètre et d'un pH-mètre, on introduit le composé **2** (1g) en solution dans un mélange eau / méthanol (10mL / 15mL). La solution est préalablement refroidie à 5°C par un bain d'eau glacée. La 4-(diethylamino)-benzenediazonium tetrafluoroborate (1.6g, composé commercial), solubilisée dans 10mL de méthanol et 10mL d'eau, est ajoutée au goutte à goutte au milieu réactionnel précédent.

On laisse sous agitation le milieu réactionnel 2h tout en laissant sa température revenir à température ambiante.

Après réaction, on verse le milieu réactionnel sur un mélange composé d'eau, de glace et de soude (pH 12-13).

Un solide précipite. Celui-ci est filtré puis séché sous vide. Une poudre foncée correspondant au composé **10/10'** est obtenue (1.63g).

Les spectres RMN et les spectres de Masse sont conformes au produit attendu (mélange des formes 10 et 10').

### Etape 2 :

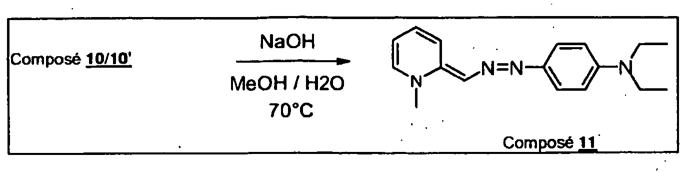

Dans un ballon muni d'un bulleur, on introduit à température ambiante le composé **10/10'** (750mg), en solution dans 12mL de méthanol et 2mL d'eau. 4mL d'une solution aqueuse concentrée d'hydroxyde de sodium sont ajoutés au milieu réactionnel précédent (pH 12-13). On laisse le milieu réactionnel sous agitation à 70°C pendant 2 heures.

Après réaction, on verse le milieu réactionnel sur 200mL d'eau glacée et on extrait au dichlorométhane la phase aqueuse obtenue. La phase organique est séchée sur Na2SO4 filtrée puis concentrée sous vide. Une poudre rouge foncée correspondant au composé **11** est obtenue (441 mg).

Les spectres RMN et les spectres de Masse sont conformes au produit 11 attendu.

### Etape 3 :

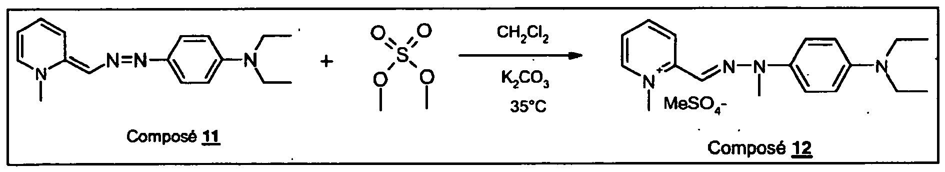

Dans un ballon équipé d'un bulleur, on met sous agitation et à 35°C le composé **11** (291mg) solubilisé dans 5mL de dichlorométhane. Trois pointes de spatule de K2CO3 sont ajoutées au milieu réactionnel précédent.

On introduit ensuite à 35°C le diméthylsulfate (1,146g ; 1,1éq) et on laisse sous agitation 1 heure.

Après réaction, le produit est précipité par addition de 100mL d'acétate d'éthyle au milieu réactionnel précédemment obtenu. Une poudre rouge foncée brillante correspondant au composé **12** est obtenue (191mg).

Les spectres RMN et les spectres de Masse sont conformes au produit 12 attendu.

### Exemple 5-

### Synthèse du sel de méthosulfate de 4-{(Z)-[{4-[4-methoxyphenyl)amino]phenyl} (methyl)hydrazono]methyl}-1-methylquinolinium (17)

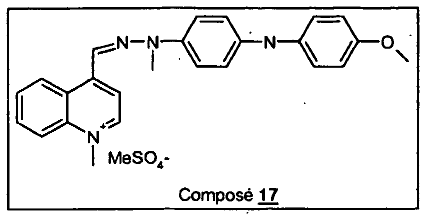

### Etape 1 :

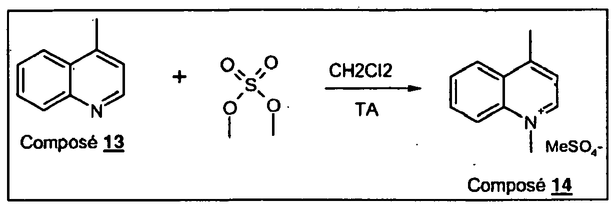

Dans un ballon équipé d'un bulleur, on met sous agitation le composé **13** (10g-produit commercial) solubilisé dans 50mL de dichlorométhane. Le diméthylsulfate (8.91g ; 1éq) est ensuite introduit au milieu réactionnel précédent.

On laisse sous agitation à température ambiante pendant 1 nuit.

Après réaction, on observe la formation d'un précipité. Celui-ci est filtré puis séché sous vide. Une poudre blanche correspondant au composé **14** (15g) est obtenue.

Les spectres RMN et les spectres de Masse sont conformes au produit attendu.

### Etape 2 :

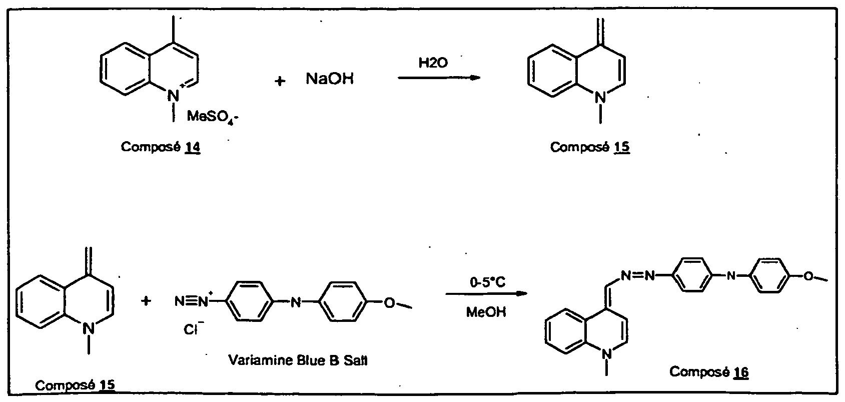

Dans un erlen, on introduit le composé **14** (3,1g) en solution dans 50mL d'eau glacée.

Une solution d'hydroxyde de sodium à 35% est additionnée au goutte à goutte au milieu réactionnel précédent. Un précipité apparaît alors ; celui-ci est filtré puis séché sous vide. Une poudre rosâtre correspondant au composé **15** est obtenue.

Dans un bécher, on solubilise 2 g de variamine blue dans 30 mL de méthanol. La solution est refroidie à 0-5°C par un bain d'eau glacée et on ajoute lentement une suspension de 1,81 g de composé **15** dans 20mL de méthanol.

On laisse agiter 2h tout en laissant la température du milieu réactionnel revenir à température ambiante.

Après réaction, le milieu réactionnel est versé sur un mélange constitué d'eau, de glace et de soude (pH12-13) : un précéipité apparaît ; celui-ci est filtré sur fritté puis séché sous vide.

2.46g d'une poudre bleue foncée correspondant au composé **16** sont obtenus.

Les spectres RMN et les spectres de Masse sont conformes au produit **16** attendu.

### Etape 3 :

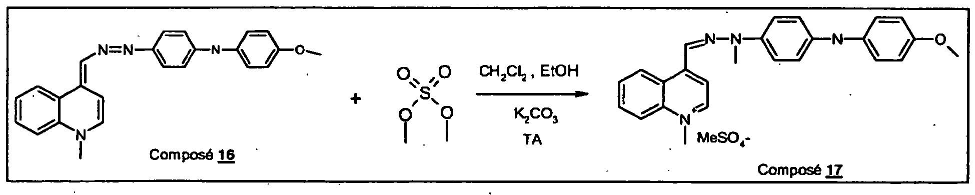

Dans un ballon équipé d'un bulleur, on met sous agitation et à 35°C le composé **16** (200mg) solubilisé dans 10mL de dichlorométhane et 1mL d'éthanol. 2 pointes de spatule de K₂CO₃ sont ajoutées au milieu réactionnel précédent.

Le diméthylsulfate (0.0665g ; 1.1éq) est introduit ensuite au milieu réactionnel que l'on laisse sous agitation 3 heures.

Après réaction, le produit est précipité par addition de 300mL d'acétate d'éthyle au milieu réactionnel.

On obtient une poudre bleue foncée correspondant au composé **17** (146mg).

Les spectres RMN et les spectres de Masse sont conformes au produit 17 attendu.

### Exemple 6-

### Synthèse , du sel de méthosulfate de 4-{(Z)-[[4-(diethylamino)phenyl](methyl) hydrazono]methyl}-1-methylqinolinium (19)

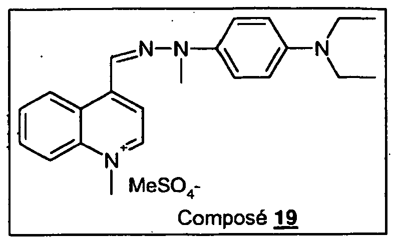

### Etape 1 :

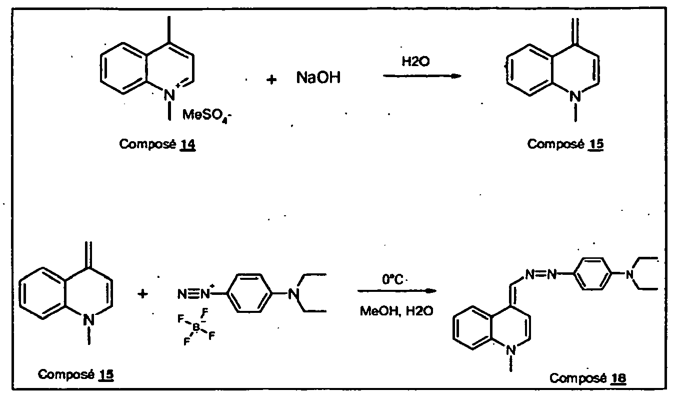

Dans un erlen, on introduit le composé **14** (5g) en solution dans 50mL d'eau glacée.

Une solution d'hydroxyde de sodium à 35% est additionnée au goutte à goutte au milieu réactionnel précédent. Un précipité apparaît alors ; celui-ci est filtré puis séché sous vide. Une poudre rosâtre correspondant au composé **15** est obtenue.

Dans un bécher est refroidi à 0°C par un bain d'eau glacée, la 4-(diethylamino)-benzenediazonium tetrafluoroborate (4.06g - produit commercial), solubilisée dans 40mL de méthanol et 10 mL d'eau.

On y ajoute lentement le composé rosâtre **15** (2.92 g) précédemment obtenu en suspension dans 30mL de méthanol.

On laisse sous agitation 2 heures tout en laissant la température du milieu réactionnel revenir à température ambiante.

Après réaction, le milieu réactionnel est versé sur un mélange constitué d'eau, de glace et de soude (pH12-13) : un précéipité foncé apparaît. Celui-ci est filtré sur fritté puis séché sous vide. 4.6g d'une poudre noire correspondant au composé **18** sont obtenus.

Les spectres RMN et les spectres de Masse sont conformes au produit 18 attendu.

### Etape 3 :

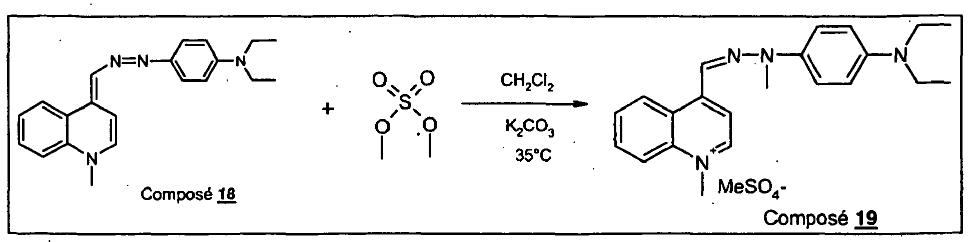

Dans un ballon équipé d'un bulleur, on met sous agitation et à 35°C le composé **18** (1.97g) solubilisé dans 10mL de dichlorométhane et 3mL d'éthanol. Deux pointes de spatule de K₂CO₃ sont ajoutées au milieu réactionnel précédent.

Le diméthylsulfate (1,622g ; 2.2éq) est ensuite introduit au milieu réactionnel précédent, et on laisse sous agitation 8h à 35°C, puis une nuit à température ambiante.

Après réaction, le produit est précipité par addition de 300mL d'acétate d'éthyle au milieu réactionnel. Celui-ci est filtré puis séché sous vide. Après purification sur colonne de silice flash (éluant CH₂Cl₂ / MeOH / AcOH 8/2/1), 0.5 g d'une poudre correspondant au composé **19** sont obtenus.

Les spectres RMN et les spectres de Masse sont conformes au produit 19 attendu.

### Exemple 7-

### Synthèse du sel de méthosulfate de 4-((Z)-[4-anilino-2-methoxypheny)(methyl) hydrazono]methyl}0-1-methylquinolinium (21)

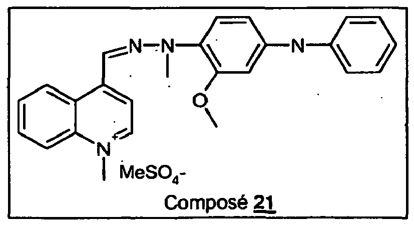

### Etape 1 :

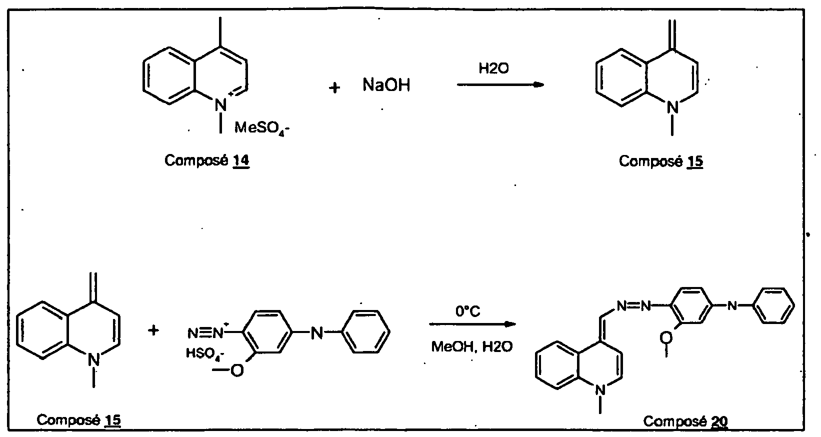

Dans un erlen, on introduit le composé **14** (3.07g) en solution dans 50mL d'eau glacée.

Une solution d'hydroxyde de sodium à 35% est additionnée au goutte à goutte au milieu réactionnel précédent. Un précipité apparaît alors ; celui-ci est filtré puis séché sous vide. Une poudre rosâtre correspondant au composé **15** est obtenue.

Dans un bécher est refroidi à 0°C par un bain d'eau glacée, la 2-methoxy-4-(phenylamino)benzenediazonium hydrogenosulfate (3.34g - produit commercial), solubilisée dans 30mL de méthanol et 30mL d'eau. On y ajoute lentement le composé rosâtre **15** (1,81 g) en suspension dans 50mL de méthanol.

On laisse agiter 2h tout en laissant la température du milieu réactionnel revenir à température ambiante.

Après réaction, on verse le milieu réactionnel sur un mélange constitué d'eau, de glace et de soude (pH12-13) : un précipité apparaît. Celui-ci est filtré sur fritté puis séché sous vide. Après purification sur colonne de silice (éluant CH2Cl2 / MeOH / AcOH 8/2/1).

1,19g d'une poudre noire correspondant au composé **20** sont obtenus.

Les spectres RMN et les spectres de Masse sont conformes au produit 20 attendu.

### Etape 2 :

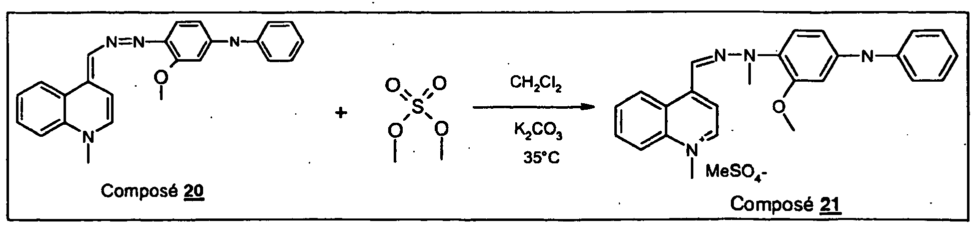

Dans un ballon équipé d'un bulleur, on met sous agitation et à 35°C le composé **20** (200mg) solubilisé dans 15mL de dichlorométhane. Deux pointes de spatule de K2CO3 sont ajoutés au milieu réactionnel précédent.

Le diméthylsulfate (0.0665g ; 1.1éq) est ensuite introduit et on laisse sous agitation. 4 heures à 35°C.

Après réaction, le produit est précipité par addition de 300mL d'acétate d'éthyle au milieu réactionnel. Le précipité est par la suite filtré sur fritté puis séché sous vide. Après purification sur colonne de silice (éluant CH2Cl2 / MeOH / AcOH 8/2/1), 51mg d'une poudre noire correspondant au composé **21** sont obtenue

Les spectres RMN et les spectres de Masse sont conformes au produit 21 attendu.

### Exemple 8-

### Synthèse du sel de méthosulfate de 4-{(Z)-[[4-lacetylamino)-2-hydroxyphenyl](methyl)

### hydrazono]methyl}-1-methylauinolinium methyl sulfate (24)

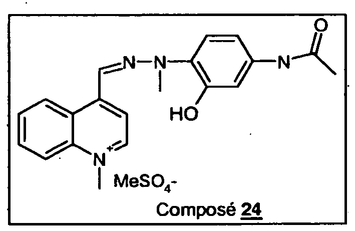

### Etape 1 :

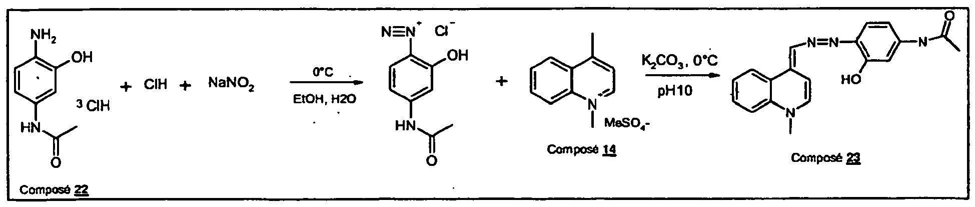

Dans un tricol muni d'un thermomètre, on introduit le composé **22** (200mg) en solution dans 1 mL d'eau, 3mL d'éthanol et 2mL d'HCl concentré (12N). La solution est préalablement refroidie à 0°C à l'aide d'un bain d'eau glacée, puis une solution de nitrite de sodium (68mg dans 2ml d'eau) est additionnée au goutte à goutte.

Après 5min d'agitation à 0°C, on introduit une solution d'urée (65mg dans 2ml d'eau).

Dans un tricol muni d'un thermomètre et d'un pH-mètre, on refroidie à 0°C, le composé **14** (197mg), solubilisée dans 5mL d'éthanol et 2mL d'eau. Le pH de la solution est ramené à 10,5 à l'aide d'une solution de carbonate de potassium (13g dans 75ml d'eau). On y ajoute lentement le sel de diazonium issu du composé **22** en maintenant la température du milieu réactionnel à 0°C et un pH de réaction comprit entre 8 et 10,5.

On laisse sous agitation pendant 2h tout en laissant la température du milieu réactionnel revenir à température ambiante.

Après réaction, on verse le milieu réactionnel sur un mélange d'eau glacée et de soude (pH12-13) : un précipité apparaît. Celui-ci est filtré sur fritté puis séché sous vide. 95.5mg d'une poudre bleue foncée correspondant au composé **23** sont obtenus.

Les spectres RMN et les spectres de Masse sont conformes au produit 23 attendu.

### Etape 2 :

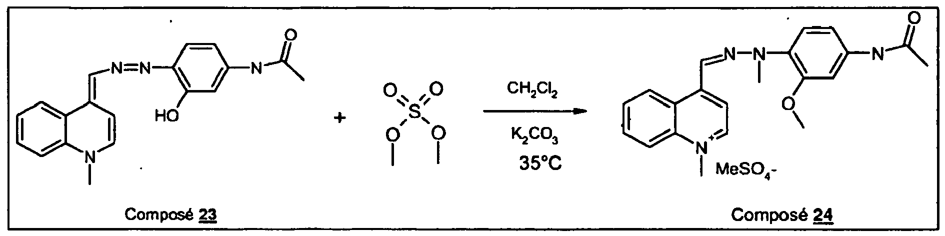

Dans un ballon équipé d'un bulleur, on met sous agitation et à 35°C le composé **23** (200mg) solubilisé dans 3mL de dichlorométhane et 2 mL d'une solution aqueuse saturée en K₂CO₃.

Le diméthylsulfate (0.133g ; 1.5éq) est ensuite introduit et on laisse sous agitation 4h à 35°C, puis 2 jours à température ambiante.

Après réaction, le produit est précipité par addition de 100mL d'éther diisopropylique au milieu réactionnel. Celui-ci est filtré sur fritté puis séché sous vide. Le produit obtenu est ensuite purifié par solubilisation dans un minimun d'éthanol puis par précipitation dans l'AcOEt.

168mg d'une poudre marron - orangée correspondant au composé **24** sont obtenus.

Les spectres RMN et les spectres de Masse sont conformes au produit 24 attendu.

### Exemples de teintures :

On a préparé les compositions tinctoriales dans les proportions suivantes

### Solution 1 :

| | |
|---|---|
| Alkyl (C8/C10 50/50) polyglucoside (2) en solution aqueuse à 60% | 120 g |
| Ethanol absolu pur | 200 g |
| Polyéthylène glycol(8 OE) 400 | 60 g |
| Alcool benzylique pur | 40 g |
| Eau déminéralisée | qsp 1000g |

### Solution 2 : TAMPON pH 9,5

| | |
|---|---|
| Chlorure d'ammonium (NH₄Cl) | 54 g |
| Ammoniaque en sol. à 20% | qsp pH=9,5 (env. 40 ml) |
| Eau déminéralisée | qsp 1000 ml |

### Solution 3 : TAMPON pH 7

| | |
|---|---|
| KH₂PO₄ | 0,026mol/l |
| Na₂PO₄ | 0,041 mol/l |
| Eau déminéralisée | qsp 500ml |

A pH 7, les compositions de coloration sont obtenues en dissolvant le colorant indiqué ci-dessous (5x10⁻³mol/l) dans la solution 1 puis en ajoutant un volume équivalent de solution tampon 3.

En condition alcaline éclaircissante, les compositions de coloration sont obtenues en dissolvant le colorant indiqué ci-dessous (5x10⁻³mol/l) dans la solution 1 puis en ajoutant un volume équivalent de solution tampon 2 et un volume d'eau oxygéné à 40 volumes.

Chaque composition est appliquée sur des cheveux gris à 90% de blancs, (1g de mèche pour 6g de solution). Après 30 min de pause, les mèches sont rincées, lavées avec un shampooing standard, rincées à nouveau puis séchées.

Les résultats de teinture suivants ont été obtenus

| | Après coloration | |
|---|---|---|
| | pH7 | Condition alcaline éclaircissante |
| 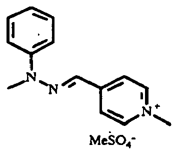 | Jaune chromatique | Jaune chromatique |
| C. 1 Basic Yellow 87: composé I non-conforme à l'invention | | |
| Composé 5 | Acajou intense | Acajou intense |
| Composé 12 | Rouge intense | Rouge intense |
| Composé 17 | Violet terne | Violet terne |

### Exemple 9-

### Synthèse du sel de méthosulfate de 1-methyl-4-{(E)-[methyl(4-pyrrolidin-1-yl-1-naphthyl)hydrazono]methyl}quinolinium (30)

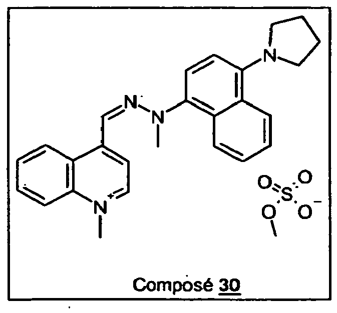

### Etape 1 :

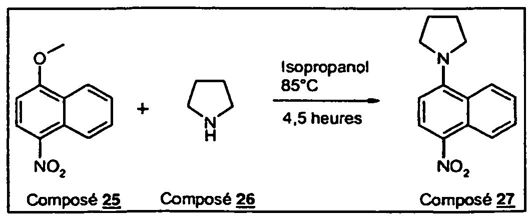

Dans un tricol de 50 ml muni d'un réfrigérant, on met sous agitation le composé **25** (1 g, 1 éq.), le composé **26** (2 ml, 5 éq.) en solution dans 1 ml d'isopropanol.

Le milieu réactionnel est ensuite chauffé à 85°C pendant 4,5 heures.

Après réaction, le milieu réactionnel est refroidi puis évaporé à sec.

Le milieu hétérogène est ensuite versé sur 200 ml d'un mélange constitué d'eau et de glace. Le précipité formé est ensuite filtré, lavé plusieurs fois à l'eau puis séché sous vide actif en présence de P₂O₅.

1,13 g d'une poudre jaune correspondant au composé **27** sont obtenus.

Les spectres RMN et les spectres de Masse sont conformes au composé **27** attendu.

### Etape 2

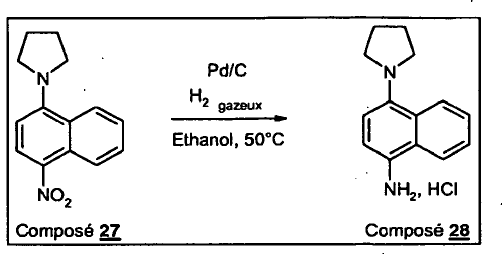

Dans un réacteur d'un litre, on met sous agitation le composé **27** (10,5 g), le palladium sur charbon (10-30%) (1,1 g) en solution dans 500 ml d'éthanol. Le milieu réactionnel est ensuite chauffé à 50°C. L'hydrogène est ensuite introduit (22 bars) au mélange précédent. Le milieu réactionnel est laissé sous hydrogène à 50°C pendant 1 heure.

Après réaction; le milieu réactionnel est refroidi puis filtré. La solution homogène obtenue est ensuite versée sur une solution diluée d'acide chlorhydrique (5N isopropanol) puis mise sous agitation pendant encore une heure.

Un précipité se forme progressivement. Celui-ci est filtré lavé à l'éther diisopropylique puis séché sous vide.

11,8 g d'une poudre rose claire correspondant au composé **28** sont obtenus.

Les spectres RMN et les spectres de Masse sont conformes au composé **28** attendu.

### Etape 3

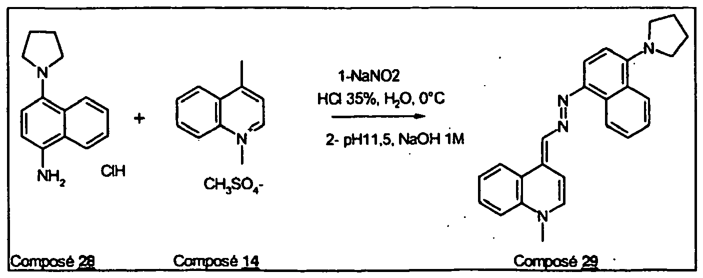

Dans un tricol muni d'un thermomètre, on introduit le composé **28** (0.5 g, 1 éq.) en solution dans 5 mL d'eau et 5 mL d'HCl concentré (35 %). La solution est préalablement refroidie à 0°C à l'aide d'un bain d'eau glacée, puis une solution de nitrite de sodium (0,128 g, 1 éq.) solubilisée dans 2ml d'eau préalablement refroidie à 0°C, est additionnée au goutte à goutte lent au milieu réactionnel précédent.

Après 20 minutes d'agitation à 0°C, on introduit une solution d'urée (120 mg dans 2ml d'eau).

Dans un tricol muni d'un thermomètre et d'un pH-mètre, on refroidit à 0°C, le composé **14** (0,495 g, 1 éq.), solubilisé dans 5 mL d'eau. On y ajoute lentement le sel de diazonium issu du composé **28** en maintenant la température du milieu réactionnel à 0°C. Par la suite, le pH de la solution est ramené à 10,5 à l'aide d'une solution aqueuse d'hydroxyde de sodium 1N (10 ml).

La solution fonce progressivement et un précipité apparaît.

On laisse sous agitation pendant 2 heures tout en laissant la température du milieu réactionnel revenir à température ambiante.

Après réaction, on verse le milieu réactionnel sur un mélange d'eau et de glace. Le précipité obtenu est filtré sur fritté puis séché sous vide actif en présence de P₂O₅.

0,5 g d'une poudre bleue foncé correspondant au composé **29** sont obtenus.

Les spectres RMN et les spectres de Masse sont conformes au produit **29** attendu.

### Etape 4 :

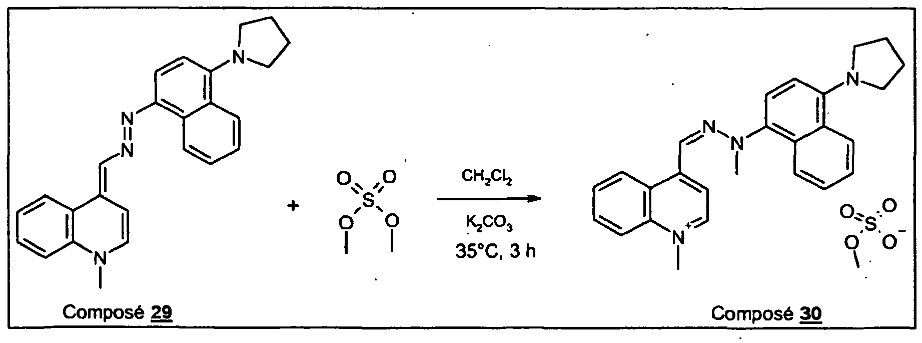

Dans un ballon de 50 ml équipé d'un bulleur et d'un réfrigérant, on met sous agitation et à 35°C le composé **29** (0,1 g), 20 mg de carbonate de potassium, solubilisé dans 4 mL de dichlorométhane.

Le diméthylsulfate (0,08 ml ; 3 éq) est ensuite introduit et on laisse sous agitation 3 heures à 35°C.

Après réaction, on laisse la température du milieu réactionnel revenir à température ambiante. Le produit attendu est précipité par addition de 100mL d'éther diisopropylique au milieu réactionnel.

Celui-ci est filtré sur fritté.

Le produit obtenu est ensuite purifié par solubilisation dans un minimun d'éthanol puis par précipitation dans l'éther diisopropylique.

96 mg d'une poudre marron - noir correspondant au composé **30** sont obtenus.

Les spectres RMN et les spectres de Masse sont conformes au produit **30** attendu.

### Exemple 10

### Synthèse du sel de méthosulfate de 1-methyl-2-{(E)-[methyl(4-pyrrolidin-1-yl-1-naphthyl)hydrazono]methyl}quinolinium (35)

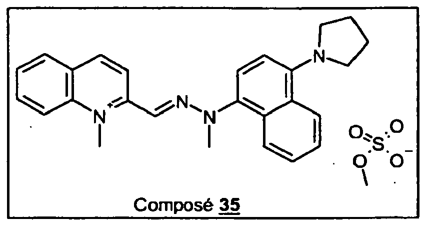

### Etape 1 :

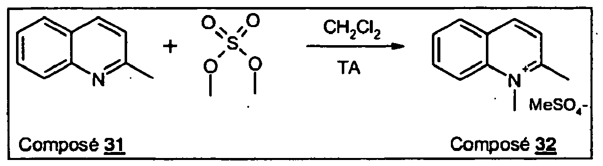

Dans un ballon équipé d'un bulleur, on met sous agitation le composé **31** (10g - produit commercial) solubilisé dans 50mL de dichlorométhane. Le diméthylsulfate (8.91g ; 1éq) est ensuite introduit au milieu réactionnel précédent.

On laisse sous agitation à température ambiante pendant 1 nuit.

Après réaction, on observe la formation d'un précipité. Celui-ci est filtré puis séché sous vide.

Une poudre blanche correspondant au composé **32** (15g) est obtenue.

Les spectres RMN et les spectres de Masse sont conformes au produit **32** attendu.

### Etape 2 :

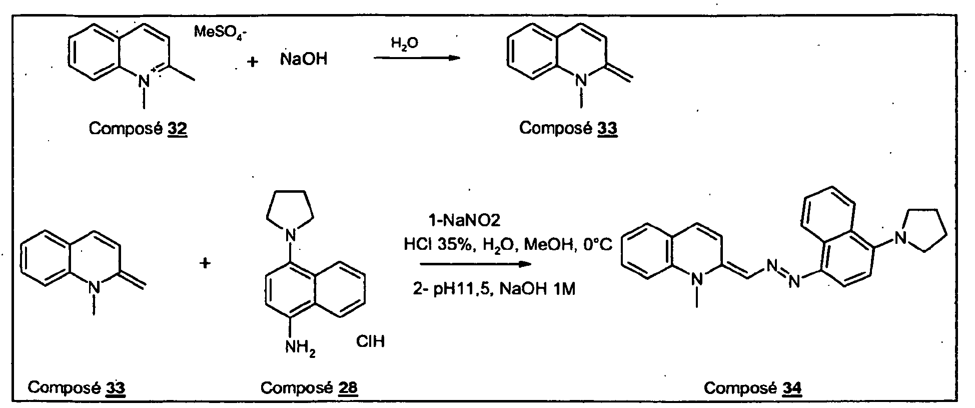

Dans un erlen, on introduit le composé **32** (1.19 g) en solution dans 100 mL d'eau glacée.

Une solution d'hydroxyde de sodium à 35% est additionnée au goutte à goutte au milieu réactionnel précédent. Un précipité apparaît alors ; celui-ci est filtré puis séché sous vide.

Une poudre rosâtre correspondant au composé **33** est obtenue.

Les spectres RMN et les spectres de Masse sont conformes au produit **33** attendu.

Dans un bécher est refroidi à 0°C par un bain d'eau glacée, le composé **28** (1 g, 1 éq.) en solution dans 10 mL d'eau et 10 mL d'HCl concentré (35 %). La solution est préalablement refroidie à 0°C à l'aide d'un bain d'eau glacée, puis une solution de nitrite de sodium (0.255 g, 1 éq.) solubilisée dans 2ml d'eau préalablement refroidie à 0°C, est additionnée au goutte à goutte lent au milieu réactionnel précédent.

Après 20 min d'agitation à 0°C, on introduit une solution d'urée (225 mg dans 2ml d'eau).

Dans un tricol muni d'un thermomètre et d'un pH-mètre, on refroidit à 0°C; le composé **33** précédemment obtenu, solubilisée dans 20 mL de méthanol. On y ajoute lentement le sel de diazonium issu du composé **28** en maintenant la température du milieu réactionnel à 0°C. Par la suite, le pH de la solution est ramené à 10,5 à l'aide d'une solution aqueuse d'hydroxyde de sodium 1 N (10 ml).

La solution fonce progressivement et un précipité apparaît.

On laisse sous agitation pendant 2h tout en laissant la température du milieu réactionnel revenir à température ambiante.

Après réaction, on verse le milieu réactionnel sur un mélange d'eau et de glace. Le précipité obtenu est filtré sur fritté puis séché sous vide actif en présence de P₂O₅.

1,1 g d'une poudre bleue foncé correspondant au composé **34** sont obtenus.

Les spectres RMN et les spectres de Masse sont conformes au produit **34** attendu.

### Etape 3 :

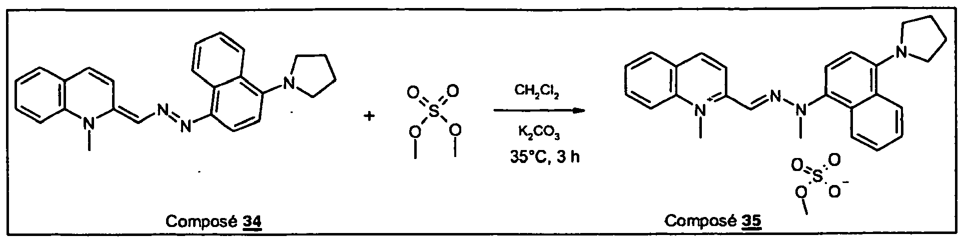

Dans un ballon de 50 ml équipé d'un bulleur et d'un réfrigérant, on met sous agitation et à 35°C le composé **34** (0,1 g), 20 mg de carbonate de potassium, solubilisé dans 4 mL de dichlorométhane.

Le diméthylsulfate (0,08 ml ; 3 éq) est ensuite introduit et on laisse sous agitation 3 heures à 35°C.

Après réaction, on laisse la température du milieu réactionnel revenir à température ambiante. Le produit attendu est précipité par addition de 100mL d'éther diisopropylique au milieu réactionnel.

Celui-ci est filtré sur fritté. Le produit obtenu est ensuite purifié par solubilisation dans un minimum d'éthanol puis par précipitation dans l'éther diisopropylique.

96 mg d'une poudre marron - noir correspondant au composé **35** sont obtenus.

Les spectres RMN et les spectres de Masse sont conformes au produit **35** attendu.

### Exemples de teintures :

On a préparé les compositions tinctoriales dans les proportions suivantes

### Solution 1 :

| | |
|---|---|
| Alkyl (C8/C10 50/50) polyglucoside (2) en solution aqueuse à 60% | 120 g |
| Ethanol absolu pur | 200 g |
| Polyéthylène glycol(8 OE) 400 | 60 g |
| Alcool benzylique pur | 40 g |
| Eau déminéralisée | qsp 1000g |

### Solution 2 : TAMPON pH 9,5

| | |
|---|---|
| Chlorure d'ammonium (NH₄Cl) | 54g |
| Ammoniaque en sol. à 20% | qsp pH=9,5 (env. 40 ml) |
| Eau déminéralisée | qsp 1000 ml |

### Solution 3 : TAMPON pH 7

| | |
|---|---|
| KH₂PO₄ | 0,026mol/l |
| Na₂PO₄ | 0,041 mol/l |
| Eau déminéralisée | qsp 500ml |

A pH 7, les compositions de coloration sont obtenues en dissolvant le colorant indiqué ci-dessous (5x10⁻³mol/l) dans la solution 1 puis en ajoutant un volume équivalent de solution tampon 3.

En condition alcaline éclaircissante, les compositions de coloration sont obtenues en dissolvant le colorant indiqué ci-dessous (5x10⁻³mol/l) dans la solution 1 puis en ajoutant un volume équivalent de solution tampon 2 et un volume d'eau oxygéné à 40 volumes..

Chaque composition est appliquée sur des cheveux gris à 90% de blancs, (1g de mèche pour 6g de solution). Après 30 min de pause, les mèches sont rincées, lavées avec un shampooing standard, rincées à nouveau puis séchées.

Les résultats de teinture suivants ont été obtenus

| | Après coloration | |
|---|---|---|
| | pH7 | Condition alcaline éclaircissante |
| Composé 30 | jaune | jaune |
| Composé 35 | jaune | jaune |

## Revendications

1. Composés monochromophoriques monocationiques comprenant une fonction hydrazone de formules (I) et (II) suivantes, leurs formes mésomères ainsi que leurs sels d'additions avec un acide et leurs solvates : 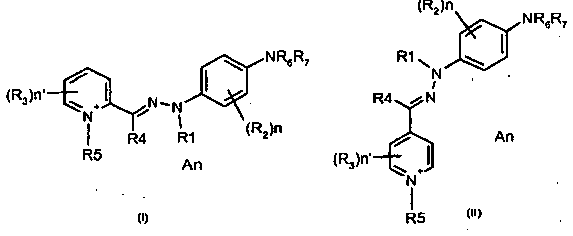 Formules (I) et/ou (II) dans lesquelles :
* Le radical **R₁** représente :
- un hydrogène
- un radical alkyle en C₁-C₂₀, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, CO, SO, SO₂ ou leurs combinaisons ; le radical alkyle ne comportant pas de fonction nitro, nitroso, peroxo ou diazo
- un radical phényle éventuellement substitué
- un radical benzyle éventuellement substitué
- un radical alkylcarbonyle (R-CO-) dans lequel R représenté un radical alkyle en C₁-C₄.
- un radical alkylsulfonyle (RSO₂-) dans lequel R représente un radical alkyle en C₁-C₄.
- un radical arylsulfonyle (R'SO₂-) dans lequel R' représente un radical phényle ou benzyle éventuellement substitué.
- un radical (di-)(alkyl)aminosulfonyle ((R)₂N-SO₂-) dans lequel les radicaux R indépendamment représentent un hydrogène, un radical alkyle en C₁-C₄.
- un radical. (di-)(alkyl) aminocarbonyle (R)₂N-CO-) dans lequel les radicaux R indépendamment représentent un hydrogène, un radical alkyle en C₁-C₄.
* Les radicaux **R₅**, identiques ou différents, représentent
- un radical alkyle en C₁-C₂₀, éventuellement substitué, éventuellement interrompu par un ou plusieurs hétéroatomes et/ou par un ou plusieurs groupements comprenant au moins un hétéroatome, de préférence choisis parmi l'oxygène, le soufre, CO, SO, SO₂ ou leurs combinaisons ; le radical alkyle ne comportant pas de fonction nitro, nitroso, peroxo ou diazo
- Un radical trimethylsilyl alkyl en C₁-C₄.
- un radical phényle éventuellement substitué
- un radical benzyle éventuellement substitué
* Les radicaux **R₂** et **R₃,** indépendamment l'un de l'autre, identiques ou différents, représentent :
- un atome d'halogène
- un radical alkyle en C₁-C₁₆ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂ ou leurs combinaisons ; le radical alkyle ne comportant pas de fonction nitro, nitroso, peroxo ou diazo
- un radical hydroxyle
- un radical alcoxy en C₁-C₄ ; un groupement (poly)hydroxyalcoxy en C₂-C₄
- un radical alcoxycarbonyle (RO-CO-) dans lequel R représente un radical alkyle en C₁-C₄.
- un radical alkylcarbonyloxy (RCO-O-) dans lequel R représente un radical alkyle en C₁-C₄.
- un radical aryloxy éventuellement substitué
- un radical (di-)arylamino éventuellement substitué
- un radical amino éventuellement substitué par un ou deux radicaux alkyle en C₁-C₄, identiques ou différents, éventuellement porteurs d'au moins un groupement hydroxyle ou alcoxy en C₁-C₂, lesdits radicaux alkyle pouvant éventuellement former avec l'atome d'azote auquel ils sont rattachés, un hétérocycle à 5 ou 7 chaînons, saturé ou insaturé, éventuellement substitué, éventuellement aromatique, comprenant éventuellement un autre hétéroatome identique ou différent de l'azote ;
- un radical alkylcarbonylamino (RCO-NR'-) dans lequel le radical R représente un radical alkyle en C₁-C₄ et R' représente un hydrogène, un radical alkyle en C₁-C₄.
- un groupement (di-)(alkyl)aminocarbonyle ((R)₂N-CO-) dans lequel les radicaux R, indépendamment l'un de l'autre représentent un hydrogène, un radical alkyle en C₁-C₄.
- un radical uréido (N(R)₂-CO-NR'-) dans lequel les radicaux R, indépendamment les uns des autres, représentent un radical alkyle en C₁-C₄ et R' représente un hydrogène, un radical alkyle en C₁-C₄.
- un radical (di-)(alkyl) aminosulfonyle ((R)₂N-SO₂-) dans lequel le radical R représente un atome d'hydrogène, un radical alkyle en C₁-C₄.
- un radical alkylthio (R-S-) dans lequel le radical R représente un radical alkyle en C₁-C₄.
- un radical alkylsulfonylamino (RSO₂-NR'-) dans lequel dans lequel le radical R représente un radical alkyle en C₁-C₄, et le radical R' représente un atome d'hydrogène, un radical alkyle en C₁-C₄.
- un radical cyano (-C≡N)
- un radical phényle
- un radical trifluorométhyle (-CF₃)
- un radical thio (-SH)
- un radical alkylsulfinyle (RSO-) dans lequel le radical R représente un radical alkyle en C₁-C₄.
- un radical alkylsulfonyle (RSO₂-) dans lequel le radical R représente un radical alkyle en C₁-C₄.
* R₁ peut former avec un radical R₂ en ortho du groupement NR₁ et avec l'atome d'azote substitué par R₁, un hétérocycle saturé ou insaturé à 5 ou 6 chaînons, substitué ou non.
* Deux radicaux R₂ adjacents peuvent former entre eux et avec les atomes de carbone auxquels ils sont reliés, un radical (hétéro)cyclique aromatique ou non, à 5 ou 6 chaînons, substitué ou non ;
* Deux radicaux R₃ adjacents peuvent former entre eux et avec les atomes de carbone auxquels ils sont rattachés, un cycle aromatique à 5 ou 6 chaînons, substitué ou non ;
* Le radical **R₄**, représente :
- un atome d'hydrogène
- un radical alkyle en C₁-C₁₆, éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂, ou leurs combinaisons ; le radical alkyle ne comportant pas de fonction nitro, nitroso, peroxo ou diazo.
- un radical amino éventuellement substitué par un ou deux radicaux alkyle en C₁-C₄, identiques ou différents, éventuellement porteurs d'au moins un groupement hydroxyle ou alcoxy en C₁-C₂, lesdits radicaux alkyle pouvant éventuellement former avec l'atome d'azote auquel ils sont rattachés, un hétérocycle à 5 ou 6 chaînons saturé ou insaturé ou aromatique éventuellement substitué, comprenant éventuellement au moins un autre hétéroatome identique ou différent de l'azote
- un radical alkylcarbonylamino (RCO-NR'-) dans lequel le radical R représente un radical alkyle en C₁-C₄ et R' représente un hydrogène, un radical alkyle en C₁-C₄
- un radical uréido (N(R)₂-CO-NR'-) dans lequel les radicaux R, indépendamment les uns des autres, représentent un radical alkyle en C₁-C₄ et R' représente un hydrogène, un radical alkyle en C₁-C₄.
- un radical alkylsulfonylamino (RSO₂-NR'-) dans lequel le radical R représente un radical alkyle en C₁-C₄ et R' représente un hydrogène, un radical alkyle en C₁-C₄
- un radical hydroxycarbonyle
- un radical alcoxycarbonyle en C₁-C₄
- un radical cyano
- un radical phényle éventuellement substitué
- un radical benzyle éventuellement substitué
* Les radicaux **R₆** et **R₇**, identiques ou différents, représentent :
- un atome d'hydrogène
- un radical alkyle en C₁-C₁₆ éventuellement substitué, éventuellement interrompu par un ou plusieurs hétéroatomes et/ou par un ou plusieurs groupements comprenant au moins un hétéroatome de préférence choisi parmi l'oxygène, l'azote, le soufre, CO, SO, SO₂ ou leurs combinaisons ; le radical alkyle ne comportant pas de fonction nitro, nitroso, peroxo ou diazo.
- un radical (alcoxy)aryle (-Ph-OR) dans lequel le radical R représente un hydrogène, un radical alkyle en C₁-C₄
- un radical (di-)(alkyl) aminoaryle (-Ph-N(R)₂) dans lequel les radicaux R indépendamment l'un de l'autre représentent un hydrogène, un radical alkyle en C₁-C₄ éventuellement substitué par un hydroxyle.
- un radical alkylsulfonyle (RSO₂-) dans lequel le radical R représente un radical alkyle en C₁-C₄
- un radical aminocarbonyle, un radical (di)alkyl(C₁-C₄)aminocarbonyle
- éventuellement un radical CH₃CO-
- un phényle
- un benzyle éventuellement substitué par un ou plusieurs hydroxy et/ou amino.
* les radicaux R₆ et R₇ peuvent éventuellement former avec l'atome d'azote auquel ils sont rattachés, un hétérocycle comprenant de 5 à 7 chaînons, saturé ou insaturé, éventuellement substitué, comprenant éventuellement un autre hétéroatome identique ou différent de l'azote
* l'un des radicaux R₆ ou R₇ peut également former avec l'atome d'azote auquel il est rattaché et avec un radical R₂ situé en ortho du groupement NR₆R₇, un hétérocycle saturé ou insaturé à 5 ou 6 chaînons substitué ou non
* les radicaux R₆ et R₇ peuvent former avec l'atome d'azote auquel ils sont rattachés et chacun avec un radical R₂ situé en ortho du groupement NR₆R₇, un hétérocycle saturé ou insaturé à 5 ou 6 chaînons substitué ou non
* n est un entier compris entre 0 et 4, lorsque n est inférieur à 4, le ou les atomes de carbone non substitués porte(nt) un atome d'hydrogène
* n' est un entier compris entre 0 et 4, lorsque n'est inférieur à 4, le ou les atomes de carbone non substitués porte(nt) un atome d'hydrogène
* l'électroneutralité des composés de formules (I) et/ou (II) étant assurée par un ou plusieurs anions An cosmétiquement acceptables, identiques ou non.

2. Composés selon la revendication précédente, **caractérisés en ce que** le radical R₁ représente un atome d'hydrogène; un radical méthyle, un radical- 2-hydroxyéthyle, un radical 3-methoxypropyle, un radical (di-) (methyl)aminoéthyle, un radical CH₃CO-, un radical CH₃SO₂-, un radical phényle, un radical 4-chlorophényl, un radical benzyle éventuellement substitué par un ou deux groupement(s) hydroxy, amino ou leur combinaison.

3. Composés selon l'une des revendications précédentes, **caractérisés en ce que** les radicaux R₅, identiques ou différents, représentent les radicaux méthyle, ethyle, propyle, butyle, pentyle, hexyle, heptyle, octyle, 2-hydroxyéthyle, 3-hydroxypropyle, 6-hydroxyhexyle, 2-methoxyéthyle, 3-methoxypropyle, 3-trimethylsilylpropyle, 2-((di-) methyl)aminopropyle, 3-((di-)methyl)aminopropyle, phényle, 4-chlorophényl, benzyle, 3,4-dihydroxybenzyle.

4. Composés selon l'une des revendications précédentes, **caractérisés en ce que** les radicaux R₂, R₃, identiques ou différents, représentent :
- un atome d'halogène choisi parmi le chlore, le fluor.
- un radical alkyle en C₁-C₈ éventuellement substitué par un ou plusieurs hydroxy ou ((di-)alkyl)amino ou sulfonylamino, éventuellement interrompu par un ou plusieurs hétéroatomes et/ou par un ou plusieurs groupements comprenant au moins un hétéroatome de préférence choisi parmi l'oxygène; le soufre, SO, SO₂, ou leurs combinaisons
- un radical hydroxyle
- un radical alcoxy en C₁-C₄ ;
- un radical alcoxy(C₁-C₄)carbonyle
- un radical aryloxy éventuellement substitué,
- un radical amino éventuellement substitué :
* par un ou deux radicaux alkyle en C₁-C₄, identiques ou différents, événtuellement porteurs d'au moins un groupement hydroxyle ou alcoxy en C₁-C₂,
* par un ou deux radicaux phényle, aminophényle, ou méthoxyphényle
- un radicaialkyl(C₁-C₄)carbonylamino dans lequel la fonction amino est substituée ou non par un radical alkyle en C₁-C₄
- un radical aminocarbonyle
- un radical aminosulfonyle, (di)alkyl (C₁-C₄)aminosulfonyle
- un radical alkyl(C₁-C₄)thio
- un radical phényle
- un radical trifluoromethyle
- un radical thio.

5. Composés selon l'une des revendications précédentes **caractérisés en ce que** le radical R₄ représente un atome d'hydrogène, un radical méthyle, éventuellement un radical hydroxycarbonyle, un radical méthoxycarbonyle, un radical phényle, un radical benzyle éventuellement substitué par un ou deux hydroxy ou amino ou leur combinaison.

6. Composés selon l'une des revendications précédentes, **caractérisés en ce que** les radicaux R₆ et R₇, identiques ou non, représentent
- Un atome d'hydrogène
- Un méthyle, un éthyle, un 2-hydroxyéthyle, 3-hydroxypropyle, un 3-methoxypropyle
- Un phényle
- Un benzyle éventuellement substitué par un ou deux hydroxyles et/ou amino,
- Un méthoxyphényle
- aminophényle
- Un (4-N,N-)diéthylaminophényle
- éventuellement un radical CH₃CO-.

7. Composition tinctoriate comprenant dans un milieu approprié pour la teinture des fibres keratiniques, à titré de colorant direct, au moins un composé de formules (I) où (II) selon l'une quelconque des revendications précédentes, ainsi que ses formes mésomères, ses sels d'additions avec un acide et ses solvates.

8. Composition selon la revendication précédente, **caractérisée en ce que** la teneur en composé de formules (I) ou (II) ou en chacun des composés de formules (I) ou (II) varie entre 0,001 et 20 % en poids par rapport au poids total de la composition tinctoriale, plus particulièrement entre 0,01 à 10 % en poids,.

9. Composition selon l'une quelconque des revendications 7 ou 8, **caractérisée en ce qu'**elle comprend au moins un colorant direct additionnel, au moins une base d'oxydation éventuellement associée à au moins un coupleur, ou leurs mélanges.

10. Composition selon l'une quelconque des revendications 7 à 9, **caractérisée en ce qu'**elle comprend au moins un agent oxydant.

11. Procédé de coloration de fibres kératiniques consistant à mettre en contact une composition selon l'une quelconque des revendications 7 à 10, avec lesdites fibres, sèches ou humides, pendant une durée suffisante pour obtenir l'effet souhaité.

12. Dispositif à plusieurs compartiments dans lequel un premier compartiment renferme la composition selon l'une quelconque des revendications 7 à 10 et un deuxième compartiment renfermant une composition oxydante.

13. Procédé de préparation des composés de formule (I) et /ou (II) selon l'une des revendications 1 à 6, dans lequel on met en oeuvre les étapes suivantes :
(a) Condensation d'un aldéhyde ou d'une cétone hétéroaromatique avec un dérivé d'hydrazine : 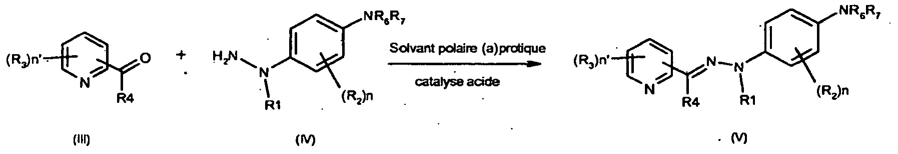
(b) Quaternisation du noyau hétérocyclique :

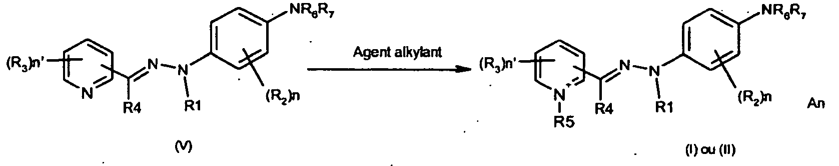

14. Procédé de préparation des composés de formule (I) et/ou (II) selon l'une des revendications 1 à 6, dans lequel on met en oeuvre les étapes suivantes :
(a) Condensation d'un aldéhyde ou d'une cétone hétéroaromatique avec un dérivé d'hydrazine 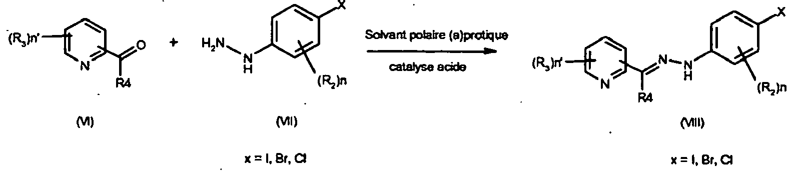
(b) Réaction de quaternisation du noyau hétérocyclique 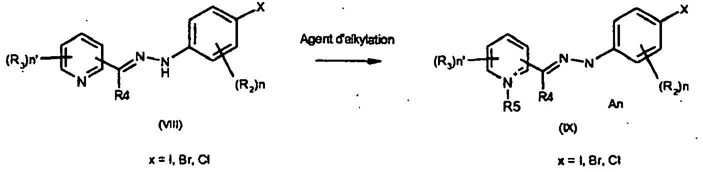
(c) Réaction d'amination 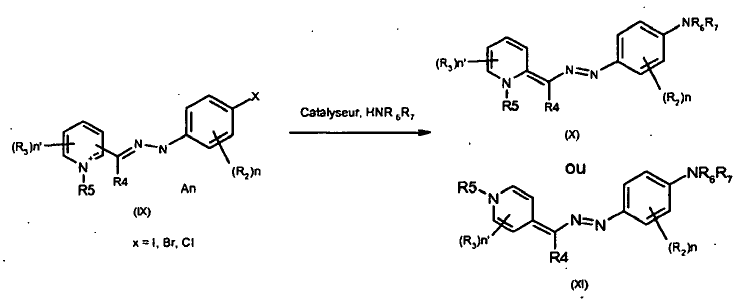
(d) Réaction d'alkylation

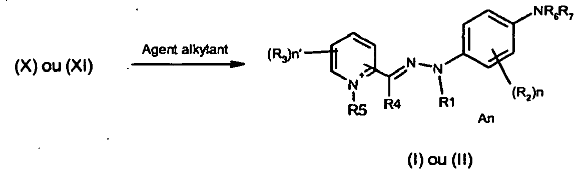

15. Procédé de préparation des composés de formule (I) et/ou (II) selon l'une des revendications 1 à 6, dans lequel on met en oeuvre les étapes suivantes :
(a) Réaction de quaternisation 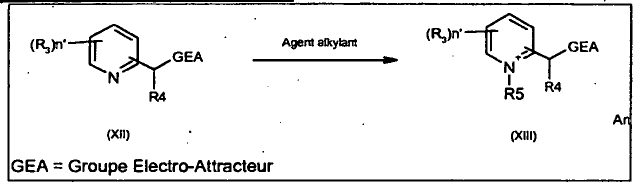
(b) Réaction de copulation d'un sel de diazonium sur un- système activé 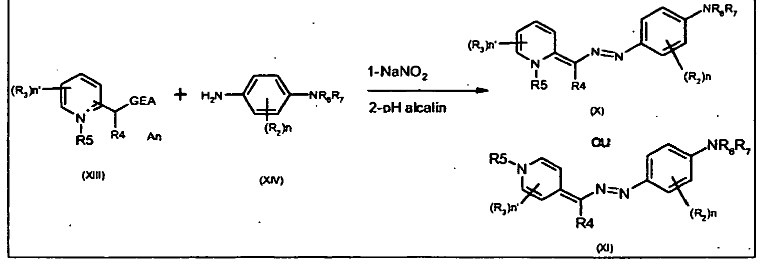
(c) Réaction d'alkylation

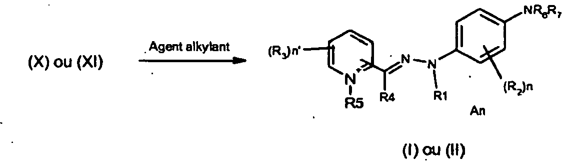

## Claims

1. Monocationic monochromophoric compounds comprising a hydrazone functional group of formulate (I) and (II) below, mesomeric forms thereof and also addition salts thereof with an acid and solvates thereof: 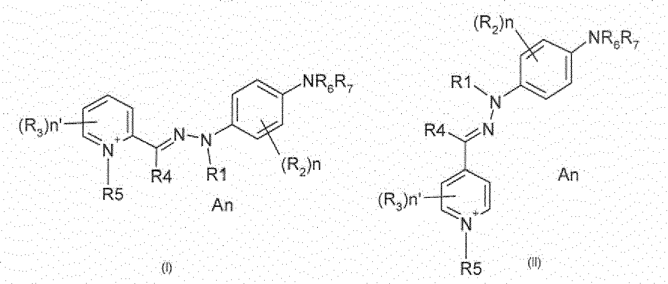 in which formulae (I) and/or (II):
* the R₁ radical represents:
- a hydrogen;
- an optionally substituted C₁-C₂₀ alkyl radical, optionally interrupted by one or more heteroatoms and/or by one or more groups comprising at least one heteroatom, preferable chosen from oxygen, nitrogen, sulphur, CO, SO and SO₂, or combinations thereof; the alkyl radical not comprising any nitro, nitroso, peroxo or diazo functional groups;
- an optionally substituted phenyl radical;
- an optionally substituted benzyl radical;
- an alkylcarbonyl radical (R-CO-) in which R represents a C₁-C₄ alkyl radical;
- an alkylsulphonyl radical (RSO₂-) in which R represents a C₁-C₄ alkyl radical;
- an arylsulphonyl radical (R'SO₂-) in which R' represents an optionally substituted phenyl or benzyl radical;
- a (di)(alkyl)aminosulphonyl radical ((R)₂N-SO₂-) in which the R radicals independently represent a hydrogen or a C₁-C₄ alkyl radical;
- a (di) (alkyl)aminocarbonyl radical ((R)₂N-CO-) in which the R radicals independently represent a hydrogen or a C₁-C₄ alkyl radical;
* the **R**₅ radicals, which may be identical or différent, represent:
- an optionally substituted C₁-C₂₀ alkyl radical, optionally interrupted by one or more heteroatoms and/or by one or more groups comprising at least one heteroatoms, preferably chosen from oxygen, sulphur, CO, SO and SO₂, or combinations thereof; the alkyl radical not comprising any nitro, nitroso, peroxo or diazo functional groups;
- a trimethylsilyl(C1-C4)alkyl radical;
- an optionally substituted phenol radical;
- an optionally substitutes benzyl radical;
* the **R**₂ and **R**₃ radicals, which may be identical or différent, represent, independently of one another:
- a halogen atom;
- an optionally substitutes C1-C16 alkyl radical, optionally interrupted by one or more heteroatoms and/or by one or more groups comprising at least one heteroatom preferably chosen from oxygen, nitrogen, sulphur, CO, SO and SO2, or combinations thereof; the alkyl radical not comprising many nitro, nitroso, peroxo or diazo functional groups;
- a hydroxyl radical;
- a C1-C4 alkoxy radical; a C2-C4 (poly)hydroxyalkoxy groups;
- an alkoxycarbonyl radical (RO-CO-) in which R represents a C1-C4 alkyl radical;
- an alkylcarbonyloxy radical (RCO-O) in which R represents a C1-C4 alkyl radical;
- an optionally substituted aryloxy radical;
- an optionally substituted (di)arylamino radical;
- an amino radical optionally substitutes by one or two C1-C4 alky radicals, which may be identical or différent, optionally bearing at least one hydroxyl or C1-C2 alkoxy groups, if bering possible for said alkyl radicals to optionally form, with the nitrogen atom to which they are attached, a saturated or unsaturated, optionally substituted, optionally aromatic heterocycle having 5 or 7 ring members, optionally comprising another heteroatom which may be identical to or different from nitrogen;
- an alkylcarbonylamino radical (RCO-NR'-) in which the R radical represents a C1-C4 alkyl radical and R' represents a hydrogen or a C1-C4 alkyl radical;
- a (di) (alkyl)aminocarbonyl group ((R)2N-CO-) in which the R radicals, independently of one another, represent a hydrogen or a C1-C4 alkyl radical;
- a ureido radical (N(R)2-CO-NR'-) in which the R radicals, independently of one another, represent a C1-C4 alkyl radical, and R' represents a hydrogen or a C1-C4 alkyl radical;
- a (di)(alkyl)aminosulphonyl radical ((R)2N-SO2-) in which the R radical represents a hydrogen atom or a C1-C4 alkyl radical;
- an alkylthio radical (R-S-) in which the R radical represents a C1-C4 alkyl radical;
- an alkylsulphonylamino radical (RSO2-NR'-) in which the R radical represents a C1-C4 alkyl radical, and the R' radical represents a hydrogen atom or a C1-C4 alkyl radical;
- a cyano radical (-C≡N);
- a phenol radical;
- a trifluoromethyl radical (-CF3);
- a thio (-SH) radical;
- an alkylsulphinyl radical (RSO-) in which the R radical represents a C1-C4 alkyl radical;
- an alkylsulphonyl radical (RSO2-) in which the R radical represents a C1-C4 alkyl radical;
* R₁ can form with an R₂ radical locates in the ortho position with respect to the NR₁ groups and with the nitrogen atom substituted by R₁, a substituted or unsubstituted, saturated or unsaturated heterocycle comprising 5 or 6 ring members;
* two adjacent R₂ radicals can form, with one another and with the carbon atoms to which they are attached, a substitutes or unsubstituted, aromatic or non-aromatic (hetero)cyclic radical comprising 5 or 6 ring members;
* two adjacent R₃ radicals can form, with one another and with the carbon atoms to which they are attached, a substituted or unsubstituted aromatic ring comprising 5 or 6 ring members;
* the R₄ radical represents:
- a hydrogen atom;
- an optionally substitute C1-C16 alkyl radical, optionally interrupted by one or more heteroatoms and/or by one or more groups comprising at least one heteroatom, preferably chosen from oxygen, nitrogen, sulphur, CO, SO and SO2, or combination thereof; the alkyl radical not comprising any nitro, nitroso, peroxo or diazo functional groups;
- an amino radical optionally substituted by one or two C1-C4 alkyl radicals, which may be identical or différent, optionally bearing at least one hydroxyl or C1-C2 alkoxy group, it being possible for said alkyl radicals to optionally form, with the nitrogen atom to which they are attached, a saturated or unsaturated or aromatic, optionally substituted heterocycle having 5 or 6 ring members, optionally comprising at least one other heteroatom which may be identical to or different from nitrogen;
- an alkylcarbonylamino radical (RCO-NR'-) in which the R radical represents a C1-C4 alkyl radial and R' represents a hydrogen or a C1-C4 alkyl radical;
- a ureido radical (N(R)2-CO-NR'-) in which the R radicals, independently of one another, represent a C1-C4 alkyl radical, and R' represents a hydrogen or a C1-C4 alkyl radical;
- an alkylsulphonylamino radical (RSO2-NR'-) in which the R radical represents a C1-C4 Alkyl radical, and R' represents a hydrogen or a C1-C4 alkyl radical;
- a hydroxycarbonyl radical;
- a C1-C4 alkoxycarbonyl radical;
- a cyano radical;
- an optionally substituted phenyl radical;
- an optionally substituted benzyl radical;
* the R₆ and R₇ radicals, which may be identical or différent, represent:
- a hydrogen atom;
- an optionally substituted C1-C16 alkyl radical, optionally interrupted by one or more heteroatoms and/or by one or more groups comprising at least one heteroatoms, preferably chosen from oxygen, nitrogen, sulphur, CO, SO and SO2, or combinations thereof; the alkyl radical not comprising any nitro, nitroso, peroxo or diazo functional groups;
- an (alkoxy)aryl radical (-Ph-OR) in which the R radical represents a hydrogen or a C1-C4 alkyl radical;
- a (di)(alkyl)aminoaryl radical (-Ph-N(R)2) in which the R radicals, independently of one another, represent a hydrogen or a C1-C4 alkyl radical optionally substituted by a hydroxyl;
- an alkylsulphonyl radical (RSO2-) in which the R radical represents a C1-C4 alkyl radical;
- an aminocarbonyl radical, a (di)(C1-C4)alkylaminocarbonyl radical;
- optionally a CH3CO- radical;
- a phenyl;
- a benzyl optionally substitutes by one or more hydroxyl and/or amino groups;
* the R₆ and R₇ radicals can optionally form with the nitrogen atom to which they are attached, a saturated or unsaturated, optionally substituted heterocycle comprising from 5 to 7 ring members, optionally comprising another heteroatom which may be identical to or different from nitrogen;
* one of the R₆ or R₇ radicals can also form with the nitrogen atom to which it is attached and with an R₂ radical located in the ortho position with respect to the NR₆R₇ group, a substituted or unsubstituted, saturated or unsaturated heterocycle comprising 5 or 6 ring members;
* the R₆ and R₇ radicals can form with the nitrogen atom to which they are attached and each with an R₂ radical located in the ortho position with respect to the NR₆R₇ group, a substituted or unsubstituted, saturated or unsaturated heterocycle comprising 5 or 6 ring members;
* n is an integer between 0 and 4, when n is below 4, the unsubstituted carbon atom or atoms bear(s) a hydrogen atom;
* n' is an integer between 0 and 4, when n' is below 4, the unsubstituted carbon atom or atoms bear(s) a hydrogen atom;
* the electroneutrality of the compounds of formulae (I) and/or (II) being ensured by one or more cosmetically acceptable anions An, which may be identical or different.

2. Compounds according to the preceding claim, **characterised in that** the R₁ radical represents a hydrogen atom; a methyl radical, a 2-hydroxyethyl radical, a 3-methoxypropyl radical, a (di) (methyl)aminoethyl radical, a CH₃CO- radical, a CH₃SO₂- radical, a phenol radical, a 4-chlorophenyl radical, a benzyl radical optionally substituted by one or two hydroxyl or amino group (s), or a combination thereof.

3. Compounds according to one of the preceding claims, **characterized in that** the R₅ radicals, which may be identical or different, represent the methyl, methyl, propyl, butyl, pentyl, hexyl, heptyl, octyl, 2-hydroxyethyl, 3-hydroxypropyl, 6-hydroxyhexyl, 2-methoxyethyl, 3-methoxypropyl, 3-trimethylsilylpropyl, 2-((di)methyl)aminopropyl, 3-((di)methyl)aminopropyl, phenyl, 4-chlorophenyl, benzyl or 3,4-dihydroxybenzyl radicals.

4. Compounds according to one of the preceding claims, **characterized in that** the R₂, R₃ radicals, which may be identical or différent, represent :
- a halogen atom chosen from chloride or fluorine;
- a C₁-C₈ alkyl radical optionally substituted by one or more hydroxyl or ((di)alkyl)amino or sulphonylamino groups, optionally interrupted by one or more heteroatoms and/or by one or more groups comprising at least one heteroatom, preferable chosen from oxygen, sulphur, SO and SO₂, or combinations thereof;
- a hydroxyl radical;
- a C₁-C₄ alkoxy radical;
- a (C₁-C₄)alkoxycarbonyl radical;
- an optionally substituted aryloxy radical;
- an optionally substituted amino radical:
■ by one or two C₁-C₄ alkyl radicals, which may be identical or différent, 8 optionally bearing at least one hydroxyl or C₁-C₂ alkoxy group; or
■ by one or two phenyl, aminophenyl or methoxyphenyl radicals;
- a (C₁-C₄)alkylcarbonylamino radical in which the amino functional group may or may not be substituted by a C₁-C₄ alkyl radical;
- an aminocarbonyl radical;
- an aminosulphonyl, (di)(C₁-C₄)alkylaminosulphonyl radical;
- a (C₁-C₄)alkylthio radical;
- a phenyl radical;
- a trifluoromethyl radical;
- a thio radical.

5. Compounds according to one of the preceding claims, **characterised in that** the R₄ radical represents a hydrogen atom, a methyl radical, optionally a hydroxycarbonyl radical, a methoxycarbonyl radical, a phenyl radical, a benzyl radical optionally substituted by one or two hydroxyl or amino groups, or a combination thereof.

6. Compounds according to one of the preceding claims, **characterised in that** the R₆ and R₇ radicals, which may be identical or différent, represent:
- a hydrogen atom;
- a methyl, ethyl, 2-hydroxyethyl, 3-hydroxypropyl or 3-methoxypropyl;
- a phenyl;
- a benzyl optionally substituted by one or two hydroxyl and/or amino groups;
- a methoxyphenyl;
- an aminophenyl;
- a (4-N,N-)diethylaminophenyl;
- optionally a CH₃CO- radical .

7. Dyeing composition comprising, as direct dye, in a medium suitable for dyeing keratin fibres, at least one compound of formula (I) or (II) according to any one of the preceding claim, and also mesomeric forms thereof, addition salts thereof with an acid and solvates thereof.

8. Composition according to the preceding claim, **characterized in that** the content of compound of formula (I) or (II) or of each of the compounds of formula (I) or (II) varies between 0.001 and 20% by weight relative to the total weight of the dyeing composition, more particularly between 0.01 to 10% by weight.

9. Composition according to either one of Claims 7 and 8, **characterized in that** it comprises at least one additional direct dye, at least one oxidation base optionally combined with at least one coupler, or mixtures thereof.

10. Composition according to any one of Claims 7 to 9, **characterized in that** it comprises at least one oxidizing agent.

11. Method for dyeing keratin fibres that consists in bringing a composition according to any one of Claims 7 to 10 into contact with said dry or wet fibres for a sufficient time to obtain the desired effect.

12. Multicompartment device in which a first compartment contains the composition according to any one of Claims 7 to 10 and a second compartment contains an oxidizing composition.

13. Method for prepaying compounds of formula (I) and/or (II) according to one of Claims 1 to 6, in which the following steps are carried out:
(a) condensation of a heteroaromatic aldehyde or ketone with a hydrazine derivative: 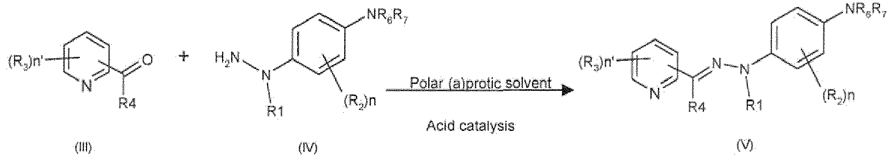
(b) Quaternization of the heterocyclic ring:

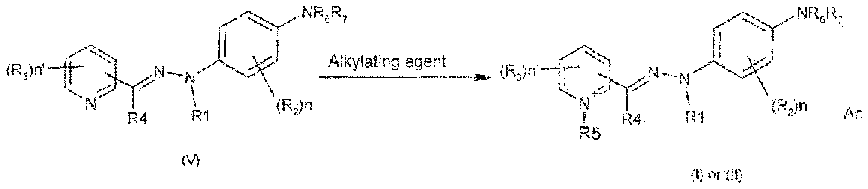

14. Method for preparing compounds of formula (I) and/or (II) according to one of Claims 1 to 6, in which the following steps are carried out:
(a) Condensation of a heteroaromatic aldehyde or ketone with a hydrazine derivative: 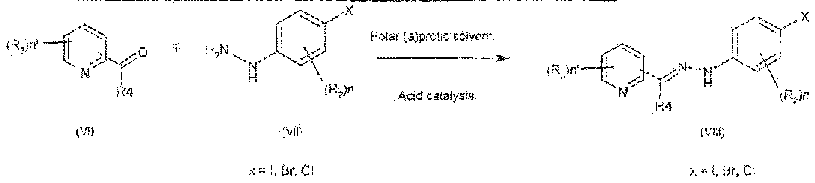
(b) Quaternization reaction of the heterocyclic ring 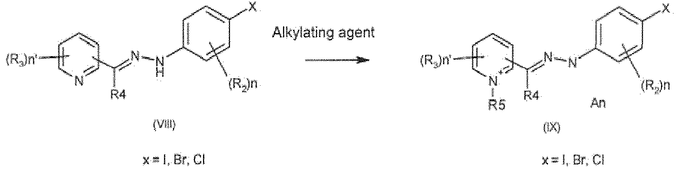
(c) Amination reaction 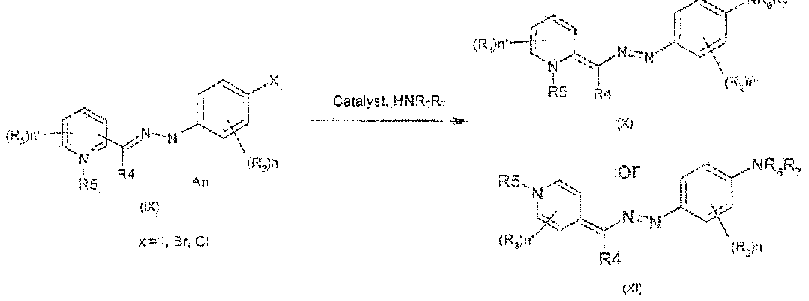
(d) Alkylation reaction

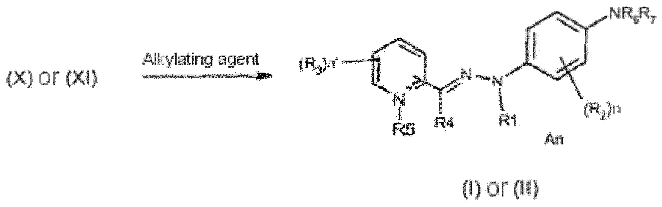

15. Method for preparing compounds of formula (I) and/or (II) according to one of Claims 1 to 6, in which the following steps are carried out: a
(a) Quaternization reaction 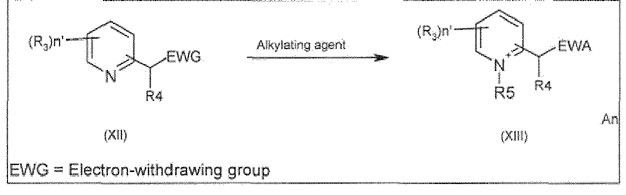
(b) Coupling reaction of a diazonium salt to an activated system 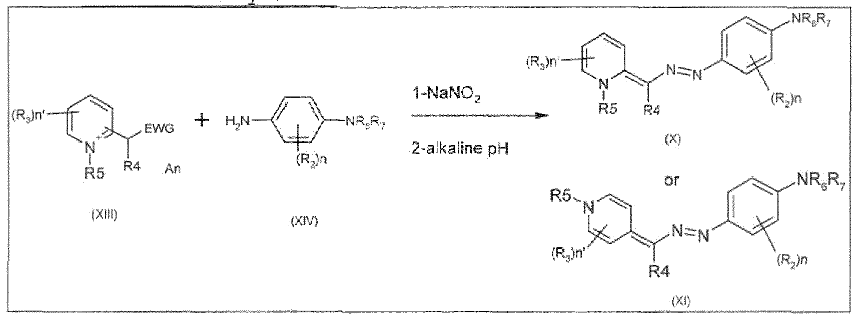
(c) Alkylation reaction

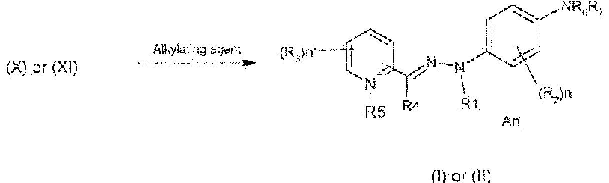

## Patentansprüche

1. Monokationische monochromophore Verbindungen mit einer Hydrazonfunktion der folgenden Formeln (I) und (II), mesomere Formen davon sowie Säureadditionssalze davon und Solvate davon: 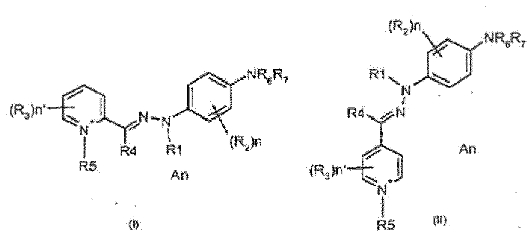 wobei in den Formeln (I) und/oder (II):
* der Rest R₁ für:
- Wasserstoff,
- einen gegebenenfalls substituierten C₁-C₂₀-Alkylrest, der gegebenenfalls durch ein oder mehrere Heteroatome und/oder eine oder mehrere Gruppen mit mindestens einem Heteroatom, das vorzugsweise unter Sauerstoff, Stickstoff, Schwefel, CO, SO, SO₂ oder Kombinationen davon ausgewählt ist, unterbrochen ist; wobei der Alkylrest keine Nitro-, Nitroso-, Peroxo- oder Diazofunktion enthält,
- einen gegebenenfalls substituierten Phenylrest,
- einen gegebenenfalls substituieren Benzylrest,
- einen Alkylcarbonylrest (R-CO-), worin R für einen C₁-C₄-Alkylrest steht,
- einem Alkylsulfonylrest (RSO₂-), worin R für einem C₁-C₄-Alkylrest steht,
- einen Arylsulfonylrest (R'SO₂-), worin R' für einen gegebenenfalls substituierten Phenyl-oder Benzylrest steht,
- einen (Di)(alkyl)aminosulfonylrest ((R₂)N-SO₂-), worin die Reste R unabhängig voneinander für Wasserstoff oder einem C₁-C₄-Alkylrest stehen,
- einem (Di)(alkyl)aminocarbonylrest ((R₂)N-CO-), worin die Reste R unabhängig voneinander für Wasserstoff oder einen C₁-C₄-Alkylrest stehen, steht,
* die Reste R₅ gleich oder verschieden sind und für:
- einen gegebenenfalls substituiertem C₁-C₂₀-Alkylrest, der gegebenenfalls durch ein oder mehrere Heteroatome und/oder eine oder mehrere Gruppen mit mindestens einem Heteroatom, das vorzugsweise unter Sauerstoff, Schwefel, CO, SO, SO₂ oder Kombinationen davon ausgewählt ist, unterbrochen ist; wobei der Alkylrest keine Nitro-, Nitroso-, Peroxo- oder Diazofunktion enthält,
- einen Trimethylsilyl-C₁-C₄-alkylrest,
- einen gegebenenfalls substituierten Phenylrest,
- einem gegebenenfalls substituierten Benzylrest stehen,
* dir Reste R₂ und R₃ unabhangig voneinander gleich oder verschieden sind und für:
- ein Halogenatom,
- einen gegebenenfalls substituierten C₁-C₁₆-Alkylrest, der gegebenenfalls durch ein oder mehrere Heteroatome und/oder eine oder mehrere Gruppen mit mindestens einem Heteroatom, das vorzugsweise unter Sauerstoff, Stickstoff, Schwefel, CO, SO, SO₂ oder Kombinationen davon ausgewählt ist, unterbrochen ist; wobei der Alkylrest keine Nitro-, Nitroso-, Peroxo- oder Diazofunktion enthält,
- einen Hydroxylrest,
- einem C₁-C₄-Alkoxyrest, eine C₂-C₄-(Poly)hydroxy-alkoxygruppe,
- einen Alkoxycarbonylrest (RO-CO-), worin R für einen C₁-C₄-Alkylrest steht,
- einen Alkylcarbonyloxyrest (RCO-O-), worin R für einen C₁-C₄-Alkylrest steht,
- einen gegebenenfalls substituierten Aryloxyrest,
- einen gegebenenfalls substituieren (Di)aryl-aminorest,
- einen Aminorest, der gegebenenfalls durch einen oder zwei gleiche oder verschiedene C₁-C₄-Alkylreste, die gegebenenfalls mindestens eine Hydroxyl- oder C₁-C₂-Alkoxygruppe tragen, substituiert ist, wobei die Alkylreste gegebenenfalls mit dem Stickstoffatom, an das sie gebunden sind, einen gegebenenfalls aromatischen, gegebenenfalls substituierten, gesättigten oder ungesättigten 5- bis 7-gliedrigen Heterocyclus, der gegebenenfalls ein weiteres Heteroatom, das mit Stickstoff identisch oder davon verschieben ist, enthält, bilden können,
- einen Alkylcarbonylaminorest (RCO-NR'-), worin der Rest R für einen C₁-C₄-Alkylrest steht und R' für Wasserstoff oder einen C₁-C₄-Alkylrest steht,
- eine (Di)(alkyl)aminocarbonylgruppe ((R₂)N-CO-), worin die Reste R unabhängig voneinander für Wasserstoff oder einen C₁-C₄-Alkylrest stehen,
- einen Ureidorest (N(R)₂-CO-NR'-), worin die Reste R unabhangig voreinander für einen C₁-C₄-Alkylrest stehen und R' für Wasserstoff oder einen C₁-C₄-Alkylrest steht,
- einen (Di)(alkyl)aminosulfonylrest ((R₂)N-SO₂-), worin der Rest R für ein Wasserstoffatom oder einen C₁-C₄-Alkylrest steht,
- einen Alkylthiorest (R-S-), worin der Rest R für einen C₁-C₄-Alkylrest steht,
- einen Alkylsulfonylaminorest (RSO₂-NR'-), worin der Rest R für einen C₁-C₄-Alkylrest steht und der Rest R' für ein Wasserstoffatom oder einem C₁-C₄-Alkylrest steht,
- einen Cyanorest (-C≡N),
- einen Phenylrest,
- eilen Trifluormethylrest (-CF₃),
- einem Thiorest (-SH),
- eilen Alkylsulfinylrest (RSO-), worin der Rest R für einen C₁-C₄-Alkylrest steht,
- einen Alkylsulfonylrest (RSO₂-), worin der Rest R für einen C₁-C₄-Alkylrest steht,
stehen,
* R₁ mit einem zur Gruppe NR₁ ortho-ständigen Rest R₂ und mit dem durch R₁ substituierten Stickstoffatom einen gegebenenfalls substituieren, gesättigten oder ungesättigtem 5- oder 6-gliedriger Heterocyclus bilden kann,
* zwei benachbarte Reste R₂ miteinander und mit den Kohlenstoffatomen, an die sie gebunden sind, einen gegebenenfalls substituierten, gegebenenfalls aromatischen 5- oder 6-gliedriger (hetero)cycli-schen Rest bilden können,
* zwei benachbarte Reste R₃ miteinander und mit den Kohlenstoffatomen, an die sie gebunden sind, einen gegebenenfalls substituierten aromatischen 5- oder 6-gliedrigen Ring bilden können,
* der Rest R₄ für:
- ein Wasserstoffatom,
- einen gegebenenfalls substituierten C₁-C₁₆-Alkylrest, der gegebenenfalls durch ein oder mehrere Heteroatome und/oder eine oder mehrere Gruppen mit mindestens einem Heteroatom, das vorzuasweise unter Sauerstoff, Stickstoff, Schwefel, CO, SO, SO₂ oder Kombinationen davon ausgewählt ist, unterbrochen ist; wobei der Allylrest kerne Vitro-, Nitrose-, Peroxo- oder Diazofunktion enthält,
- einem Aminrest, der gegebenenfalls durch einen oder zwei gleiche oder verschiedene C₁-C₄-Alkylreste, die gegebenenfalls mindestens eine Hydroxyd- oder C₁-C₂-Alkoxygruppe tragen, substituiert ist, wobei die Alkylreste gegebenenfalls mit dem Stickstoffatom, an das sie gebunden sind, einen gegebenenfalls substituierten, gesättigten oder ungesättigten oder aromatischen 5- bis 6-gliedrigen Heterocyclus, der gegebenenfalls mindestens ein weiteres Heteroatom, das mit Stickstoff identisch oder davon verschieben list, enthält, bilden können,
- einen Alkylcarbonylaminorest (RCO-NR'-), worin der Rest R für einem C₁-C₄-Alkylrest steht und R' für Wasserstoff oder einen C₁-C₄-Alkylrest steht,
- einen Ureidorest (N(R)₂-CO-NR'-), worin die Reste R unabhängig voreinander für einen C₁-C₄-Allylrest stehen und R' für Wasserstoff oder einen C₁-C₄-Alkylrest steht,
- einem Alkylsulfonylaminorest (RSO₂-NR'-), worin der Rest R für einem C₁-C₄-Alkylrest steht und R' für Wasserstoff oder einen C₁-C₄-Alkylrest steht,
- einem Hydroxycarbonylrest,
- einem C₁-C₄-Alkoxycarbonylrest,
- einen Cyanorest,
- einen gegebenenfalls substituierten Phenylrest,
- einen gegebenenfalls substituierten Benzoylrest, steht,
* die Reste R₆ und R₇ gleich oder verschieben sind und für:
- ein Wasserstoffatom,
- einem gegebenenfalls substituierten C₁-C₁₆-Allylrest, der gegebenenfalls durch ein oder mehrere Heteroatome und/oder eine oder mehrere Gruppen mit mindestens einem Heteroatom, das vorzuasweise unter Sauerstoff, Stickstoff, Schwefel, CO, SO, SO₂ oder Kombinationen davon ausgewählt ist, unterbrochen ist; wobei der Allylrest keine Nitro-, Nitroso-, Peroxo- oder Diazofunktion enthält,
- einen (Alkoxy)arylrest (-Ph-OR), worin der Rest R für Wasserstoff oder einen C₁-C₄-Alkylrest steht,
- einen (Di)(alkyl)aminoarylrest (PH-N(R)₂) worin die Reste R unabhangig voreinander für Wasserstoff oder einen gegebenenfalls durch ein Hydroxyl substituierten C₁-C₄-Alkylrest stehen,
- einen Alkylsulfonylrest (RSO₂-), worin der Rest R für einen C₁-C₄-Alkylrest steht,
- einen Aminocarbonylrest, einen (Di)(C₁-C₄)-alkylaminocarbonylrest,
- gegebenenfalls einen CH₃CO-Rest,
- Phenyl,
- gegebenenfalls durch eine oder mehrere Hydroxy-und/oder Aminogruppen substituiertes Benzoyl
stehen,
* die Reste R₆ und R₇ gegebenenfalls mit dem Stickstoffatom, an das sie gebunden sind, einen gegebenenfalls substituierten, gesättigten ober ungesättigten 5- bis 7-gliedrigen Heterocyclus, der gegebenenfalls ein weiteres Heteroatom, das mit Stickstoff identisch oder davon verschieden ist, enthält, bilden können,
* einer der Reste R₆ und R₇ auch mit dem Stickstoffatom, an das er gebunden ist, und mit einem zur Gruppe NR₆R₇ ortho-ständigen Rest R₂ einen gegebenenfalls substituierten, gesättigten oder ungesättigten 5- oder 6-gliedrigen Heterocyclus bilden kann,
* die Reste R₆ und R₇ mit dem Stickstoffatom, an das sie gebunden sind, und jeweils mit einen zur Gruppe NR₆R₇ ortho-ständigen Rest R₂ einen gegebenenfalls substituierten, gesättigten oder ungesättigten 5- oder 6-gliedrigen Heterocyclus bilden können,
* n für eine ganze Zahl zwischen 0 und 4 steht und dann, wenn n kleiner als 4 ist, das unsubstituierte Kohlenstoffatom bzw. die unsubstituierten Kohlenstoffatome ein Wasserstoffatom trägt bzw. tragen,
* n' für eine ganze Zahl zwischen 0 und 4 steht und dann, wenn n' kleiner als 4 ist, das unsubstituierte Kohlenstoffatom bzw. die unsubstituierten Kohlenstoffatome ein Wasserstoffatom trägt bzw. tragen,
* wobei die Elektroneutralität der Verbindungen der Formel (I) und/oder (II) durch ein oder mehrere gleiche oder verschiedene kosmetisch umbedenkliche Anionen An gewährleistet ist.

2. Verbindungen nach dem vorhergehenden Anspruch, **dadurch gekennzeichnet, dass** der Rest R₁ für ein Wasserstoffatom, eilen Methylrest, einen 2-Hydroxyethylrest, einen 3-Methoxypropylrest, einen (Di)(methyl)aminoethylrest, einen CH₃CO-Rest, einen CH₃SO₂-Rest, einen Phenylrest, einem 4-chlorophenylrest oder einen gegebenenfalls durch eine oder zwei Hydroxy- oder Aminogruppen oder eine Kombination davon substituierten Benzylrest steht.

3. Verwindungen nach einem der vorhergehenden Ansprüche, **dadurch gekennzeichnet, dass** die Reste R₅ gleich oder verschieben sind und für Methyl-, Methyl-, Propyl-, Butyl-, Pentyl-, Hexyl-, Heptyl-, Octyl-, 2-Hydroxyethyl-, 3-Hydroxypropyl-, 6-Hydroxyhexyl-, 2-Methoxyethyl-, 3-Methoxypropyl-, 3-Trimethylsilylpropyl-, 2-((Di)methyl)amino-propyl-, 3-((Di)methyl)aminopropyl-, Phenyl-, 4-Chlorphenyl-, Benzoyl- oder 3,4-Dihydroxybenzylreste stehen.

4. Verwindungen nach einem der vorhergehenden Ansprüche, **dadurch gekennzeichnet, dass** die Reste R₂ und R₃ gleich oder verschieden sind und für:
- ein unter Chlor und Fluor ausgewähltes Halogenatom,
- einen gegebenenfalls durch eine oder mehrere Hydroxy- oder ((Di)alkyl)amino- oder Sulfonylaminogruppen substituierten C₁-C₈-Alkylrest, der gegebenenfalls durch ein oder mehrere Heteroatome und/oder eine oder mehrere Gruppen mit mindestens einem Heteroatom, das vorzuasweise unter Sauerstoff, Schwefel, SO, SO₂ oder Kombinationen davon ausgewählt ist, unterbrochen ist,
- einen Hydroxylrest,
- einen C₁-C₄-Alkoxyrest,
- einen (C₁-C₄)-Alkoxycarbonylrest,
- einem gegebenenfalls substituierten Aryloxyrest,
- einen Aminorest, der gegebenenfalls:
■ durch einen oder zwei gleiche oder verschiedene C₁-C₄-Alkylreste, die gegebenenfalls mindestens eine Hydroxyl- oder C₁-C₂-Alkoxygruppe tragen,
■ durch einen oder zwei Phenyl-, Aminophenyl-oder Methoxyphenylreste
substituiert ist,
- einen (C₁-C₄)-Alkylcarbonylaminorest, in dem die Aminofunktion gegebenenfalls durch einem C₁-C₄-Allylrest substituiert ist,
- einem Aminocarbonylrest,
- einen Aminosulfonylrest, (Di)(C₁-C₄)-alkyl-aminosulfonylrest,
- einem (C₁-C₄)-Alkylthiorest,
- einen Phenylrest,
- einen Trifluormethylrest,
- einen Thiorest
stehen.

5. Verwindungen nach einem der vorhergehenden Ansprüche, **dadurch gekennzeichnet, dass** der Rest R₄ für ein wasserstoffatom, einen Methylrest, gegebenenfalls einen Hydroxycarbonylrest, einen Methoxycarbonylrest, einen Phenylrest oder einen gegebenenfalls durch eine oder zwei Hydroxy- oder Aminogruppen oder eine Kombination davon substituierten Benzoylrest steht.

6. Verwindungen nach einem der vorhergehenden Ansprüche, **dadurch gekennzeichnet, dass** die Reste R₆ und R₇ gleich oder verschieben sind und für:
- ein Wasserstoffatom,
- Methyl, Ethyl, 2-Hydroxyethyl, 3-Hydroxypropyl oder 3-Methoxypropyl,
- Phenyl,
- eine gegebenenfalls durch eine oder zwei Hydroxyd- und/oder Aminogruppen substituierte Benzylgruppe,
- Methoxyphenol,
- Aminophenol,
- (4-N,N-)Diethylaminophenyl,
- gegebenenfalls einen CH₃CO-Rest
stehen.

7. Färbezusammensetzung, die in einem zum Färben von Keratinfaser geeigneten Medium als Direktfarbstoff mindestens eine Verbindung der Formel (I) oder (II) nach einem der vorhergehenden Ansprüche sowie mesomere Formen davon, Säureadditionssalze davon und Solvate davon umfasst.

8. Zusammensetzung nach dem vorhergehenden Anspruch, dadurch gekenntzeichnet, dass der Gehalt an Verbindung der Formeln (I) oder (II) oder jeder der verwindungen der Formel (I) oder (II) zwischen 0,001 und 20 Gew.-%, bezogen auf das Gesamtgewicht der Färbezusammensetzung, insbesonzwischen 0,01 und 10 Gew.-%, variiert.

9. Zusammensetzung nach einem der Ansprüche 7 oder 8, dadurch gekenntzeichnet, dass sie mindestens einen zusätzlichen Direktfarbstoff, mindestens eine oxidationsbase, die gegebenenfalls mit mindestens einem Koppler kombiniert ist, oder Mischungen davon umfasst.

10. Zusammensetzung nach einem der Ansprüche 7 bis 9, **dadurch gekennzeichnet, dass** sie mindestens ein Oxidationsmittel umfasst.

11. Verfahren zum Färben von Keratinfasern, das darin besteht, dass man eine Zusammensetzung nach einem der Ansprüche 7 bis 10 mit den trockenen oder feuchten Fasern über einen zur Erzielung des gewünschten Effekts ausreichenden Zeitraum in Berührung bringt.

12. Vorrichtung mit mehreren Kompartimenten, in der ein erstes Compartiment die Zusammensetzung nach einem der Ansprüche 7 bis 10 enthält und ein zweites Compartiment eine oxidierend wirkende Zusammensetzung enthält.

13. Verfahren zur Verstellung von Verbindungen der Formel (I) und/oder (II) nach einem der Ansprüche 1 bis 6, bei dem man die folgenden Schritte durchführt:
(a) Kondensation eines heteroaromatischen Aldehyds oder Ketons mit einem Hydrazinderivat: 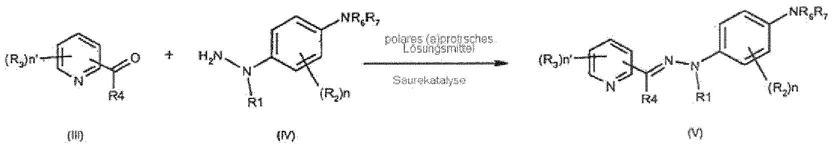
(b) Quaternisierung des heterocyclischen Rings:

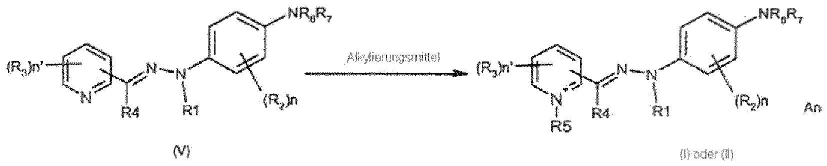

14. Verfahren zur Verstellung von Verbindungen der Formel (I) und/oder (II) nach einem der Ansprüche 1 bis 6, bei dem man die folgenden Schritte durchführt:
(a) Kondensation eines heteroaromatischen Aldehyds oder Ketons mit einem Hydrazinderivat: 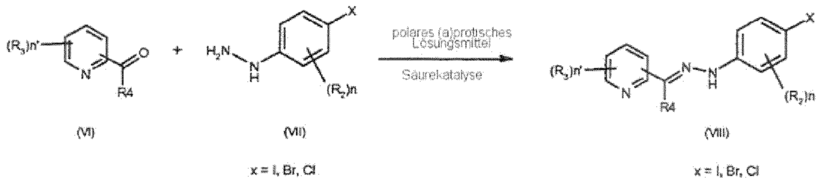
(b) Quaternisierungsreakt des heterocyclischen Rings: 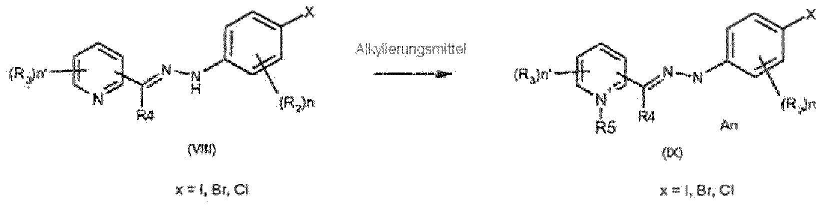
(c) Aminierungsreaktion 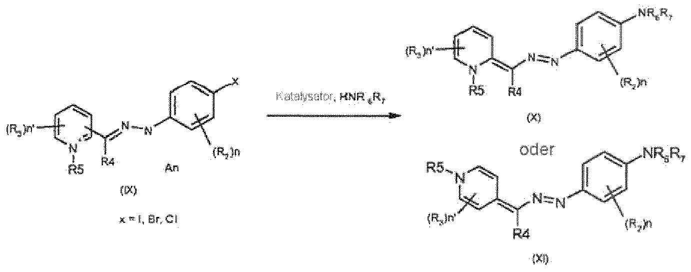
(d) Alkylierungsreaktion

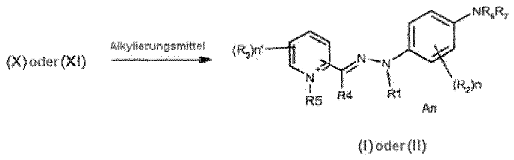

15. Verfahren zur Herstellung von Verbindungen der Formel (I) und/oder (II) nach einem der Ansprüche 1 bis 6, bei dem man die folgenden Schritte durchführt:
(a) Quaternisierungsreaktion 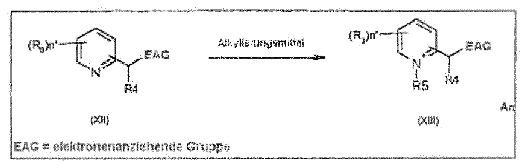
(b) Kupplungsreaktion eines Diazoniumsalzes an ein aktiviertes Sytem 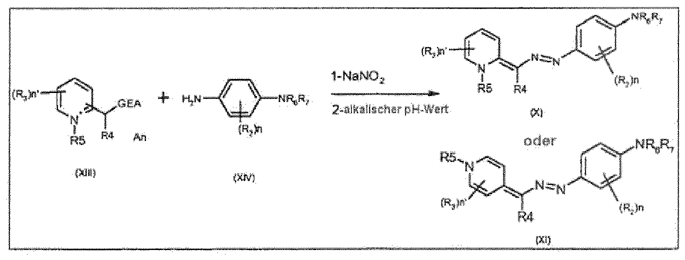
(c) Alkylierungsreaktion
